# EUROPEAN PATENT APPLICATION

(11) **EP 3 827 808 A1**
(43) Date of publication of application: **02.06.2021**
(21) Application number: 19840987.2
(22) Date of filing: 24.07.2019
(51) Int. Cl.: A61K 8/85, A45D 33/38, A61K 8/02, A61K 8/73, A61Q 1/00, A61Q 19/00

(54) **SKIN ADHESIVE FILM, AND TRANSFER SHEET**

(30) Priority: 25.07.2018 JP 2018139458; 06.11.2018 JP 2018209016; 20.12.2018 JP 2018238037; 20.12.2018 JP 2018238038
(71) Applicant: Toppan Printing Co., Ltd., Tokyo 110-0016 (JP)
(72) Inventor: WATANABE Manabu, Tokyo 110-0016 (JP); SAKAIRI Koji, Tokyo 110-0016 (JP); HAYASAKA Yuichiro, Tokyo 110-0016 (JP); MINOWA Kazuyo, Tokyo 110-0016 (JP); MORISHIMA Natsumi, Tokyo 110-0016 (JP)
(74) Representative: TBK
(86) International application number: PCT/JP2019/029032
(87) International publication number: WO 2020/022383

(57) **Abstract**

A skin-adhesive film has an average thickness of 5 µm or less. A blockage ratio, which is a ratio of blocking evaporation of water having a temperature equal to a human body temperature at a site where the skin-adhesive film is provided relative to that at a site where the skin-adhesive film is not provided, is 5% or more and 60% or less.

## Description

### [Technical Field]

The present invention relates to a skin-adhesive film, and a transfer sheet having the skin-adhesive film.

### [Background Art]

Ultra-thin films that can be adhered to a surface of an organ of a living body are known. Such films, having a thickness of the order of several nanometers to several micrometers, are used adhered to an organ such as skin.

For example, NPL 1 discloses that the above film can be used as a wound dressing. Further, the above film can also be used as a film adhered to the skin for assisting in skin care and assisting with makeup. For example, PTL 1 and PTL 2 disclose a polylactic acid film carrying sodium hyaluronate as a skin-adhesive film. Further, PTL 2 proposes a method of cosmetic application in which cosmetics are applied to the film after the film is adhered to the skin.

### [Citation List]

### [Patent Literature]

PTL 1: WO 2014/058060
PTL 2: WO 2014/058066

### [Non-Patent Literature]

NPL 1: T. Fujie et al., Adv. Funct. Mater., 2009, vol. 19, pp. 2560-2568

### [Summary of the Invention]

### [Problem to be Solved by the Invention]

In adhesion of the above film to the skin, the film preferably has at least one of the following properties to increase utility in cosmetic applications. That is, the properties include good adhesion of cosmetics to the film, high permeability of water vapor through the film, prevention of sebum penetration through the film, good moisturizing properties to the skin, inconspicuous appearance of the film when adhered, a soft focus effect produced by the film, good adhesion of the film to the skin, high UV blocking effect by the film, decorative effect required for makeup, and abrasion resistance against rubbing with a finger or a cloth. The soft focus effect refers to properties that blur fine imperfections such as wrinkles, spots, and freckles on the skin surface while allowing the presence of the skin to be recognized via the adhered film.

The present invention has been made to provide a skin-adhesive film and a transfer sheet having increased utility in cosmetic applications.

### [Solution to Problem]

A skin-adhesive film for solving the above problem is a skin-adhesive film having an average thickness of 5 µm or less, wherein a blockage ratio, which is a ratio of blocking evaporation of water having a temperature equal to a human body temperature at a site where the skin-adhesive film is provided relative to that at a site where the skin-adhesive film is not provided, is 5% or more and 60% or less.

With this configuration, good moisturizing properties are obtained at a position where the skin-adhesive film is adhered. Accordingly, the skin-adhesive film has increased utility in cosmetic applications.

A skin-adhesive film for solving the above problem is a skin-adhesive film having an average thickness of 5 µm or less including: a first surface; and a second surface which is a surface opposite to the first surface, the second surface being configured to be adhered to a skin surface, wherein the skin-adhesive film has a plurality of recesses that are recessed from the first surface, and a ratio of the recesses identified by binarizing an image of the first surface by a discriminant analysis method, which is a ratio of a total area occupied by the recesses per unit area calculated based on the image binarized, is 0.05% or more and 30% or less.

With this configuration, good moisturizing properties are obtained at a position where the skin-adhesive film is adhered. Accordingly, the skin-adhesive film has increased utility in cosmetic applications.

A skin-adhesive film for solving the above problem is a skin-adhesive film including: a first surface; and a second surface which is a surface opposite to the first surface, the second surface being configured to be adhered to a skin surface, wherein the skin-adhesive film has a portion having a thickness of 5 µm or less, and the first surface has an arithmetic average roughness Ra of 100 nm or more and 5 µm or less, a ten-point average roughness Rz of 1 µm or more and 50 µm or less, and an average interval between peaks and valleys Sm of 10 µm or more and 500 µm or less.

With this configuration, since the first surface has roughness with sufficient height and density while ensuring a strength of the skin-adhesive film, cosmetics are likely to be retained by the asperities on the first surface. Therefore, adhesion of cosmetics to the skin-adhesive film is increased. Accordingly, the skin-adhesive film has increased utility in cosmetic applications.

A skin-adhesive film for solving the above problem is a skin-adhesive film including: a first surface, and a second surface which is a surface opposite to the first surface, the second surface being configured to be adhered to a skin surface, wherein the skin-adhesive film has an average mass of 0.1 g/m² or more and 4.0 g/m² or less, and a haze of 7% or more and 65% or less.

With this configuration, due to the haze being 7% or more, the skin-adhesive film provides a light-scattering effect sufficient to suppress gloss and interference colors. Accordingly, a site on the skin where the skin-adhesive film is adhered is rendered inconspicuous, and the soft focus effect is improved. Further, due to the haze being 65% or less, the skin-adhesive film appears cloudy, and a site where the skin-adhesive film is adhered is rendered inconspicuous. Accordingly, the skin-adhesive film has increased utility in cosmetic applications.

A skin-adhesive film for solving the above problem is a skin-adhesive film including: a thin film portion having an average thickness of 0.05 µm or more and less than 0.3 µm; and a thick film portion having an average thickness of 0.3 µm or more, wherein a plurality of regions are virtually defined and arranged in a circumferential direction in a peripheral region extending along an outer edge of the skin-adhesive film as viewed in a direction perpendicular to a surface of the skin-adhesive film, the plurality of regions being included in the thin film portion.

With this configuration, since the film has a small thickness at the peripheral region in which the thin film portion is located, that is, at the edge of the skin-adhesive film, skin adhesion is increased. On the other hand, the thick film portion can provide improvement in moisturizing properties, UV blocking effect, decorative effect, and abrasion resistance. Accordingly, when the skin-adhesive film is adhered to the skin, the film can successfully exhibit functions derived from the thick film while preventing peeling at the outer edge of the film. That is, both adhesion to the skin and properties derived from the thick film can be achieved. Accordingly, the skin-adhesive film has increased utility in cosmetic applications.

A transfer sheet for solving the above problem is a transfer sheet including: the skin-adhesive film described above; and a support substrate that supports the skin-adhesive film.

With this configuration, the skin-adhesive film is prevented from becoming deformed during storage and transport of the transfer sheet. Further, since the skin-adhesive film is supported by the support substrate, ease of handling of the skin-adhesive film is increased.

### [Advantageous Effects of Invention]

According to the present invention, skin-adhesive films can be provided with increased utility in cosmetic applications.

### [Brief Description of Drawings]

Fig. 1 is view illustrating a cross-sectional structure of a skin-adhesive film according to a first embodiment.
Fig. 2 is a view illustrating a cross-sectional structure of a transfer sheet according to the first embodiment.
Fig. 3 is a view illustrating an example of a planar structure of the skin-adhesive film according to the first embodiment.
Fig. 4 is a view illustrating an example of a planar structure of the skin-adhesive film according to the first embodiment.
Fig. 5 is a view illustrating an example of a planar structure of the skin-adhesive film according to the first embodiment.
Fig. 6 is a view illustrating a method for measuring an area ratio of a bottomed section to the skin-adhesive film of the first embodiment.
Fig. 7 is a view illustrating a measurement principle of an area ratio of a bottomed section to the skin-adhesive film of the first embodiment.
Fig. 8 is a view illustrating a measurement principle of an area ratio of a bottomed section to the skin-adhesive film of the first embodiment.
Fig. 9 is a view illustrating a measurement principle of an area ratio of a bottomed section to the skin-adhesive film of the first embodiment.
Fig. 10 is a view illustrating a cross-sectional structure of a first type skin-adhesive film according to a third embodiment.
Fig. 11 is a view illustrating a cross-sectional structure of a second type skin-adhesive film according to the third embodiment.
Fig. 12 is a view illustrating another example of a cross-sectional structure of the skin-adhesive film according to the third embodiment.
Fig. 13 is a view illustrating a cross-sectional structure of a transfer sheet of the third embodiment.
Fig. 14 is a view illustrating a cross-sectional structure of a first type skin-adhesive film according to a fourth embodiment.
Fig. 15 is a view illustrating a cross-sectional structure of a second type skin-adhesive film according to the fourth embodiment.
Fig. 16 is a view illustrating a cross-sectional structure of a third type skin-adhesive film according to the fourth embodiment.
Fig. 17 is a view illustrating a cross-sectional structure of a transfer sheet of the fourth embodiment.
Fig. 18 is view illustrating an example of a planar structure of a skin-adhesive film according to a fifth embodiment.
Fig. 19 is a view illustrating another example of a planar structure of the skin-adhesive film according to the fifth embodiment.
Fig. 20 is a view illustrating another example of a planar structure of the skin-adhesive film according to the fifth embodiment.
Fig. 21 is a view illustrating an example of a measurement target region of a film thickness of the skin-adhesive film according to the fifth embodiment.
Fig. 22 is a view illustrating a cross-sectional structure of a first type transfer sheet according to the fifth embodiment.
Fig. 23 is a view illustrating cosmetics applied to the skin in a process of transferring a skin-adhesive film to the skin by using a transfer sheet of the fifth embodiment.
Fig. 24 is a view illustrating a transfer sheet being adhered to the skin in a process of transferring a skin-adhesive film to the skin by using a transfer sheet of the fifth embodiment.
Fig. 25 is a view illustrating a skin-adhesive film having been adhered to the skin in a process of transferring a skin-adhesive film to the skin by using a transfer sheet of the fifth embodiment.
Fig. 26 is a view illustrating a cross-sectional structure of a second type skin-adhesive film according to the fifth embodiment.
Fig. 27 is a view illustrating a cross-sectional structure of a third type skin-adhesive film according to the fifth embodiment.
Fig. 28 is a view illustrating a cross-sectional structure of a fourth type skin-adhesive film according to the fifth embodiment.
Fig. 29 is a view illustrating a cross-sectional structure of a fifth type skin-adhesive film according to the fifth embodiment.
Fig. 30 is a view illustrating a cross-sectional structure of a sixth type skin-adhesive film according to the fifth embodiment.
Fig. 31 is a view illustrating a cross-sectional structure of a seventh type skin-adhesive film according to the fifth embodiment.
Fig. 32 is a view illustrating a cross-sectional structure of an eighth type skin-adhesive film according to the fifth embodiment.
Fig. 33 is a view illustrating a cross-sectional structure of a ninth type skin-adhesive film according to the fifth embodiment.
Fig. 34 is a view illustrating a cross-sectional structure of a tenth type skin-adhesive film according to the fifth embodiment.
Fig. 35 is a view illustrating a cross-sectional structure of an eleventh type skin-adhesive film according to the fifth embodiment.
Fig. 36 is a view illustrating a cross-sectional structure of a twelfth type skin-adhesive film according to the fifth embodiment.
Fig. 37 is a view illustrating a cross-sectional structure of a thirteenth type skin-adhesive film according to the fifth embodiment.

### [Description of Embodiments]

### (First Embodiment)

A skin-adhesive film adhered to the skin on the face can prevent sebum exudation onto the skin. Accordingly, when cosmetics are applied to the film, prevention of makeup deterioration is expected. In order to enhance the effect of preventing sebum exudation, a skin-adhesive film preferably has few defects such as micropores. However, as the film surface has fewer defects and higher smoothness, application of cosmetics to the skin-adhesive film becomes difficult. Further, when a skin-adhesive film is too dense, water vapor evaporation from the skin may be excessively suppressed, and a user may feel discomfort due to humidity on the skin.

Therefore, there is a demand for skin-adhesive films which balance suppression of permeation of sebum with improvement in adhesion of cosmetics and permeability of water vapor. An object of the first embodiment is to provide such a skin-adhesive film and a transfer sheet.

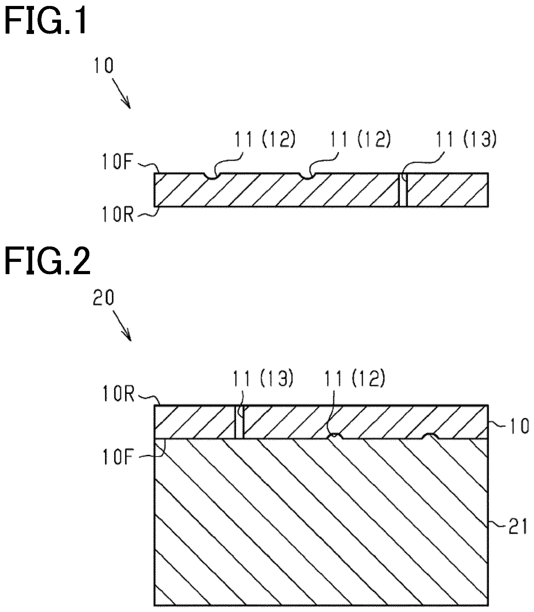

With reference to Figs. 1 to 9, the first embodiment of a skin-adhesive film and a transfer sheet will be described.

### [Overall Configuration of Skin-Adhesive Film And Transfer Sheet]

Fig. 1 is a view illustrating a cross-sectional structure of a skin-adhesive film 10. The skin-adhesive film 10 has a first surface 10F, and a second surface 10R, which is a surface opposite to the first surface 10F. The second surface 10R is adhered to the skin. The first surface 10F is a surface exposed to the outside and also a surface to which cosmetics are applied when the skin-adhesive film 10 is adhered to the skin.

The skin-adhesive film 10 includes a plurality of recesses 11 recessed from the first surface 10F. Each recess 11 is either a bottomed section 12 having a bottom in the skin-adhesive film 10 or a penetrating section 13 that penetrates the entire skin-adhesive film 10 from the first surface 10F to the second surface 10R. The plurality of recesses 11 may be composed of both the bottomed sections 12 and the penetrating sections 13, or may be composed of either the bottomed sections 12 or the penetrating sections 13.

The skin-adhesive film 10 has a thickness of 1 nm or more and 5000 nm or less. This thickness of the skin-adhesive film 10 is an average thickness, which is obtained by averaging the film thicknesses in the cross-section of the skin-adhesive film 10 at five or more measurement points by using an electron microscope. The thickness of the skin-adhesive film 10 is preferably 1 nm or more and 1000 nm or less, more preferably 1 nm or more and 500 nm or less, and still more preferably 150 nm or more and 500 nm or less in order to increase adhesion of the skin-adhesive film 10 to the skin, increase conformability of the skin-adhesive film 10 to the surface shape of the skin, and reduce the discomfort that the user may feel when the skin-adhesive film 10 is adhered to the skin. The skin-adhesive film 10 may be configured as a single layer film, or may be configured as a multi-layered film.

The material for forming the skin-adhesive film 10 is not particularly limited. The material of the skin-adhesive film 10 is preferably a resin having low toxicity, skin irritation, and skin sensitization. Examples of the material for the skin-adhesive film 10 include natural polymers such as hyaluronic acid, fibroin, and chitosan, and ester resins such as polylactic acid, polyglycolic acid, and polycaprolactone, and copolymer resins thereof. Further, resins for use as film-formers in cosmetics may also be used as the material for the skin-adhesive film 10. Examples of such resins include acrylic resin, silicone, and copolymer resins thereof, and cellulose derivatives such as cellulose acetate, cellulose acetate propionate, and cellulose acetate butyrate. Further, examples of the material for the skin-adhesive film 10 include resins that are commonly used as materials for medical products, such as polycarbonate, cycloolefin copolymer, styrene-butadiene elastomer, polyimide, acrylic resin, urethane resin, and copolymer resins thereof. The molecular weight of the materials for the skin-adhesive film 10 is not particularly limited. For example, the skin-adhesive film 10 may be made of one type of material having a predetermined average molecular weight, or may also be made of a plurality of types of materials having different average molecular weights.

In addition, the skin-adhesive film 10 may contain functional materials that perform a predetermined function to the skin. Examples of these functional materials include cosmetics and cosmetic ingredients used for skin care, such as moisturizing creams and beauty essence, dye, drugs, proteins, and enzymes. The skin-adhesive film 10 may contain one type of functional material, or may also contain two or more types of functional materials.

Fig. 2 is a view illustrating a cross-sectional structure of a transfer sheet 20. The transfer sheet 20 includes the skin-adhesive film 10, and a support substrate 21 that supports the skin-adhesive film 10. The support substrate 21 is in contact with the first surface 10F of the skin-adhesive film 10. The support substrate 21 has a function of reducing deformation of the skin-adhesive film 10 during storage of the skin-adhesive film 10 and transport of the skin-adhesive film 10 to an adhesion site when in use. Since the skin-adhesive film 10 is supported by the support substrate 21, ease of handling of the skin-adhesive film 10 can be increased.

The depth of the recesses 11 may be completely or partially filled with the support substrate 21, or may not be filled with the support substrate 21. When the depth of the recess 11 is at least partially filled with the support substrate 21, it is possible to prevent deformation of the recess 11 and entry of foreign substances into the recess 11 until the support substrate 21 is removed from the skin-adhesive film 10.

The support substrate 21 is made of a porous substrate. The porous substrate has a large number of fine pores inside the substrate so that liquid can infiltrate or penetrate through the pores. Examples of the porous substrate that can be used as the support substrate 21 include sheets made of fibrous materials such as non-woven fabric, paper, knitted fabric, and woven fabric, and resin sheets having a structure with gaps, such as a mesh. Among these substrates, a non-woven fabric is preferably used due to the ability to allow quick absorption and diffusion of water. Examples of fibers forming the non-woven fabric include natural fibers such as cotton, hemp, wool, and pulp, semi-synthetic fibers such as rayon, and synthetic fibers such as polyvinyl alcohol, polyacrylates. Among the above fibers, natural fibers, especially pulps, are preferably used. The non-woven fabric may be formed of a single type of fiber, or two or more types of fibers.

The methods for manufacturing the non-woven fabric used as the support substrate 21 are not specifically limited. The non-woven fabric may be manufactured by one of the methods including spunlacing, spunbonding, needle-punching, melt blowing, air-laying, flash spinning, and resin adhesion. The non-woven fabric used as the support substrate 21 preferably has a basis weight of 10 g/m² or more and 150 g/m² or less, and more preferably 20 g/m² or more and 50 g/m² or less in view of texture for good tactile feel. The basis weight of the non-woven fabric refers to a mass per unit area of the non-woven fabric.

Further, the support substrate 21 is not limited to a porous substrate, and may also be formed of a substrate such as a resin sheet or metal foil having no pores inside.

The transfer sheet 20 may further include a protective layer that covers the second surface 10R of the skin-adhesive film 10. The protective layer has a function of protecting the second surface 10R. The protective layer is preferably made of a porous substrate. The porous substrate for forming the protective layer may be made of the same material as that of the porous substrate for forming the support substrate 21. The protective layer and the support substrate 21 may be formed of the same type of porous substrates, or may be formed of different types of porous substrates. In addition, the protective layer is not limited to a porous substrate, and may also be formed of a substrate such as a resin sheet or metal foil having no pores inside.

### [Planar Structure of Skin-Adhesive Film]

The details of the recess 11 in the skin-adhesive film 10 will be described below.

When viewed in a direction perpendicular to the first surface 10F, the recesses 11 are formed in a circular, elliptical, polygonal, or linear shape, or an indefinite shape other than these shapes. The linear shape may be any of a curved, polygonal, wavy, or straight shape. In particular, when the recesses 11 have a shape selected from the group consisting of a circular, elliptical, rectangular, and linear shapes, formation of the recesses 11 and measurement of the area of the recesses 11 can be easily performed. Further, the plurality of recesses 11 may include recesses 11 having different shapes from each other. The recesses 11 may also have a shape, such as a mesh, which is continuous across the entire skin-adhesive film 10.

The plurality of recesses 11 may be arranged regularly or irregularly as viewed in the direction perpendicular to the first surface 10F. Further, the plurality of recesses 11 may include recesses 11 arranged regularly and recesses 11 arranged irregularly.

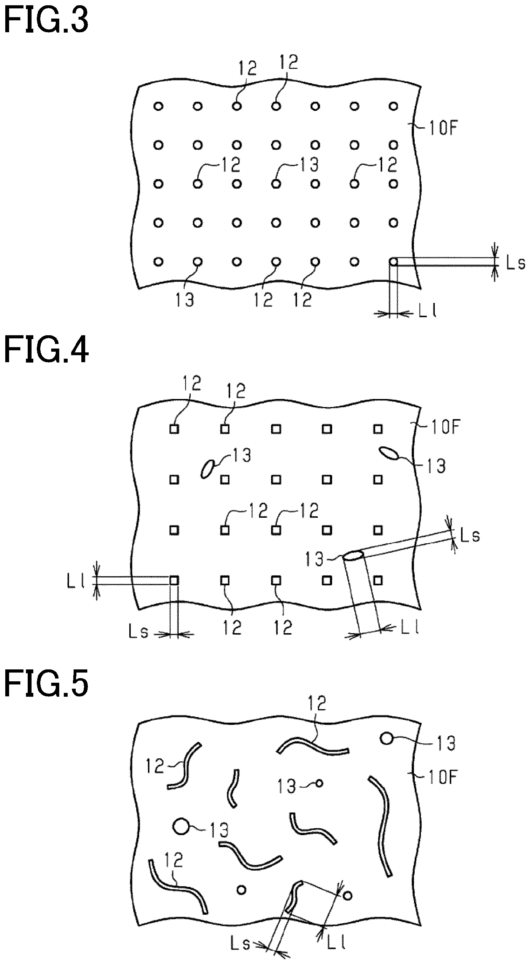

Figs. 3 to 5 show examples of the shape and arrangement of the recesses 11 in plan view as viewed perpendicular to the first surface 10F. As shown in Fig. 3, a plurality of recesses 11 composed of the bottomed sections 12 and the penetrating sections 13 may be regularly arranged. Fig. 3 illustrates that the plurality of recesses 11 are arranged in a matrix. In the regular arrangement of the recesses 11, a plurality of bottomed sections 12 may be arranged regularly or irregularly. Further, in the regular arrangement of the recesses 11, a plurality of penetrating sections 13 may also be arranged regularly or irregularly. Although Fig. 3 shows that all the recesses 11 including the bottomed sections 12 and the penetrating sections 13 have the same circular shape, the shapes of the plurality of recesses 11 may not necessarily be the same.

As shown in Fig. 4, a plurality of recesses 11 may be arranged irregularly as a whole, in which a plurality of bottomed sections 12 are arranged regularly, and a plurality of penetrating sections 13 are arranged irregularly. Alternatively, a plurality of recesses 11 may be arranged irregularly as a whole, in which a plurality of bottomed sections 12 are arranged irregularly, and a plurality of penetrating sections 13 are arranged regularly. In Fig. 4, the bottomed section 12 and the penetrating section 13 have different shapes from each other. All the plurality of bottomed sections 12 have the same rectangular shapes, and all the plurality of penetrating sections 13 have the same elliptical shapes. These configurations are not exhaustive, and the shapes of the plurality of bottomed sections 12 may not necessarily be constant, and the shapes of the plurality of penetrating sections 13 may not necessarily be constant. Alternatively, the bottomed sections 12 and the penetrating sections 13 may have the same shape.

As shown in Fig. 5, a plurality of recesses 11 may be arranged irregularly as a whole, in which both a plurality of bottomed sections 12 and a plurality of penetrating sections 13 are arranged irregularly. In Fig. 5, the plurality of bottomed sections 12 have curved shapes with irregular lengths, and the plurality of penetrating sections 13 have circular shapes with irregular diameters. These configurations are not exhaustive, and the shapes of the plurality of bottomed sections 12 may be constant, and the shapes of the plurality of penetrating sections 13 may be constant. Alternatively, the bottomed sections 12 and the penetrating sections 13 may have the same shape.

The size of the recess 11 will be described below. When viewed in the direction perpendicular to the first surface 10F, each of the plurality of recesses 11 preferably has a ratio of a length L1 in the longitudinal direction to a length Ls in the lateral direction in a range of 1 or more and 500 or less. Further, the length Ls of the recess 11 in the lateral direction is preferably 0.1 µm or more and 1 µm or less. When viewed in the direction perpendicular to the first surface 10F, the length Ls in the lateral direction is a short side length of a minimum virtual rectangle that circumscribes the recess 11. Further, the length L1 in the longitudinal direction is a long side length of the above virtual rectangle. When the size of the recess 11 is within the above range, the recess 11 is prevented from being too large, and an area having asperities on the first surface 10F can be sufficient for the area ratio of the recesses 11. That is, the size of the recesses 11 can be appropriate for adhesion of cosmetics and permeation of water vapor.

The recesses 11 have an area ratio Rt of 0.05% or more and 25% or less. The area ratio Rt of the recesses 11 refers to a ratio of a total area occupied by the recesses 11 per unit area when viewed in the direction perpendicular to the first surface 10F. That is, the area ratio Rt is a ratio of the total area occupied by the plurality of recesses 11 to the total area of the skin-adhesive film 10 in a region having a unit area.

A method for measuring the area ratio Rt of the recesses 11 will be described below. The area ratio Rt is obtained by separately measuring an area ratio Ra of the bottomed sections 12 and an area ratio Rb of the penetrating sections 13, and then summing the area ratio Ra and the area ratio Rb. The area ratio Ra of the bottomed sections 12 refers to a ratio of the total area occupied by the bottomed sections 12 per unit area, and the area ratio Rb of the penetrating sections 13 refers to a ratio of the total area occupied by the penetrating sections 13 per unit area.

In measuring the area ratio Ra of the bottomed sections 12, a total area of the bottomed sections 12 in a binarized image is used as the total area occupied by the bottomed sections 12. The binarized image refers to an image obtained by binarizing a captured image of an observation region, which is a part of the first surface 10F, by a discriminant analysis method. Further, the above length Ls of the bottomed section 12 in the lateral direction and the length L1 in the longitudinal direction are also measured by using a binarized image.

Specifically, the area ratio Ra of the bottomed sections 12 is measured by the following procedure.
1) A double-sided tape which uses a plastic foam (manufactured by Nichiban Co., Ltd.: Nice Tack Sponge double-sided tape, sponge type, NW-P15) is punched with a punch or the like to form a circular aperture. Then, a protective film on one side of the double-sided tape is removed, and the second surface 10R of the skin-adhesive film 10 in the transfer sheet 20 is bonded to the double-sided tape. A diameter of the above aperture in the double-sided tape may be a size that does not cause distortion in a portion to be observed in the skin-adhesive film 10 after the step described in 2) below is performed, and for example may be approximately 3 mm.
2) The support substrate 21 of the transfer sheet 20 in the structure formed in the above 1) is moistened with water and removed from the transfer sheet 20. Thus, a sample for measuring the area ratio Ra is obtained.
3) In the sample formed in the above 2), a protective film on the other side of the double-sided tape is removed. Then, the sample is bonded to a black plate. Then, a portion of the skin-adhesive film 10 exposed through the aperture of the double-sided tape is imaged by a dark field method using an 8-bit monochrome digital camera. The number of pixels in the image is, for example, 5 million pixels (2448 x 2048 pixels), and an imaging region is, for example, a rectangular region of 7.06 mm x 8.44 mm.
4) The image captured in the above 3) is subjected to image processing to obtain the area ratio Ra of the bottomed sections 12. The image processing is performed using ImageJ, which is open source image processing software. An observation region to be measured is a region having an area larger than a square with sides of 1 mm. Such an observation region is sufficiently large to generally observe the distribution of the recesses 11 to thereby calculate the area ratio Ra. First, the above image is subjected to sharpening at a radius of 4 pixels and a mask weight of 0.9 to emphasize the portion where the bottomed sections 12 are present. Subsequently, a binarization threshold is determined by, for example, Otsu's method for discriminant analysis, and the above image is binarized by using the threshold to obtain a binarized image. The binarized image is subjected to particle analysis to thereby obtain the area ratio Ra of the bottomed sections 12. In addition, the method for determining a threshold is not limited to Otsu's method for discriminant analysis. Any other method can also be used as long as the same effects as those of Otsu's method can be obtained.

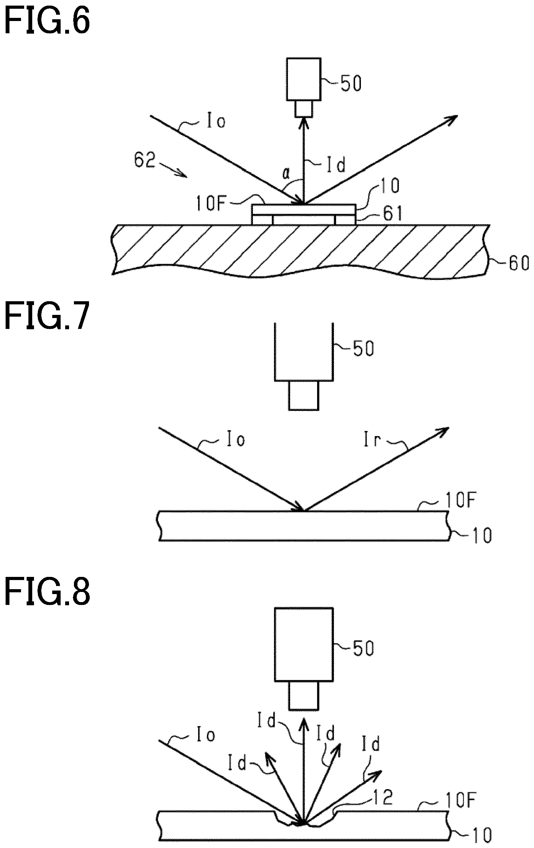

With reference to Figs. 6 to 9, the principle by which the bottomed sections 12 can be identified by imaging using a digital camera will be described.

As shown in Fig. 6, as described in 1) to 3) in the above procedure, a sample 62 composed of the skin-adhesive film 10 and a double-sided tape 61 is bonded to the black plate 60. Incident light Io is allowed to be incident on the first surface 10F of the skin-adhesive film 10 in the sample 62 at an incident angle α of, for example, 45° or more. Then, the first surface 10F is imaged in a direction normal to the first surface 10F by using a camera 50, which is an 8-bit monochrome digital camera. Alight receiving element in the camera 50 receives, among the reflected light from the incident light Io, a scattered light Id emitted in the normal direction to the first surface 10F and directions adjacent thereto.

As shown in Fig. 7, at a flat portion of the first surface 10F, that is, a portion where the bottomed sections 12 and the penetrating sections 13 are not present, diffuse reflection is not likely to occur. Accordingly, most of a reflected light Ir from the incident light Io is components of specular reflection light. Therefore, the reflected light Ir is unlikely to be incident on the camera 50, so the flat portion of the first surface 10F is observed as a dark area in the captured image.

As shown in Fig. 8, at a portion where the bottomed section 12 is present, the incident light Io is reflected by diffuse reflection at an inner surface of the bottomed section 12. Accordingly, part of the scattered light Id is incident on the camera 50. Therefore, in the captured image, a portion where the bottomed section 12 is present is observed as a bright area.

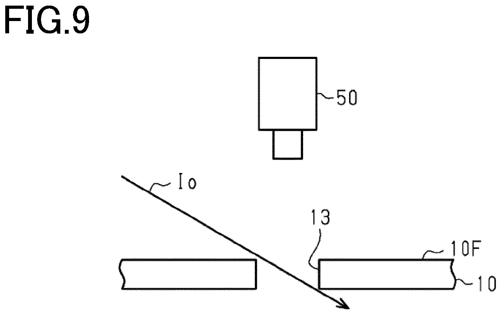

As shown in Fig. 9, at a portion where the penetrating section 13 is present, most of the incident light Io reaches the black plate 60 underlying the skin-adhesive film 10 through the penetrating section 13 since the penetrating section 13 does not have a bottom, and is absorbed by the black plate 60. Therefore, in the captured image, a portion where the penetrating section 13 is present is observed as a dark area.

As described above, in the image captured by the camera 50, a portion where the bottomed section 12 is present appears with a brightness different from the other portions. Thus, the area ratio Ra of the bottomed section 12 can be calculated by image analysis.

On the other hand, in the image captured by the digital camera according to the above method, it is difficult to identify the penetrating section 13. Therefore, the area ratio Rb of the penetrating section 13 is measured by using an image obtained by an electron microscope. In measuring the area ratio Rb of the penetrating sections 13 as well, a total area of the penetrating sections 13 in a binarized image is used as the total area occupied by the bottomed sections 13. The binarized image refers to an image obtained by binarizing a captured image of an observation region, which is a part of the first surface 10F, by a discriminant analysis method. Further, the above length Ls of the penetrating section 13 in the lateral direction and the length L1 in the longitudinal direction are also measured by using a binarized image.

Specifically, the area ratio Rb of the penetrating sections 13 is measured by the following procedure.
1) A surface on one side of a double-sided tape which uses a plastic foam (manufactured by Nichiban Co., Ltd.: Nice Tack Sponge double-sided tape, sponge type, NW-P15) is bonded to a thick paper, which is in turn punched with a two-hole punch to form an aperture. The aperture is formed to have a diameter of 6 mm. Then, a protective film on the other side of the double-sided tape is removed, and the second surface 10R of the skin-adhesive film 10 in the transfer sheet 20 is bonded to the double-sided tape.
2) The support substrate 21 of the transfer sheet 20 in the structure formed in the above 1) is moistened with water and removed from the transfer sheet 20. Then, the structure is dried for 1 day. Thus, a sample for measuring the area ratio Rb is obtained.
3) The sample formed in the above 2) is cut into a square piece with 8 mm sides, which mainly includes a portion of the skin-adhesive film 10 exposed through the aperture of the double-sided tape. The cut out sample is fixed to a sample table, and the first surface 10F of the skin-adhesive film 10 is imaged by using a field emission scanning electron microscope (manufactured by Toyo Corporation: SIGMA 500). In the captured image, a portion where the penetrating section 13 is present is observed as a black area.
4) The image captured in the above 3) is subjected to image processing in the same manner as in the procedure for measuring the area ratio Ra of the bottomed section 12 as described in 4) to obtain the area ratio Rb of the penetrating sections 13. That is, the above image is subjected to sharpening and binarizing to obtain a binarized image. The binarized image is subjected to particle analysis to thereby obtain the area ratio Rb of the penetrating sections 13. In addition, various conditions for the size and the image processing of the observation region are the same as those with the area ratio Ra of the bottomed section 12.

A sum of the area ratio Ra of the bottomed sections 12 and the area ratio Rb of the penetrating sections 13 obtained as described above is the area ratio Rt of the recesses 11.

The ratio of the recesses 11 to the first surface 10F contributes to adhesion of the substance to the first surface 10F and permeability of the substance through the skin-adhesive film 10. In the present embodiment, distortion of the skin-adhesive film 10 is not included in the recesses 11.

When the area ratio Rt is 0%, the first surface 10F has very high smoothness. A cosmetic, when applied to the first surface 10F having very high smoothness, adheres only with difficulty. The reason for this seems to be that the first surface 10F does not have asperities sufficient to hold the cosmetic on the first surface 10F. Further, the first surface 10F having high smoothness means that the skin-adhesive film 10 is highly dense. The denser the skin-adhesive film 10, the lower the permeability of the skin-adhesive film 10. Accordingly, the skin-adhesive film 10 adhered to the skin is highly effective in suppressing exudation of sebum. On the other hand, evaporation of water vapor from the skin is suppressed. Accordingly, when the skin-adhesive film 10 remains adhered to the skin for a long period of time, the user tends to feel discomfort due to humidity on the skin.

On the other hand, the greater the area ratio Rt, the higher the adhesion of cosmetics to the first surface 10F. The reason for this seems to be that the first surface 10F have asperities sufficient to hold the cosmetic on the first surface 10F. Further, the greater the area ratio Rt, the higher the permeability of the skin-adhesive film 10. Accordingly, when the skin-adhesive film 10 is adhered to the skin, the user is less likely to feel discomfort due to humidity on the skin, while the effect of suppressing exudation of sebum is reduced. In addition, the greater the area ratio Rt, the lower the strength of the skin-adhesive film 10. Accordingly, the skin-adhesive film 10 is susceptible to tearing, which reduces ease of handling of the skin-adhesive film 10.

When the area ratio Rt is 0.05% or more, the first surface 10F permits good adhesion of cosmetics, and good evaporation of water vapor. On the other hand, when the area ratio Rt is 25% or less, exudation of sebum is successfully reduced. Therefore, when the area ratio Rt is 0.05% or more and 25% or less, it is possible to balance the suppression of permeation of sebum with the improvement in adhesion of cosmetics and permeability of water vapor. Further, when the area ratio Rt is 25% or less, the skin-adhesive film 10 has good strength, which increases ease of handling of the skin-adhesive film 10.

Moreover, the greater the ratio of the penetrating sections 13 to the plurality of recesses 11, the higher the permeability and the higher the adhesion of cosmetics. When the area ratio Rt is 0.05% or more and 25% or less, both the evaporation of water vapor and the exudation of sebum are good, and the adhesion of cosmetics is also good, regardless of the ratio between the bottomed sections 12 and the penetrating sections 13. On the other hand, the greater the ratio of the penetrating sections 13, the lower the strength of the skin-adhesive film 10. In order to increase the strength of the skin-adhesive film 10, the ratio of the bottomed sections 12 to the plurality of recesses 11 is preferably high. That is, the ratio of the total area occupied by the bottomed sections 12 to the total area occupied by the recesses 11 per unit area is preferably larger than 50%. In other words, the total area occupied by the recesses 12 per unit area is preferably larger than the total area occupied by the bottomed sections 13. Further, the number of the bottomed sections 12 per unit area is preferably larger than the number of the penetrating sections 13.

The strength of the skin-adhesive film 10 in the present embodiment indicates a breaking strength, and is measured by the following procedures.
1) A double-sided tape (manufactured by Nichiban Co., Ltd.: Nice Tack Sponge double-sided tape, sponge type, NW-P15) is bonded to a thick paper having 1 mm thickness. Then, the thick paper to which the double-sided tape is bonded is punched with a two-hole punch to form an aperture. The aperture is formed to have a diameter of 6 mm.
2) The thick paper, which is punched in the above 1), is cut with scissors to form a square piece with 20 mm sides, which mainly includes a portion exposed through the aperture formed with a punch.
3) A protective film on one side of the double-sided tape in the structure formed in the above 2) is removed, and the second surface 10R of the skin-adhesive film 10 in the transfer sheet 20 is bonded to the double-sided tape. Then, a moistened cotton swab is pressed against the support substrate 21 of the transfer sheet 20 for moistening, and the support substrate 21 is removed by 180° peeling. Thus, a sample in which the skin-adhesive film 10 is bonded to the thick paper by the double-sided tape is obtained.
4) In the sample formed in the above 3), a protective film on the other side of the double-sided tape is removed. Then, the sample is fixed to a cylindrical support table mounted on a table-top compression and tensile tester (manufactured by Shimazu Corporation: EZ-Test, Load cell: 100N).
5) A stage of the table-top compression and tensile tester is moved so that a cylindrical indenter having 3 mm diameter is positioned above the center of the 6 mm diameter aperture formed in the thick paper. Then, the cylindrical indenter is pressed against the skin-adhesive film 10 of the sample at a compression speed of 10 mm/min. A strength (mN) at the time when the skin-adhesive film 10 is ruptured is measured to obtain a breaking strength.

### [Method for Producing Skin-Adhesive Film And Transfer Sheet]

An example of a method for producing the skin-adhesive film 10 and the transfer sheet 20 will be described below. The method for producing the skin-adhesive film 10 and the transfer sheet 20 is not limited to the method described below as long as the skin-adhesive film 10 having the area ratio Rt of the recesses 11 in a range of 0.05% or more and 25% or less can be formed.

First, a skin-adhesive film 10 is formed on a surface of a film-formation substrate. The film-formation substrate may be formed of a resin sheet made of a thermoplastic resin, a heat-curable resin, or a water soluble resin.

Specifically, a coating solution in which a material for the skin-adhesive film 10 is dissolved is applied to a surface of the film-formation substrate to form a coating film. The coating film is then dried to form a skin-adhesive film 10. As a solvent for the above coating solution, a polar protic solvent or a polar aprotic solvent may be used depending on the properties of the material for the skin-adhesive film 10. Examples of the polar protic solvent include water, ethanol, isopropanol, and acetic acid. Examples of the polar aprotic solvent include ethyl acetate, butyl acetate, propyl acetate, methyl ethyl ketone, acetone, and dimethyl sulfoxide.

The method of applying a coating solution is not specifically limited, and any method for forming a thin film can be used. The method of applying a coating solution is preferably one of gravure coating, micro gravure coating, spin coating, spray coating, and electrospinning. Further, a drying temperature of the coating film is preferably a boiling point or higher of the solvent used for the above coating solution.

Subsequently, the support substrate 21 is brought into contact with a surface of the skin-adhesive film 10 on the film-formation substrate to transfer the skin-adhesive film 10 from the film-formation substrate to the support substrate 21. Known transfer methods such as peeling by suction and a method using a sacrificial film can be used. Thus, the transfer sheet 20 having the skin-adhesive film 10 is formed. If necessary, an outer shape of the transfer sheet 20 may be adjusted into a desired shape by punching or the like.

When the transfer sheet 20 having the protective layer is desired, a protective layer is provided on a surface of the skin-adhesive film 10 on a side opposite to that in contact with the support substrate 21.

A method of forming the recesses 11 is not particularly limited. For example, the recesses 11 may be formed in a thin film formed on the film-formation substrate before the skin-adhesive film 10 is formed. In this case, the recesses 11 can be formed by processing such as laser irradiation or pressing with a roll having a surface on which projections are formed. The laser irradiation range and the laser power, or the arrangement density of the projections on the roll and the height of the projections can be controlled to control formation of the bottomed sections 12 and the penetrating sections 13, and the area ratio of the recesses 11.

Alternatively, the recesses 11 may be formed during transfer of the thin film from the film-formation substrate to the support substrate 21 to form the skin-adhesive film 10. For example, the strength of suction during transfer and the surface shape of the support substrate 21 can be controlled to control formation of the bottomed sections 12 and the penetrating sections 13, and the area ratio of the recesses 11.

### [Method for Adhering Skin-Adhesive Film]

A method for adhering the skin-adhesive film 10, that is, a method for transferring the skin-adhesive film by using the transfer sheet 20 will be described below. In a method described below, a porous substrate is used as the support substrate 21, and the support substrate 21 is moistened in transfer of the skin-adhesive film 10 to the skin.

First, liquid such as water is supplied to a site on the skin where the skin-adhesive film 10 is to be adhered. A supplied liquid, which is liquid to be supplied to the skin, may be any liquid that can moisten the transfer sheet 20. Specifically, liquid containing water, or oil such as massage oil can be used. For example, water or cosmetics such as a lotion can be used as the supplied liquid.

Subsequently, the transfer sheet 20 is placed on the skin with the second surface 10R of the skin-adhesive film 10 being in contact with the site on the skin where the skin-adhesive film 10 is to be adhered. The support substrate 21 of the transfer sheet 20 is pressed with a finger so that the supplied liquid infiltrates into the support substrate 21. A load applied to the skin-adhesive film 10 when the transfer sheet 20 is pressed is preferably 10 g/cm² or more and 900 g/cm² or less. Further, for reducing damage to the skin, the load is more preferably 10 g/cm² or more and 20 g/cm² or less. The load in this range is sufficient to adhere the skin-adhesive film 10 to the skin.

Then, the support substrate 21 is removed from the skin-adhesive film 10. Thus, the skin-adhesive film 10 is adhered to the skin. Since the transfer sheet 20 is moistened, the support substrate 21 made of a porous substrate swells, or the supplied liquid infiltrates between the support substrate 21 and the skin-adhesive film 10, which facilitates removal of the support substrate 21 from the skin-adhesive film 10.

In the above transfer method, the supplied liquid is supplied to the skin before the transfer sheet 20 is placed on the skin. However, the supplied liquid may also be supplied to the transfer sheet 20 after the transfer sheet 20 is placed on the skin. Further, the support substrate 21 may also be removed from the skin-adhesive film 10 without moistening the transfer sheet 20. In this case, the support substrate 21 may not necessarily be a porous substrate.

After the skin-adhesive film 10 is adhered to the skin, cosmetics are applied to the skin-adhesive film 10. That is, cosmetics are applied to the first surface 10F of the skin-adhesive film 10. The type of cosmetics applied is not limited, and, for example, cosmetics for makeup such as foundations and cosmetics for sun care such as sun screens can be used.

### [Examples]

The skin-adhesive film of the first embodiment will now be described by using specific examples.

### <Production of Transfer Sheet>

DL-polylactic acid (manufactured by Musashino Chemical Laboratory, Ltd.) was dissolved in ethyl acetate to generate a coating solution for forming a skin-adhesive film at a solid content in the solution of 5% or more and 10% or less. The average molecular weight of the polylactic acid was a predetermined value selected from a range of 50,000 or more and 440,000 or less. The coating solution was applied to a PET sheet (manufactured by Toray Industries Inc.: Lumirror, S 10) as a film-formation substrate by using a wire bar to form a coating film. The coating film was heated in an oven for drying and curing to form a skin-adhesive film. The skin-adhesive film was formed with a dry thickness of a predetermined value, which is 100 nm or more and 400 nm or less. A heating temperature during drying was selected from a range of 70°C or more and 120°C or less.

Then, a non-woven fabric (manufactured by Futamura Chemical Co., Ltd.) as a support substrate was laminated on the skin-adhesive film, and the film-formation substrate was peeled off to transfer the skin-adhesive film from the film-formation substrate to the support substrate. The main component of the non-woven fabric was cellulose, and the basis weight was 20 g/m².

In the above production method, by varying whether the skin-adhesive film on the film-formation substrate was pressed by a roll having a surface on which projections are formed, the conditions for pressing, and the conditions for transfer of the skin-adhesive film from the film-formation substrate to the support substrate, eight test examples having different area ratios Rt of the recesses were obtained as transfer sheets of Test Examples 1-1 to 1-8.

For Test Examples 1-1 to 1-8, the area ratio Rt of the recesses and the breaking strength were measured according to the measurement method described in the above embodiment. In measurement of the area ratio Rt, an observation region was a rectangular region with a short side of 1.65 mm and a long side of 2.2 mm, and a threshold for binarization was determined by Otsu's discriminant analysis method.

### <Evaluation Method>

For each test example, the items described below were evaluated.

### (Adhesion of Cosmetics)

For each test example, three test pieces having a square shape with sides of 20 mm were prepared. An artificial leather (manufactured by Ideatex Japan, Co., Ltd.: Supplale) having a square shape with sides of 70 mm was provided, and a surface of the artificial leather was moistened with water. A second surface of the skin-adhesive film of the test piece was adhered to the artificial leather, and the support substrate was removed. Thus, a sample for each test piece was prepared. A powder foundation was applied to a target surface of the sample, which is a surface on which the skin-adhesive film was adhered. The powder foundation was applied by using a sponge in one direction from the upper left end to the upper right end of the target surface. The above step of applying the powder foundation to the sample was repeated four times while shifting downward so that the entire target surface was covered. The target surface was then rotated by 90 degrees and the powder foundation was again applied to the entire target surface in the same manner as described above. This step was repeated until the application starting from each of the four sides of the target surface to the lower side was completed. After the application of the foundation was completed, the target surface on the sample was imaged to calculate a difference in luminance of a portion where the skin-adhesive film is adhered relative to a reference value, which is a luminance of a portion where the skin-adhesive film is not adhered. The luminance differences of three samples in each test example were obtained, and an average of these was defined as a luminance difference of each test example.

A negative luminance difference indicated that the degree of adhesion of cosmetics to the first surface of the skin-adhesive film was lower than that to the portion where the skin-adhesive film is not adhered. The more negative the luminance difference, the lower the degree of adhesion of cosmetics. In evaluation of adhesion of cosmetics, a test example with the luminance difference more negative than or equal to -5 was rated as "poor," a test example with the luminance difference greater than -5 and smaller than or equal to 0 was rated as "good," and a test example with the luminance difference greater than 0 was rated as "excellent."

### (Permeability of Sebum)

A center portion of a square-shaped thick paper having sides of 75 mm was cut out to form a frame from the remaining peripheral portion having a 5 mm width. For each test example, the skin-adhesive film was adhered to the frame by using a double-sided tape, and the support substrate was removed. Thus, a test piece was prepared. Artificial sebum was applied to a polypropylene film at an undried thickness of 36 µm, and filter paper (manufactured by Kiriyama Glass Works Co.: Kiriyama funnel filter paper No. 5A) having a diameter of 60 mm was placed thereon. The test piece was press-fit to the filter paper to prepare a sample.

When 4 hours elapsed after the test piece was press-fit to the filter paper, a blotting sheet was pressed against the test piece to absorb sebum exuded on the skin-adhesive film in the test piece. An area of the blotting sheet where sebum was absorbed was calculated by image analysis. The area of exuded sebum thus calculated was divided by the area of a region to be observed to obtain a sebum permeation area ratio. In evaluation of permeability of sebum, a test example with sebum permeation area ratio greater than 10% was rated as "poor," a test example with sebum permeation area ratio greater than 5% and smaller than or equal to 10% was rated as "good," and a test example with sebum permeation area ratio smaller than or equal to 5% was rated as "excellent."

### (Permeability of Water Vapor)

### [Skin Humidity]

For each test example, a test piece having a square shape with sides of 30 mm was prepared. A skin surface on the forearm of a human on the same side as the palm of the hand was moistened with water. The second surface of the skin-adhesive film of the test piece was adhered to the moistened surface, and the support substrate was removed. After one hour, the skin-adhesive film was removed from the skin for sensory evaluation of the humidity on the skin, that is, the degree of humidity perceived. Feeling of discomfort due to humidity on the skin was evaluated as "poor," feeling of slight humidity was evaluated as "fair," and no feeling of humidity on the skin was evaluated as "good."

### [Stratum Corneum Water Content]

In order to supplement the sensory evaluation of the skin humidity, stratum corneum water content was measured in the evaluation of skin humidity. The stratum corneum water content at a site on the skin where the skin-adhesive film has been removed after adhesion for one hour was measured by using a stratum corneum moisture meter (manufactured by Delfin Technologies Ltd.: MSC1001). The stratum corneum water content in each test example was calculated relative to the measurement result of the stratum corneum water content at a site where the skin-adhesive film was not adhered, which was taken as 100.

### <Evaluation Results>

Table 1 shows the recess area ratio Rt, the ratio between the bottomed sections and the penetrating sections in the recesses, and the breaking strength in each test example. Further, Table 2 shows data and evaluation results obtained from the evaluation on adhesion of cosmetics, permeability of sebum, and skin humidity in each test example.

**[Table 1]**

| | Recess area ratio (%) | Ratio between bottomed sections and penetrating sections | | Breaking strength (mN) |
|---|---|---|---|---|
| | | Bottomed sections | Penetrating sections | |
| Test Example 1-1 | 0.02 | 100 | 0 | 91 |
| Test Example 1-2 | 0.57 | 100 | 0 | 80 |
| Test Example 1-3 | 3.95 | 99 | 1 | 60 |
| Test Example 1-4 | 6.83 | 98 | 2 | 80 |
| Test Example 1-5 | 9.11 | 99 | 1 | 56 |
| Test Example 1-6 | 9.60 | 99 | 1 | 53 |
| Test Example 1-7 | 22 | - | | 45 |
| Test Example 1-8 | 30 | 70 | 30 | 38 |

**[Table 2]**

| | Adhesion of cosmetics | | Permeability of sebum | | Skin humidity | | Overall evaluation |
|---|---|---|---|---|---|---|---|
| | Luminance difference | Evaluation | Sebum permeation area ratio (%) | Evaluation | Evaluation | Stratum corneum water content | |
| Test Example 1-1 | -7.8 | Poor | 1 | Excellent | Fair | 150 | Poor |
| Test Example 1-2 | -2.00 | Good | 1 | Excellent | Fair | 130 | Good |
| Test Example 1-3 | -4.50 | Good | 3 | Excellent | Fair | 120 | Good |
| Test Example 1-4 | 0.76 | Excellent | 7.5 | Good | Good | 100 | Good |
| Test Example 1-5 | 0.17 | Excellent | 5.5 | Good | Good | 100 | Good |
| Test Example 1-6 | 5.33 | Excellent | 9.6 | Good | Good | 100 | Good |
| Test Example 1-7 | 8.51 | Excellent | 10 | Good | Good | 100 | Good |
| Test Example 1-8 | 10.70 | Excellent | 15 | Poor | Good | 100 | Poor |

As seen from Tables 1 and 2, with an increase in the recess area ratio Rt, adhesion of cosmetics increases and feeling of humidity on the skin decreases, while the permeation of sebum is more likely to occur. On the other hand, with a decrease in the recess area ratio Rt, suppression of sebum permeation increases, while adhesion of cosmetics decreases and feeling of humidity on the skin tends to increase.

Specifically, in Test Example 1-1 having an area ratio Rt of less than 0.05%, adhesion of cosmetics is significantly lowered. On the other hand, in Test Examples 1-2 to 1-8 having an area ratio Rt of 0.05% or more, good adhesion of cosmetics is observed. Particularly, in Test Examples 1-4 to 1-8 having an area ratio Rt of 5% or more, adhesion of cosmetics is high.

Further, in evaluation of skin humidity, no test example shows discomfort due to skin humidity. Test Examples 1-1 to 1-3 having the area ratio Rt of less than 5% show slight discomfort due to skin humidity, while Test Examples 1-4 to 1-8 having the area ratio Rt of 5% or more show no discomfort due to skin humidity. In addition, it was found that there was a correlation between the sensory evaluation on the feeling of skin humidity and the stratum corneum water content, and the stratum corneum water content was higher in the cases showing discomfort due to skin humidity than in the cases showing no discomfort due to skin humidity.

Further, while Test Example 1-8 having the area ratio Rt of greater than 25% shows high permeation of sebum, Test Examples 1-1 to 1-7 having the area ratio Rt of 25% or less show that permeation of sebum is successfully suppressed. In particular, Test Examples 1-1 to 1-3 having the area ratio Rt of less than 5% show low permeation of sebum.

As seen from Table 1, the smaller the area ratio Rt, the higher the breaking strength.

Summarizing the above evaluations, good results were obtained for suppression of permeation of sebum through the skin-adhesive film, adhesion of cosmetics, and permeability of water vapor when the recess area ratio Rt is 0.05% or more and 25% or less.

As described in the above embodiment and examples, according to the skin-adhesive film and the transfer sheet according to the first embodiment, the following advantages can be obtained.
(1-1) Due to the area ratio Rt of the recesses 11 being 0.05% or more, good adhesion of cosmetics and permeability of water vapor are achieved. Further, due to the area ratio Rt of the recesses 11 being 25% or less, permeation of sebum is successfully suppressed. Accordingly, it is possible to balance the suppression of permeation of sebum with the improvement in adhesion of cosmetics and permeability of water vapor, and the skin-adhesive film 10 has increased utility in cosmetic applications.
   Further, regardless of the area ratio Rt, the skin-adhesive film 10 having a configuration in which the recesses 11 are provided has improved adhesion of cosmetics compared with the skin-adhesive film 10 having a configuration in which no recesses 11 are provided. That is, the skin-adhesive film 10, when adhered to the skin, can reduce sebum exudation compared with the case where the skin-adhesive film 10 is not adhered. When it is desired to improve adhesion of cosmetics to the skin-adhesive film 10, the problem can be solved by providing the skin-adhesive film 10 having a configuration in which the recesses 11 are provided.
(1-2) When the plurality of recesses 11 include the bottomed sections 12 and the penetrating sections 13, and a total area occupied by the bottomed sections 12 per unit area is larger than a total area occupied by the penetrating sections 13 per unit area as viewed in a direction perpendicular to the first surface 10F, the breaking strength of the skin-adhesive film 10 is increased compared with the case where a ratio of the area occupied by the penetrating sections 13 in the recesses 11 is higher.
(1-3) When the shape of a plurality of recesses 11 includes at least one selected from the group consisting of a circular, elliptical, linear, and rectangular shapes, formation of the recesses 11 and control of the area ratio Rt can be easily performed compared with the case where the recesses 11 have a complicated outer shape such as a polygonal shape.
(1-4) When a ratio of the length L1 of the recess 11 in the longitudinal direction to the length Ls in the lateral direction is 1 or more and 500 or less, and the length Ls in the lateral direction is 0.1 µm or more and 1 µm or less, the recess 11 has a size suitable for adhesion of cosmetics and permeation of water vapor.
(1-5) When the skin-adhesive film 10 is configured to be supported by the support substrate 21, ease of handling of the skin-adhesive film 10 can be increased.

Further, when the depth of the recess 11 is at least partially filled with the support substrate 21, it is possible to prevent deformation of the recess 11 and entry of foreign substances into the recess 11 until the support substrate 21 is removed from the skin-adhesive film 10. That is, the skin-adhesive film 10 having the recesses 11 can improve adhesion of cosmetics. When it is desired to hold the shape of the recesses 11 in the skin-adhesive film 10 until it is used, the problem can be solved by providing a configuration in which the depth of the recess 11 is at least partially filled with the support substrate 21.

### [Appendix]

The measure for solving the foregoing problems embraces the following items as technical concepts derived from the first embodiment.
(Item 1) A skin-adhesive film having an average thickness of 5 µm or less, including: a first surface; and a second surface, which is a surface opposite to the first surface, the second surface being configured to be adhered to a skin surface, wherein
   the skin-adhesive film has a plurality of recesses that are recessed from the first surface, and
   a ratio of the recesses identified by binarizing an image of the first surface by a discriminant analysis method, which is a ratio of a total area occupied by the recesses per unit area calculated based on the image binarized, is 0.05% or more and 25% or less.
   With this configuration, due to the area ratio of the recesses being 0.05% or more, good adhesion of cosmetics and permeability of water vapor are achieved. Further, due to the area ratio of the recesses being 25% or less, permeation of sebum is successfully suppressed. Accordingly, it is possible to balance the suppression of permeation of sebum with the improvement in adhesion of cosmetics and permeability of water vapor, and the skin-adhesive film has increased utility in cosmetic applications.
(Item 2) The skin-adhesive film according to Item 1, wherein each of the plurality of recesses is either a bottomed section having a bottom in the skin-adhesive film or a penetrating section penetrating through the skin-adhesive film, the plurality of recesses include the bottomed sections and the penetrating sections, and a total area occupied by the bottomed sections per unit area is larger than a total area occupied by the penetrating sections per unit area.
   With this configuration, the breaking strength of the skin-adhesive film is increased compared with the case where a ratio of the area occupied by the penetrating sections in the recesses is higher.
(Item 3) The skin-adhesive film according to Item 1 or 2, wherein the shape of the plurality of recesses as viewed in a direction perpendicular to the first surface includes at least one selected from the group consisting of a circular, elliptical, linear, and rectangular shapes.
   With this configuration, formation of the recesses and control of the area ratio can be easily performed compared with the case where the recesses have a complicated outer shape such as a polygonal shape.
(Item 4) The skin-adhesive film according to any one of Items 1 to 3, wherein a ratio of the length of the recess in the longitudinal direction to the length in the lateral direction as viewed in a direction perpendicular to the first surface is 1 or more and 500 or less, and the length in the lateral direction is 0.1 µm or more and 1 µm or less.
   With this configuration, the size of the recesses can be appropriate for adhesion of cosmetics and permeation of water vapor.
(Item 5) A transfer sheet including: the skin-adhesive film according to any one of Items 1 to 4; and a porous substrate that supports the first surface of the skin-adhesive film.

With this configuration, since the skin-adhesive film is supported by the porous substrate, ease of handling of the skin-adhesive film can be increased.

### (Second Embodiment)

An object of the second embodiment is to provide a skin-adhesive film and a transfer sheet with improved moisturizing properties to the skin. The following description will be described focusing on differences between the second embodiment and the first embodiment, and configuration that is the same as that of the first embodiment will be referred to by the same reference numbers and the description thereof will be omitted.

The skin-adhesive film 10 of the second embodiment has the same configuration as that of the skin-adhesive film 10 of the first embodiment shown in Fig. 1. That is, the skin-adhesive film 10 of the second embodiment includes a plurality of recesses 11. The plurality of recesses 11 may be composed of both the bottomed sections 12 and the penetrating sections 13, or may be composed of either the bottomed sections 12 or the penetrating sections 13. The thickness and materials of the skin-adhesive film 10 of the second embodiment are the same as those of the first embodiment.

In the second embodiment, a blockage ratio Br in the skin-adhesive film 10 having the plurality of recesses 11 is measured. The blockage ratio Br refers to a parameter indicating the permeability of a substance through the skin-adhesive film 10. Specifically, the blockage ratio Br is a ratio of blocking evaporation of water having a temperature equal to a human body temperature at a site where the skin-adhesive film 10 is provided relative to that at a site where the skin-adhesive film 10 is not provided. The blockage ratio Br is measured by the following method.
1) Warm water kept at 37°C in a water bath is circulated in a tight-box (manufactured by Sanplatec Corp.). A glass sample bottle is placed in the tight-box, and warm water at 37°C is introduced into the glass sample bottle to a position 4 cm vertically below an opening of the glass sample bottle.
2) A plastic plate in which an aperture having a diameter of 10 mm is formed is placed over the opening of the glass sample bottle described in the above 1). A PTFE membrane filter (manufactured by Merck Millipore Ltd., pore diameter: 10 µm, diameter: 25 mm, white, plain) is placed over the opening, and allowed to stand for 5 minutes.
3) After the step described in the above 2), a moisture evaporation amount at a position of the aperture in the plastic plate is measured above the membrane filter by using a transdermal moisture evaporation meter (manufactured by ASCH JAPAN Co., Ltd.: VAPO SCAN AS-VT100RS) using a probe set to a 10 mm diameter. The value thus measured is set as an initial value.
4) The transfer sheet 20 is placed on the membrane filter described in the above 2) with the second surface 10R of the skin-adhesive film 10 being in contact with the membrane filter. The transfer sheet 20 is pressed with a finger so that the transfer sheet 20 is adhered to the membrane filter. Then, the support substrate 21 is removed from the skin-adhesive film 10, which is allowed to stand for 5 minutes.
5) After the step described in the above 4), a moisture evaporation amount at a position of the aperture in the plastic plate is measured above the skin-adhesive film 10 as in the above 3) by using the above transdermal moisture evaporation meter. The value thus measured is set as a sample value. The blockage ratio Br is calculated by the formula: blockage ratio Br = (initial value - sample value)/initial value × 100.

When the blockage ratio Br is 5% or more, good moisturizing properties are obtained at a position where the skin-adhesive film 10 is adhered. The blockage ratio Br is preferably 10% or more for further improved moisturizing properties. Further, when the blockage ratio Br is excessively high, the user tends to feel discomfort due to humidity on the skin at a site where the skin-adhesive film 10 is adhered. Therefore, the blockage ratio Br is preferably 60% or less.

The blockage ratio Br has a correlation with the area ratio Rt of the recesses 11. That is, as the area ratio Rt of the recesses 11 increases, the blockage ratio Br decreases. When the area ratio Rt of the recesses 11 is 30% or less, the blockage ratio Br of 5% or more is likely to be achieved, that is, good moisturizing properties are obtained at a position where the skin-adhesive film 10 is adhered. Further, when the area ratio Rt of the recesses 11 is 20% or less, the blockage ratio Br of 10% or more is likely to be achieved, that is, moisturizing properties are further increased. In order to prevent the blockage ratio Br from excessively increasing, that is, in order to prevent discomfort due to humidity on the skin, the area ratio Rt of the recesses 11 is preferably 0.05% or more. The area ratio Rt of the recesses 11 is calculated in the same manner as in the first embodiment.

As with the first embodiment, the skin-adhesive film 10 of the second embodiment forms the transfer sheet 20 when laminated on the support substrate 21. Further, a method for producing the skin-adhesive film 10 and the transfer sheet 20 and a method for adhering the skin-adhesive film 10 of the second embodiment is the same as those of the first embodiment.

### [Examples]

The skin-adhesive film of the second embodiment will now be described by using specific examples.

### <Production of Transfer Sheet>

DL-polylactic acid (manufactured by Musashino Chemical Laboratory, Ltd.) was dissolved in ethyl acetate to generate a coating solution for forming a skin-adhesive film at a solid content in the solution of 5% or more and 10% or less. The average molecular weight of the polylactic acid was a predetermined value selected from a range of 50,000 or more and 440,000 or less. The coating solution was applied to a PET sheet (manufactured by Toray Industries Inc.: Lumirror, S10) as a film-formation substrate by using a wire bar to form a coating film. The coating film was heated in an oven for drying and curing to form a skin-adhesive film. The skin-adhesive film was formed with a dry thickness of a predetermined value, which is 100 nm or more and 400 nm or less. A heating temperature during drying was selected from a range of 70°C or more and 120°C or less.

Then, a non-woven fabric (manufactured by Futamura Chemical Co., Ltd.) as a support substrate was laminated on the skin-adhesive film, and the film-formation substrate was peeled off to transfer the skin-adhesive film from the film-formation substrate to the support substrate. The main component of the non-woven fabric was cellulose, and the basis weight was 20 g/m².

In the above production method, by varying whether the skin-adhesive film on the film-formation substrate was pressed by a roll having a surface on which projections are formed, varying the conditions for pressing, and varying the conditions for transfer of the skin-adhesive film from the film-formation substrate to the support substrate, ten test examples having different area ratios Rt of the recesses were obtained as transfer sheets of Test Examples 2-1 to 2-10.

For Test Examples 2-1 to 2-10, the area ratio Rt of the recesses was measured according to the measurement method described in the first embodiment. In measurement of the area ratio Rt, an observation region was a rectangular region with a short side of 1.65 mm and a long side of 2.2 mm, and a threshold for binarization was determined by Otsu's discriminant analysis method. For Test Examples 2-1 to 2-10, the blockage ratio Br was measured according to the measurement method described in the second embodiment.

### <Evaluation Method>

For each test example, the moisturizing properties were evaluated by the following procedure.
1) A test site on the forearm of a subject was washed with water. Then, the water was wiped off, and the test site was allowed to stand for 15 minutes.
2) A stratum corneum water content at the test site was measured by using a skin hydration measurement instrument (manufactured by Integral Corporation: courage-karaka Corneometer CM825). The measured value was set as an initial stratum corneum water content.
3) 200 µl of water was supplied to the test site. After water was sufficiently supplied to the test site, the water at a site where the test piece of the skin-adhesive film was not adhered was wiped off. The test piece of the skin-adhesive film, which was cut into a disc shape having a diameter of 30 mm, was adhered to a portion of the test site where the test piece of the skin-adhesive film was to be adhered.
4) When 1 hour had elapsed after the step described in the above 3), the test piece was removed. Then, the stratum corneum water content at the site where the test piece was adhered and the site where the test piece was not adhered were measured by using the above skin hydration measurement instrument. Each of the stratum corneum water content thus measured was compared with the above initial stratum corneum water content to obtain an increase in the stratum corneum water content from the initial stratum corneum water content. Test examples in which an increase in stratum corneum water content was larger in the site where the test piece was adhered than the site where the test piece was not adhered were evaluated as "good," and test examples in which an increase in stratum corneum water content is smaller in the site where the test piece was adhered than the site where the test piece was not adhered were evaluated as "poor."

### <Evaluation Results>

Table 3 shows the recess area ratio Rt, the blockage ratio Br, and the moisturizing properties in each test example.

**[Table 3]**

| | Recess area ratio (%) | Blockage ratio (%) | Moisturizing properties |
|---|---|---|---|
| Test Example 2-1 | 2 | 54 | Good |
| Test Example 2-2 | 4 | 40 | Good |
| Test Example 2-3 | 5 | 31 | Good |
| Test Example 2-4 | 7 | 29 | Good |
| Test Example 2-5 | 10 | 21 | Good |
| Test Example 2-6 | 13 | 15 | Good |
| Test Example 2-7 | 16 | 12 | Good |
| Test Example 2-8 | 18 | 12 | Good |
| Test Example 2-9 | 30 | 5 | Good |
| Test Example 2-10 | 87 | 0 | Poor |

As seen from Table 3, in Test Examples 2-1 to 2-9 in which the blockage ratio Br is 5% or more, an increase in stratum corneum water content was larger at the site where the test piece was adhered than the site where the test piece was not adhered, which shows that adhesion of the skin-adhesive film increases the moisturizing properties.

Further, in Test Examples 2-1 to 2-9 in which the recess area ratio Rt is 30% or less, an increase in stratum corneum water content was larger at the site where the test piece was adhered than the site where the test piece was not adhered, which shows that adhesion of the skin-adhesive film increases the moisturizing properties.

On the other hand, in Test Example 2-10 in which the blockage ratio Br is significantly lower than 5% and the recess area ratio Rt is significantly larger than 30%, an increase in stratum corneum water content was smaller at the site where the test piece was adhered than the site where the test piece was not adhered, which shows that the moisturizing properties are poor.

As described in the above embodiments and examples, according to the skin-adhesive film and the transfer sheet according to the second embodiment, the following advantages can be obtained in addition to the effect of (1-2), (1-3), (1-4), and (1-5) in the first embodiment.
(2-1) Due to the blockage ratio Br being 5% or more and 60% or less, good moisturizing properties are achieved by the skin-adhesive film 10. Accordingly, the skin-adhesive film 10 has increased utility in cosmetic applications.
(2-2) Due to the area ratio Rt of the recesses 11 being 0.05% or more and 30% or less, good moisturizing properties are achieved by the skin-adhesive film 10. Accordingly, the skin-adhesive film 10 has increased utility in cosmetic applications.
(2-3) Due to the blockage ratio Br being 5% or more and 60% or less and the area ratio Rt of the recesses 11 being 0.05% or more and 30% or less, good moisturizing properties are achieved by the skin-adhesive film 10. Accordingly, the skin-adhesive film 10 has increased utility in cosmetic applications.

### (Third Embodiment)

It has been proposed to provide adhesive films containing various particles in order to impart functions to adhesive films or enhance functions of the films. For example, JP 2014-227389 A discloses adding particles made of metal oxide and the like as a material for a film in order to provide a film with UV blocking function. Further, JP 2013-1661 A discloses to add particles such as titanium oxide and polymethyl methacrylate as a material for a film in order to improve the effect of concealing imperfections such as wrinkles by adhering the film.

Since skin-adhesive films are ultra-thin, the skin-adhesive film conforms to uneven texture of the skin and is closely fit to the skin when adhered to the skin. Accordingly, since a boundary between a site where the skin-adhesive film is adhered and the remaining part is not easily recognizable, a site where the skin-adhesive film is adhered is inconspicuous when cosmetics are applied on the skin-adhesive film, and the makeup can be natural. However, if it is difficult to apply cosmetics to the surface of the skin-adhesive film, the effect of the makeup will decrease. In order to overcome this problem, for example, JP 2016-140717 A discloses providing a sheet to be adhered to the skin via an adhesive layer with increased surface roughness by forming asperities on the sheet to thereby enhance adhesion of cosmetics.

In a skin-adhesive film having a surface on which fine asperities are formed for enhancement of adhesion of cosmetics, such asperities are formed of a number of projections and recesses dispersed on a surface of the film. In this case, important factors for enhancement of adhesion of cosmetics include not only the height of the asperities, but also the arrangement of the asperities in the directions parallel to the surface of the film. However, regarding features of the asperities suitable for enhancing adhesion of cosmetics, no knowledge focusing on both the height direction and directions parallel to the surface of the film has been reported yet.

An object of the third embodiment is to provide a skin-adhesive film and a transfer sheet with enhanced adhesion of cosmetics.

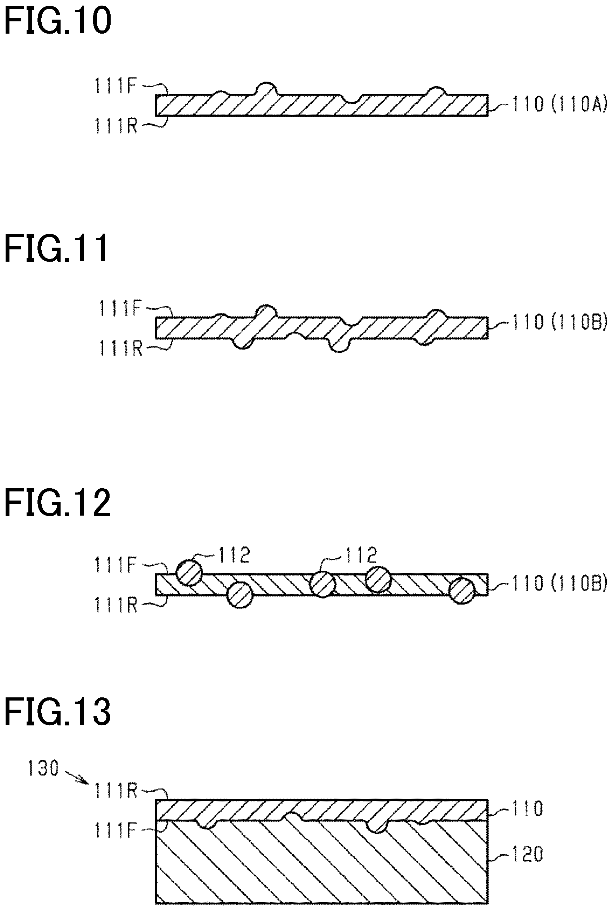

With reference to Figs. 10 to 13, the third embodiment of a skin-adhesive film and a transfer sheet will be described.

### [Configuration of Skin-Adhesive Film]

A skin-adhesive film 110 of the third embodiment has either a first type configuration or a second type configuration. First, the first type will now be described. Fig. 10 illustrates a cross-sectional structure of a first type skin-adhesive film 110A.

As shown in Fig. 10, the skin-adhesive film 110A has a first surface 111F, and a second surface 111R, which is a surface opposite to the first surface 111F. The second surface 111R is adhered to the skin. The first surface 111F is a surface exposed to the outside and also a surface to which cosmetics are applied when the skin-adhesive film 110A is adhered to the skin.

The first surface 111F has asperities and satisfies all the (a), (b), and (c) described below in terms of surface roughness.
(a) A first arithmetic average roughness Ra1, which is an arithmetic average roughness Ra of the first surface 111F, is 100 nm or more and 5 µm or less.
(b) A first ten-point average roughness Rz1, which is a ten-point average roughness Rz of the first surface 111F, is 1 µm or more and 50 µm or less.
(c) A first average interval Sm1, which is an average interval between peaks and valleys Sm on the first surface 111F, is 10 µm or more and 500 µm or less.

The first arithmetic average roughness Ra1, the first ten-point average roughness Rz1, and the first average interval Sm1 are all measured in accordance with JISB 0601-1994. Further, the average interval between peaks and valleys is the average interval between peaks.

Since the first surface 111F has the asperities, a surface area of the first surface 111F to which cosmetics can be adhered increases and constituents such as particles and the like of cosmetics are likely to be held by the asperities, compared with a case where the first surface 111F is flat. Therefore, adhesion of cosmetics to the first surface 111F is enhanced.

When the first arithmetic average roughness Ra1 is less than 100 nm, or when the first ten-point average roughness Rz1 is less than 1 µm, the roughness of the skin-adhesive film 110A in the thickness direction is too small to hold cosmetics by the asperities. Further, when the first average interval Sm1 is larger than 500 µm, the interval between peaks and valleys is too large to hold cosmetics by the asperities. On the other hand, when the first average interval Sm1 is less than 10 µm, the interval between peaks and valleys is too small to hold cosmetics by the asperities. A height and a density of the roughness on the first surface 111F sufficient to enhance adhesion of cosmetics to the first surface 111F are achieved when the first arithmetic average roughness Ra1 is 100 nm or more, the first ten-point average roughness Rz1 is 1 µm or more, and the first average interval Sm1 is 10 µm or more and 500 µm or less.

Cosmetics are adhered to the first surface 111F mainly by being held in a portion recessed relative to the first surface 111F. Accordingly, in order to enhance adhesion of cosmetics, such recesses are required to have a sufficient depth and a sufficient interval to hold the constituents such as particles of cosmetics in the recesses. Therefore, in the present embodiment, the degree of surface roughness that can enhance adhesion of cosmetics is defined by using the arithmetic average roughness Ra and the ten-point average roughness Rz, which are parameters regarding the size of roughness in the height direction, and the average interval between peaks and valleys Sm, which is a parameter regarding the density of roughness in the lateral direction, among various parameters defining surface roughness.

Due to ten-point average roughness Rz, in addition to the arithmetic average roughness Ra, being used to define the size of roughness in the height direction, a size of roughness more suitable for holding constituents such as particles of cosmetics can be defined. Further, the average interval between peaks and valleys Sm can be used to define an interval between peaks and valleys that is sufficient to hold the above constituents and enhance the uniform adhesion of cosmetics to the first surface 111F.

When the first arithmetic average roughness Ra1 is larger than 5 µm, or when the first ten-point average roughness Rz1 is larger than 50 µm, the skin-adhesive film 110A may be locally thin, which tends to reduce the strength of the skin-adhesive film 110A. Therefore, when the first arithmetic average roughness Ra1 is 5 µm or less and the first ten-point average roughness Rz1 is 50 µm or less, the skin-adhesive film 110A is prevented from being easily torn.

The surface shape of the first surface 111F satisfying the above (a) to (c) may be formed by a plurality of peaks on the first surface 111F, a plurality of valleys on the first surface 111F, or combinations of such peaks and valleys. The size of the projections and recesses may or may not be constant. Further, the projections and recesses may be arranged regularly or irregularly.

In the first type skin-adhesive film 110A, the surface roughness of the second surface 111R does not satisfy at least one of the conditions for the arithmetic average roughness Ra, the ten-point average roughness Rz, and the average interval Sm described in the above (a) to (c). For example, the second surface 111R has the asperities finer than those of the first surface 111F. That is, the second surface 111R satisfies at least one of the arithmetic average roughness Ra of less than 100 nm, the ten-point average roughness Rz of less than 1 µm, and the average interval between peaks and valleys Sm less than 10 µm.

Next, the second type will be described. Fig. 11 illustrates a cross-sectional structure of a second type skin-adhesive film 110B. The skin-adhesive film 110B has a first surface 111F on which cosmetics are to be applied, and a second surface 111R, which is a surface opposite to the first surface 111F, to be adhered to the skin.

The first surface 111F has the same configuration as the first type, that is, the first surface 111F satisfies all the (a), (b), and (c) described above in terms of surface roughness.

On the other hand, the second surface 111R of the second type has asperities, and satisfies all the (d), (e), and (f) described below in terms of surface roughness.
(d) A second arithmetic average roughness Ra2, which is an arithmetic average roughness Ra of the second surface 111R, is 100 nm or more and 5 µm or less.
(e) A second ten-point average roughness Rz2, which is a ten-point average roughness Rz of the second surface 111R, is 1 µm or more and 50 µm or less.
(f) A second average interval Sm2, which is an average interval between peaks and valleys Sm on the second surface 111R, is 10 µm or more and 500 µm or less.

The second arithmetic average roughness Ra2, the second ten-point average roughness Rz2, and the second average interval Sm2 are all measured in accordance with JIS B 0601-1994.

When the second surface 111R has asperities, the skin-adhesive film 110B tends to form fine gaps between the skin surface and the skin-adhesive film 110B while following the surface shape of the skin when the skin-adhesive film 110B is adhered to the skin. Accordingly, the moisture existing between the skin surface and the skin-adhesive film 110B is easily discharged through these fine gaps. As a result, adhesion of the skin-adhesive film 110B to the skin can be increased. In particular, when water such as lotion is supplied to the skin before the skin-adhesive film 110B is adhered, water is likely to exist between the skin surface and the skin-adhesive film 110B immediately after the skin-adhesive film 110B is adhered. Therefore, the asperities on the second surface 111R are advantageous in promoting discharge of water.

The height and density of the roughness on the second surface 111R sufficient to form gaps between the skin surface and the skin-adhesive film 110B are achieved when the second arithmetic average roughness Ra2 is 100 nm or more, the second ten-point average roughness Rz2 is 1 µm or more, and the second average interval Sm2 is 10 µm or more and 500 µm or less. Accordingly, good adhesion of the skin-adhesive film 110B to the skin can be obtained.

Further, when the second arithmetic average roughness Ra2 is 5 µm or less and the second ten-point average roughness Rz2 is 50 µm or less, a decrease in strength of the skin-adhesive film 110B is prevented.

The surface shape of the second surface 111R satisfying the above (d) to (f) may be formed by a plurality of peaks on the second surface 111R, a plurality of valleys on the second surface 111R, or combinations of such peaks and valleys. The size of the projections and recesses may or may not be constant. Further, the projections and recesses may be arranged regularly or irregularly.

As described above, the asperities on the first surface 111F have a function of holding cosmetics, and the asperities on the second surface 111R have a function of forming gaps between the skin and the skin-adhesive film 110B. In order to appropriately perform these functions, the asperities on the first surface 111F are preferably larger and rougher than the asperities of the second surface 111R. That is, the first arithmetic average roughness Ra1 is preferably larger than the second arithmetic average roughness Ra2, and the first ten-point average roughness Rz1 is preferably larger than the second ten-point average roughness Rz2, and the first average interval Sm1 is preferably larger than the second average interval Sm2. With this configuration, the first surface 111F has a surface roughness more suitable for holding cosmetics compared to the second surface 111R, and the second surface 111R has a surface roughness more suitable for forming gaps between the skin surface and the skin-adhesive film 110B compared to the first surface 111F. Accordingly, the skin-adhesive film 110B having a configuration in which the first surface 111F and the second surface 111R perform different functions can be obtained.

In the following description, a configuration of the skin-adhesive film 110 common to the first type and the second type will be described.

The skin-adhesive film 110 has adhesion to the skin. In other words, the skin-adhesive film 110 has a portion sufficiently thin to exhibit the above adhesion. Specifically, the skin-adhesive film 110 has a thin film portion, which is a portion having a thickness of 5 µm or less. When the thin film portion is 5 µm or less, the thin film portion itself can be adhered to the skin without using an adhesive layer.

In order to expand an area that is adhesive to the skin, a portion of the first surface 111F where the peaks are not formed and a portion of the second surface 111R where the peaks are not formed preferably have an average thickness of 10 nm or more and 5 µm or less, and more preferably 10 nm or more and 2 µm or less. Further, in order to enhance adhesion to the skin, increase conformability of the skin-adhesive film 110 to the surface shape of the skin, and reduce the discomfort that the user may feel when the skin-adhesive film 110 is adhered to the skin, a portion of the first surface 111F where the peaks are not formed and a portion of the second surface 111R where the peaks are not formed preferably have an average thickness of 150 nm or more and 1 µm and less. The average thickness is an average thickness, which is obtained by averaging the film thicknesses in the cross-section at five or more measurement points by using an electron microscope. The skin-adhesive film 110 may be configured as a single layer film, or may be configured as a multi-layered film.

The thinness to a degree that the skin-adhesive film 110 exhibits adhesion to the skin can be expressed in mass of the skin-adhesive film 110, instead of thickness. For example, when the density of the skin-adhesive film 110 is 1 g/cm³ or more and 3 g/cm³ or less, the mass per unit area of the skin-adhesive film 110 is 0.01 g/m² or more and 10 g/m² or less. The above mass is a mass of a portion of the skin-adhesive film 110 having an area of 1 m² in plan view of the first surface 111F. When the mass per unit area of the skin-adhesive film 110 is within the above range, the skin-adhesive film 110 itself can be adhered to the skin without using an adhesive layer.

The material for forming the skin-adhesive film 110 is not particularly limited. The material of the skin-adhesive film 110 is preferably a resin having low toxicity, and low in degree of causing skin irritation and skin sensitization. Examples of the material for the skin-adhesive film 110 include natural polymers such as hyaluronic acid, fibroin, and chitosan, and ester resins such as polylactic acid, polyglycolic acid, and polycaprolactone, and copolymer resins thereof. Further, resins for use as film-formers in cosmetics may also be used as the material for the skin-adhesive film 110. Examples of such resins include acrylic resin, silicone, and copolymer resins thereof, and cellulose derivatives such as cellulose acetate, cellulose acetate propionate, and cellulose acetate butyrate. Further, examples of the material for the skin-adhesive film 110 include resins that are commonly used as materials for medical products, such as polycarbonate, cycloolefin copolymer, styrene-butadiene elastomer, polyimide, acrylic resin, urethane resin, and copolymer resins thereof. The molecular weight of the materials for the skin-adhesive film 110 is not particularly limited. For example, the skin-adhesive film 110 may be made of one type of material having a predetermined average molecular weight, or may also be made of a plurality of types of materials having different average molecular weights.

In addition, the skin-adhesive film 110 may contain functional materials that perform a predetermined function for the skin. Examples of the functional materials include cosmetics and cosmetic ingredients used for skin care, such as moisturizing creams and beauty essence, dye, drugs, proteins, and enzymes. The skin-adhesive film 110 may contain one type of functional material, or may contain two or more types of functional materials.

The asperities on the first surface 111F of the first type and the asperities on the second surface 111R of the second type may be formed by shaping the surface of the skin-adhesive film 110 or may be formed by conforming to the shape of particles dispersed in the skin-adhesive film 110.

Specific examples of the above methods of shaping the skin-adhesive film 110 include embossing a thin film used as the skin-adhesive film 110 with a heated plate having a pattern of asperities, pattern printing, and applying a skin-adhesive film 110 to a film-formation substrate having a surface on which asperities are formed.

Fig. 12 illustrates the second type skin-adhesive film 110B having a shape that follows the shape of particles. The asperities on the first surface 111F are formed by projections formed on the first surface 111F by particles 112 protruding from the first surface 111F. Similarly, the asperities on the second surface 111R are formed by projections formed on the second surface 111R by the particles 112 protruding from the second surface 111R.

The particles 112 are made of, for example, inorganic oxides, organic oxides, or metal oxides. The particles 112 are preferably made of chemically stable substances. The average particle size of the particle 112 is preferably 20 µm or less. In view of reducing precipitation of the particles 112, the average particle size is preferably 5 µm or less. The content of the particles 112 is preferably 1% or more and 40% or less, and more preferably 10% or less to the resin solid content in a coating solution for forming the skin-adhesive film 110.

Further, the asperities on the first surface 111F and the second surface 111R may also be formed by both shaping the surface of the skin-adhesive film 110 and containing the particles 112.

### [Configuration of Transfer Sheet]

A transfer sheet 130 used for adhering the skin-adhesive film 110 to the skin will be described. As shown in Fig. 13, the transfer sheet 130 includes the skin-adhesive film 110, and a support substrate 120 that supports the skin-adhesive film 110. Although Fig. 13 illustrates the transfer sheet 130 supporting the first type skin-adhesive film 110A, the transfer sheet 130 may also be provided with the second type skin-adhesive film 110B.

The support substrate 120 is in contact with the first surface 111F of the skin-adhesive film 110. The asperities on the first surface 111F may be or may not be filled with the support substrate 120. The support substrate 120 has a function of reducing deformation of the skin-adhesive film 110 during storage of the transfer sheet 130 and transport of the skin-adhesive film 110 onto the skin in use of the transfer sheet 130. Since the skin-adhesive film 110 is supported by the support substrate 120, ease of handling of the skin-adhesive film 110 can be increased.

The support substrate 120 is preferably a porous substrate. The porous substrate has a large number of fine pores inside the substrate so that liquid can infiltrate or penetrate through the pores. Examples of the porous substrate that can be used as the support substrate 120 include sheets made of fibrous materials such as non-woven fabric, paper, knitted fabric, and woven fabric, and resin sheets having a structure with gaps, such as a mesh. Among these substrates, a non-woven fabric is preferably used due to the ability to allow quick absorption and diffusion of water. Examples of fibers forming the non-woven fabric include natural fibers such as cotton, hemp, wool, and pulp, semi-synthetic fibers such as rayon, and synthetic fibers such as polyvinyl alcohol, polyacrylates. Among the above fibers, natural fibers, especially pulps, are preferably used. The non-woven fabric may be formed of a single type of fiber, or two or more types of fibers.

The methods for manufacturing the non-woven fabric used as the support substrate 120 are not specifically limited. The non-woven fabric may be manufactured by one of the methods including spunlacing, spunbonding, needle-punching, melt blowing, air-laying, flash spinning, and resin adhesion. The non-woven fabric used as the support substrate 120 preferably has a basis weight of 10 g/m² or more and 150 g/m² or less, and more preferably 20 g/m² or more and 50 g/m² or less in view of texture for good tactile feel. The basis weight of the non-woven fabric refers to a mass per unit area of the non-woven fabric.

Further, the support substrate 120 is not limited to the porous substrate, and may also be formed of a substrate such as a resin sheet or metal foil having no pore inside.

The transfer sheet 130 may further include a protective layer that covers the second surface 111R of the skin-adhesive film 110. The protective layer has a function of protecting the second surface 111R. The protective layer is preferably made of a porous substrate. The porous substrate for forming the protective layer may be made of the same material as those of the porous substrate for forming the support substrate 120. The protective layer and the support substrate 120 may be formed of the same type of porous substrates, or may be formed of different types of porous substrates. In addition, the protective layer is not limited to a porous substrate, and may also be formed of a substrate such as a resin sheet or metal foil having no pores inside.

### [Method for Producing Skin-Adhesive Film And Transfer Sheet]

An example of a method for producing the skin-adhesive film 110 and the transfer sheet 130 will be described below. The method for producing the skin-adhesive film 110 and the transfer sheet 130 is not limited to the method described below as long as the first surface 111F and the second surface 111R having surface roughness satisfying the conditions applied to the above first type or the second type.

First, the skin-adhesive film 110 is formed on a surface of a film-formation substrate. The film-formation substrate may be formed of a resin sheet made of a thermoplastic resin, a heat-curable resin, or a water soluble resin. Specifically, a coating solution in which a material for the skin-adhesive film 110 is dissolved in a solvent is applied to a surface of the film-formation substrate to form a coating film. The coating film is then dried to form a skin-adhesive film 110. As the above solvent, a polar protic solvent or a polar aprotic solvent may be used depending on the properties of the material for the skin-adhesive film 110. Examples of the polar protic solvent include water, ethanol, isopropanol, and acetic acid. Examples of the polar aprotic solvent include ethyl acetate, butyl acetate, propyl acetate, methyl ethyl ketone, acetone, and dimethyl sulfoxide.

The method of applying a coating solution is not specifically limited. The method of applying a coating solution is preferably one of direct gravure coating, micro gravure coating, spin coating, and spray coating. Further, a drying temperature of the coating film is preferably a boiling point or higher of the solvent used for the above coating solution.

Subsequently, the support substrate 120 is brought into contact with the first surface 111F, which is a surface of the skin-adhesive film 110 on the film-formation substrate to transfer the skin-adhesive film 110 from the film-formation substrate to the support substrate 120. Known transfer methods such as peeling by suction and a method using a sacrificial film can be used.

As described above, the asperities on the first surface 111F and the second surface 111R are formed by shaping the surface of the skin-adhesive film 110 and mixing particles into the above coating solution.

Thus, the transfer sheet 130 having the skin-adhesive film 110 is formed. If necessary, an outer shape of the transfer sheet 130 may be adjusted into a desired shape by punching or the like.

When the transfer sheet 130 having the protective layer is desired, a protective layer is provided on the second surface 111R, which is a surface of the skin-adhesive film 110 on a side opposite to that in contact with the support substrate 120.

### [Method for Adhering Skin-Adhesive Film]

A method for adhering the skin-adhesive film 110, that is, a method for transferring a skin-adhesive film which uses the transfer sheet 130 will be described below. In a method described below, a porous substrate is used as the support substrate 120, and the support substrate 120 is moistened in transfer of the skin-adhesive film 110 to the skin.

First, liquid such as water is supplied to a site on the skin where the skin-adhesive film 110 is to be adhered. A supplied liquid, which is liquid to be supplied to the skin, may be any liquid that can moisten the transfer sheet 130. Specifically, liquid containing water, or oil such as massage oil can be used. For example, water or cosmetics such as a lotion can be used as the supplied liquid.

Subsequently, the transfer sheet 130 is placed on the skin with the second surface 111R of the skin-adhesive film 110 being in contact with the site on the skin where the skin-adhesive film 110 is to be adhered. The support substrate 120 of the transfer sheet 130 is pressed with a finger so that the supplied liquid infiltrates into the support substrate 120. A load applied to the skin-adhesive film 110 when the transfer sheet 130 is pressed is preferably 10 g/cm² or more and 900 g/cm² or less. Further, for reducing damage to the skin, the load is more preferably 10 g/cm² or more and 20 g/cm² or less. The load in this range is sufficient to adhere the skin-adhesive film 110 to the skin.

Then, the support substrate 120 is removed from the skin-adhesive film 110. Thus, the skin-adhesive film 110 is adhered to the skin. Since the transfer sheet 130 is moistened, the support substrate 120 made of a porous substrate swells, or the supplied liquid infiltrates between the support substrate 120 and the skin-adhesive film 110, which facilitates removal of the support substrate 120 from the skin-adhesive film 110.

In the above transfer method, the supplied liquid is supplied to the skin before the transfer sheet 130 is placed on the skin. However, the supplied liquid may also be supplied to the transfer sheet 130 after the transfer sheet 130 is placed on the skin. Further, the support substrate 120 may also be removed from the skin-adhesive film 110 without moistening the transfer sheet 130. In this case, the support substrate 120 may not be necessarily a porous substrate.

After the skin-adhesive film 110 is adhered to the skin, cosmetics are applied to the skin-adhesive film 110. That is, cosmetics are applied to the first surface 111F of the skin-adhesive film 110. The type of cosmetics applied is not limited, and, for example, cosmetics for makeup such as foundations and cosmetics for sun care such as sun screens can be used.

### [Examples]

The transfer sheet of the third embodiment will be described by using specific examples and comparative examples.

### (Example 3-1)

DL-polylactic acid (manufactured by Musashino Chemical Laboratory, Ltd.) was dissolved in ethyl acetate to generate a coating solution for forming a skin-adhesive film. A polyethylene terephthalate sheet having a surface made of a silicone release layer was used as a film-formation substrate, and the coating solution was applied to the film-formation substrate by using a wire bar to form a coating film. The coating film was then heated in an oven for drying and curing. In this step, the coating film was formed to have a dry density of 1 g/cm³ or more and 3 g/cm³ or less, and a mass per unit area of 0.1 g/m² or more and 10 g/m² or less. A heating temperature during drying was selected from a range of 70°C or more and 120°C or less. Then, a metal plate having a surface on which asperities are formed was pressed against the cured film to transfer the asperities on the metal plate to the surface of the film. For the surface roughness of the surface of the metal plate, the arithmetic average roughness Ra was 250 nm, the ten-point average roughness Rz was 1.2 µm, and the average interval between peaks and valleys Sm was 64 µm. Thus, a skin-adhesive film of Example 3-1 having asperities on the first surface was obtained. The first surface had asperities derived from the asperities on the surface of the metal plate.

Then, a non-woven fabric (manufactured by Futamura Chemical Co., Ltd.) as a support substrate was laminated on the skin-adhesive film, and the film-formation substrate was peeled off to transfer the skin-adhesive film from the film-formation substrate to the support substrate. The main component of the non-woven fabric was cellulose, and the basis weight was 20 g/m². Thus, a transfer sheet of Example 3-1 was obtained.

### (Example 3-2)

A skin-adhesive film and a transfer sheet of Example 3-2 were prepared in the same manner as in Example 3-1 except that a metal plate with a surface having an arithmetic average roughness Ra of 300 nm, a ten-point average roughness Rz of 2.2 µm, and an average interval between peaks and valleys Sm of 91 µm was used.

### (Example 3-3)

A skin-adhesive film and a transfer sheet of Example 3-3 were prepared in the same manner as in Example 3-1 except that a metal plate with a surface having the arithmetic average roughness Ra of 340 nm, the ten-point average roughness Rz of 2.8 µm, and the average interval between peaks and valleys Sm of 115 µm was used.

### (Example 3-4)

DL-polylactic acid (manufactured by Musashino Chemical Laboratory, Ltd.) was dissolved in ethyl acetate to generate a coating solution for forming a skin-adhesive film. A polyethylene terephthalate sheet having a surface made of a silicone release layer was used as a film-formation substrate, and the coating solution was applied to the film-formation substrate by using a wire bar to form a coating film. The surface of the film-formation substrate was treated to produce a matte finish. For the surface roughness of the film-formation substrate, the arithmetic average roughness Ra was 120 nm, the ten-point average roughness Rz was 0.7 µm, and the average interval between peaks and valleys Sm was 60 µm. The coating film was then heated in an oven for drying and curing. In this step, the coating film was formed to have a dry density of 1 g/cm³ or more and 3 g/cm³ or less, and a mass per unit area of 0.1 g/m² or more and 10 g/m² or less. A heating temperature during drying was selected from a range of 70°C or more and 120°C or less.

Then, a metal plate having a surface on which asperities are formed was pressed against the cured film to transfer the asperities on the metal plate to the surface of the film. For the surface roughness of the surface of the metal plate, the arithmetic average roughness Ra was 250 nm, the ten-point average roughness Rz was 1.2 µm, and the average interval between peaks and valleys Sm was 64 µm.

Thus, a skin-adhesive film of Example 3-4 having asperities on the first and second surfaces was obtained. The first surface had asperities derived from the asperities on the metal plate, and the second surface had asperities derived from the asperities on the film-formation substrate.

Then, a non-woven fabric (manufactured by Futamura Chemical Co., Ltd.) as a support substrate was laminated on the skin-adhesive film, and the film-formation substrate was peeled off to transfer the skin-adhesive film from the film-formation substrate to the support substrate. The main component of the non-woven fabric was cellulose, and the basis weight was 20 g/m². Thus, a transfer sheet of Example 3-4 was obtained.

### (Example 3-5)

A skin-adhesive film and a transfer sheet of Example 3-5 were prepared in the same manner as in Example 3-4 except that a film-formation substrate and a metal plate having different surface roughness from those of Example 3-4 were used.

For the surface roughness of the surface of the film-formation substrate used in Example 3-5, the arithmetic average roughness Ra was 160 nm, the ten-point average roughness Rz was 1.3 µm, and the average interval between peaks and valleys Sm was 81 µm. For the surface roughness of the surface of the metal plate used in Example 3-5, the arithmetic average roughness Ra was 300 nm, the ten-point average roughness Rz was 2.2 µm, and the average interval between peaks and valleys Sm was 91 µm.

### (Example 3-6)

A skin-adhesive film and a transfer sheet of Example 3-6 were prepared in the same manner as in Example 3-4 except that a film-formation substrate and a metal plate having different surface roughness from those of Example 3-4 were used.

For the surface roughness of the surface of the film-formation substrate used in Example 3-6, the arithmetic average roughness Ra was 205 nm, the ten-point average roughness Rz was 2.2 µm, and the average interval between peaks and valleys Sm was 100 µm. For the surface roughness of the surface of the metal plate used in Example 3-6, the arithmetic average roughness Ra was 340 nm, the ten-point average roughness Rz was 2.8 µm, and the average interval between peaks and valleys Sm was 115 µm.

### (Example 3-7)

DL-polylactic acid (manufactured by Musashino Chemical Laboratory, Ltd.) was dissolved in ethyl acetate to generate a coating solution at a solid content in the solution of 10%. Then, titanium oxide having an average particle size of 3 µm was added to the coating solution at percentage of 30% based on the weight of DL-polylactic acid, and dispersed therein.

A polyethylene terephthalate sheet having a surface made of a silicone release layer was used as a film-formation substrate, and the coating solution was applied to the film-formation substrate by using a wire bar to form a coating film. The coating film was then heated in an oven for drying and curing. In this step, the coating film was formed to have a dry density of 1 g/cm³ or more and 3 g/cm³ or less, and a mass per unit area of 0.1 g/m² or more and 10 g/m² or less. A heating temperature during drying was selected from a range of 70°C or more and 120°C or less. Thus, a skin-adhesive film of Example 3-7 having asperities on the first and second surfaces was obtained. The first surface and second surfaces had asperities derived from the particles contained therein.

Then, a non-woven fabric (manufactured by Futamura Chemical Co., Ltd.) as a support substrate was laminated on the skin-adhesive film, and the film-formation substrate was peeled off to transfer the skin-adhesive film from the film-formation substrate to the support substrate. The main component of the non-woven fabric was cellulose, and the basis weight was 20 g/m². Thus, a transfer sheet of Example 3-7 was obtained.

### (Comparative Example 3-1)

A skin-adhesive film and a transfer sheet of Comparative Example 3-1 were prepared in the same manner as in Example 3-1 except that formation of asperities on the film surface by using a metal plate was not performed.

### <Evaluation Method>

For the skin-adhesive films of the above examples and comparative examples, the items described below were evaluated.

### (Surface Roughness)

For each of the first surface and the second surface of the skin-adhesive films of the respective examples and comparative examples, parameters of the arithmetic average roughness Ra, the ten-point average roughness Rz, and the average interval between peaks and valleys Sm were measured. In measurement, test pieces having a square shape with sides of 20 mm were prepared from the skin-adhesive films of the examples and comparative examples. A smooth substrate surface was moistened with water, and a surface of the test piece on a side opposite to a surface to be measured was adhered to the substrate. The test piece was dried for approximately several to 24 hours, and the above parameters were measured. The measurement was performed by using a surface roughness tester (manufactured by Tokyo Seimitsu Co., Ltd.: Surfcom 130A) in accordance with JIS B 0601:1994.

### (Adhesion of Cosmetics)

From the transfer sheets of the respective examples and comparative examples, test pieces having a square shape with sides of 20 mm were prepared. An artificial leather (manufactured by Ideatex Japan, Co., Ltd.: Supplale) having a square shape with sides of 70 mm was provided, and a surface of the artificial leather was moistened with water. A second surface of the skin-adhesive film of the test piece was adhered to the artificial leather, and the support substrate was removed. Thus, a sample for each test piece was prepared. A powder foundation was applied to a target surface of the sample, which is a surface on which the skin-adhesive film was adhered. The powder foundation was applied by using a sponge in one direction from the upper left end to the upper right end of the target surface. The above step of applying the powder foundation to the sample was repeated four times while shifting downward so that the entire target surface was covered. The target surface was then rotated by 90 degrees and the powder foundation was again applied to the entire target surface in the same manner as described above. This step was repeated until the application starting from each of the four sides of the target surface to the lower side was completed. After the application of the foundation was completed, the target surface on the sample was imaged to calculate a difference in luminance of a portion where the skin-adhesive film is adhered relative to a reference value, which is a luminance of a portion where the skin-adhesive film is not adhered. The larger the luminance difference, the higher the degree of adhesion of cosmetics.

### (Skin Adhesion)

For the skin-adhesive films of the examples and comparative examples, skin adhesion was evaluated by measuring the adhesion strength by the following procedure.
1) From the transfer sheet, a test piece having a square shape with sides of 40 mm was prepared. An artificial leather (manufactured by Ideatex Japan, Co., Ltd.: Supplale) having a square shape with sides of 70 mm was provided, and a surface of the artificial leather was moistened with water. A second surface of the skin-adhesive film of the test piece was adhered to the artificial leather, and the support substrate was removed. Thus, a sample was prepared.
2) The sample prepared in the above 1) was adhered to a measurement table of a tabletop compression and tensile tester (manufactured by Shimazu Corporation: EZ-Test, Load cell: 100N) with the skin-adhesive film upside by using a double-sided tape (manufactured by Nichiban Co., Ltd.: Nice Tack Sponge double-sided tape, sponge type, NW-P15).
3) A double-sided tape (manufactured by Nichiban Co., Ltd.: Nice Tack for outdoor use, NW-N50) having a circular shape of 30 mm diameter was bonded to a 30-mm-diameter cylindrical indenter, and a protective film of the double-sided tape was removed.
4) The cylindrical indenter prepared in the above 3) was pressed against the skin-adhesive film of the sample placed on the measurement table of the above 2) at a compression speed of 20 mm/min and a pressure of 3N for 3 seconds, and then the above cylindrical indenter was vertically lifted at a peeling speed of 10 mm/min.
5) The data of peak strength (mN) when the skin-adhesive film is separated from the artificial leather was obtained, and the value was taken as an adhesion strength.

### <Evaluation Results>

Table 4 shows the measurement results of surface roughness for the respective examples and comparative examples, and Table 5 shows the measurement results of adhesion of cosmetics and skin adhesion of the skin-adhesive film. In evaluation of adhesion of cosmetics, a case where the luminance difference was 40 or less was rated as "poor," a case where the luminance difference was greater than 40 and less than 50 was rated as "good," and a case where the luminance difference was 50 or more was rated as "excellent." In evaluation of skin adhesion, a case where the adhesion strength was 3000 mN or less was rated as "fair," a case where the adhesion strength was greater than 3000 mN and 4000 mN or less was rated as "good," and a case where the adhesion strength was greater than 4000 mN was rated as "excellent."

**[Table 4]**

| | First surface: surface roughness (nm) | | | Second surface: surface roughness (nm) | | |
|---|---|---|---|---|---|---|
| | Ra1 | Rz1 | Sm1 | Ra2 | Rz2 | Sm2 |
| Example 3-1 | 200 | 1180 | 63000 | 105 | 580 | 57004 |
| Example 3-2 | 245 | 1985 | 90805 | 105 | 580 | 57004 |
| Example 3-3 | 300 | 2500 | 114453 | 105 | 580 | 57004 |
| Example 3-4 | 200 | 1180 | 63000 | 113 | 610 | 57900 |
| Example 3-5 | 245 | 1985 | 90805 | 155 | 1230 | 80000 |
| Example 3-6 | 300 | 2500 | 114453 | 200 | 2015 | 99800 |
| Example 3-7 | 320 | 2450 | 98752 | 210 | 2252 | 90600 |
| Comparative example 3-1 | 110 | 600 | 57300 | 105 | 580 | 57004 |

**[Table 5]**

| | Adhesion of cosmetics | | Skin Adhesion | |
|---|---|---|---|---|
| | Luminance difference | Evaluation | Adhesion strength (mN) | Evaluation |
| Example 3-1 | 44 | Good | 2657 | Fair |
| Example 3-2 | 45 | Good | 2657 | Fair |
| Example 3-3 | 52 | Excellent | 2657 | Fair |
| Example 3-4 | 44 | Good | 3000 | Fair |
| Example 3-5 | 45 | Good | 3850 | Good |
| Example 3-6 | 52 | Excellent | 5031 | Excellent |
| Example 3-7 | 50 | Excellent | 4780 | Excellent |
| Comparative example 3-1 | 40 | Poor | 2657 | Fair |

As seen from Tables 4 and 5, Examples 3-1 to 3-7, in which the first surface has surface roughness that satisfies the above (a) to (c), show high adhesion of cosmetics compared to Comparative Example 3-1, in which the first surface has surface roughness that does not satisfy the above (b). Further, among the examples, Examples 3-3, 3-6, and 3-7, having the first arithmetic average roughness Ra1 of 300 nm or more, the first ten-point average roughness Rz1 of 2400 nm or more, and the first average interval Sm1 of 98 µm or more, show especially high adhesion of cosmetics.

Further, Examples 3-5 to 3-7, in which the second surface has surface roughness that satisfies the above (d) to (f) show high skin adhesion compared to Comparative Example 3-1 and Examples 3-1 to 3-4, in which the second surface has surface roughness that does not satisfy the above (e). Among Examples 3-5 to 3-7, Examples 3-6 and 3-7, having the second arithmetic average roughness Ra2 of 200 nm or more, the second ten-point average roughness Rz2 of 2 µm or more, and the second average interval Sm2 of 90 µm or more, show especially high skin adhesion.

As described in the above embodiments and examples, the skin-adhesive film and the transfer sheet of the third embodiment can provide the following advantages.
(3-1) When the first surface 111F of the skin-adhesive film 110 satisfies the above (a) to (c), the first surface 111F has roughness with sufficient height and density while ensuring a strength of the skin-adhesive film. Accordingly, cosmetics are likely to be retained by the asperities on the first surface 111F, which enhances adhesion of cosmetics to the skin-adhesive film 110. As a result, the skin-adhesive film 110 has increased utility in cosmetic applications.
   Further, by virtue of the asperities provided on the first surface 111F, coloring materials such as pigments are likely to be retained in a desired range on the first surface 111F when printing is performed onto the first surface 111F, for example, by an ink jet method. Accordingly, printability on the surface of the skin-adhesive film 110 can be improved.
(3-2) When the second surface 111R of the skin-adhesive film 110 satisfies the above (d) to (f), the second surface 111R has roughness with sufficient height and frequency while ensuring a strength of the skin-adhesive film. Accordingly, fine gaps are likely to be formed between the skin surface and the skin-adhesive film 110. This facilitates discharge of moisture existing between the skin surface and the skin-adhesive film 110, and thus the skin adhesion of the skin-adhesive film 110 is increased.
   Further, by virtue of the asperities provided on the second surface 111R, printability on the second surface 111R can be improved as with the case of the first surface 111F.
(3-3) The first arithmetic average roughness Ra1 is larger than the second arithmetic average roughness Ra2, the first ten-point average roughness Rz1 is larger than the second ten-point average roughness Rz2, and the first average interval Sm1 is larger than the second average interval Sm2. With this configuration, the first surface 111F has a surface roughness more suitable for holding cosmetics compared to the second surface 111R, and the second surface 111R has a surface roughness more suitable for forming gaps between the skin surface and the skin-adhesive film 110 compared to the first surface 111F. Accordingly, the skin-adhesive film 110 having a configuration in which the first surface 111F and the second surface 111R perform different functions can be obtained.

### [Modifications]

The third embodiment can be modified as follows.
- When the second surface 111R satisfies the above (d) to (f), skin adhesion of the skin-adhesive film 110 increases regardless of whether the first surface 111F satisfies the above (a) to (c). That is, if water contained in the skin or water such as lotion supplied to the skin before the skin-adhesive film is adhered exists on the skin surface, the skin-adhesive film is difficult to adhere to the skin, particularly immediately after the adhesion. Therefore, when it is an object to provide a skin-adhesive film and a transfer sheet having improved skin adhesion, a skin-adhesive film having the following configuration can solve the problem.
   A skin-adhesive film including: a first surface; and a second surface, which is a surface opposite to the first surface, the second surface being configured to be adhered to a skin surface, wherein the skin-adhesive film has a portion having a thickness of 5 µm or less, and the second surface has an arithmetic average roughness Ra of 100 nm or more and 5 µm or less, a ten-point average roughness Rz of 1 µm or more and 50 µm or less, and an average interval between peaks and valleys Sm of 10 µm or more and 500 µm or less.
- The skin-adhesive film 110 may include a portion which is convex on the first surface 111F and is concave on the second surface 111R, in other words, a portion having a wavy shape in cross-section. With this configuration, on the assumption that the thickness of the skin-adhesive film 110 is in a desired range, the skin-adhesive film 110 is prevented from being locally thin when the above (a) to (f) are satisfied.

### [Appendix]

The measure for solving the foregoing problems embraces the following items as technical concepts derived from the third embodiment.

### (Item 11)

A skin-adhesive film including: a first surface; and a second surface, which is a surface opposite to the first surface, the second surface being configured to be adhered to a skin surface, wherein the skin-adhesive film has a portion having a thickness of 5 µm or less, and the first surface has an arithmetic average roughness Ra of 100 nm or more and 5 µm or less, a ten-point average roughness Rz of 1 µm or more and 50 µm or less, and an average interval between peaks and valleys Sm of 10 µm or more and 500 µm or less.

With this configuration, since the first surface has roughness with sufficient height and density while ensuring a strength of the skin-adhesive film, cosmetics are likely to be retained by the asperities on the first surface. Therefore, adhesion of cosmetics to the skin-adhesive film is increased. As a result, the skin-adhesive film has increased utility in cosmetic applications.

### (Item 12)

The skin-adhesive film according to Item 11, wherein the arithmetic average roughness Ra of the first surface is larger than the arithmetic average roughness Ra of the second surface, the ten-point average roughness Rz of the first surface is larger than the ten-point average roughness Rz of the second surface, and the average interval between peaks and valleys Sm of the first surface is larger than the average interval between peaks and valleys Sm of the second surface.

According to the above configuration, the first surface has a surface roughness more suitable for holding cosmetics compared to the second surface, and the second surface 111R has a surface roughness more suitable for forming gaps between the skin surface and the skin-adhesive film 110 compared to the first surface. Accordingly, the skin-adhesive film having a configuration in which the first surface and the second surface perform different functions can be obtained.

### (Item 13)

A transfer sheet including: the skin-adhesive film according to Item 11 or 12; and a support substrate that supports the skin-adhesive film.

With this configuration, the skin-adhesive film is prevented from becoming deformed during storage and transport of the transfer sheet. Further, since the skin-adhesive film is supported by the support substrate, ease of handling of the skin-adhesive film is increased.

### (Fourth Embodiment)

In the skin-adhesive film formed in a film shape having a front surface and a rear surface, problems due to the action of light on each surface may occur, which are not likely to occur in cosmetics in a cream or powder form. Specifically, a glossy appearance due to reflection of light on the surface, that is, oily or shiny appearance of skin, and interference colors due to interference of light reflected from the front and rear surfaces may cause the skin-adhesive film adhered to be conspicuous.

Further, skin-adhesive films used for assisting in skin care and makeup are desired to produce a high soft focus effect. The soft focus effect refers to properties that blur fine imperfections such as wrinkles, spots, and freckles on the skin surface while allowing the presence of the skin to be recognized via the skin-adhesive film adhered.

An object of the fourth embodiment is to provide a skin-adhesive film and a transfer sheet having an improved soft focus effect while making the film adhered to the skin less conspicuous.

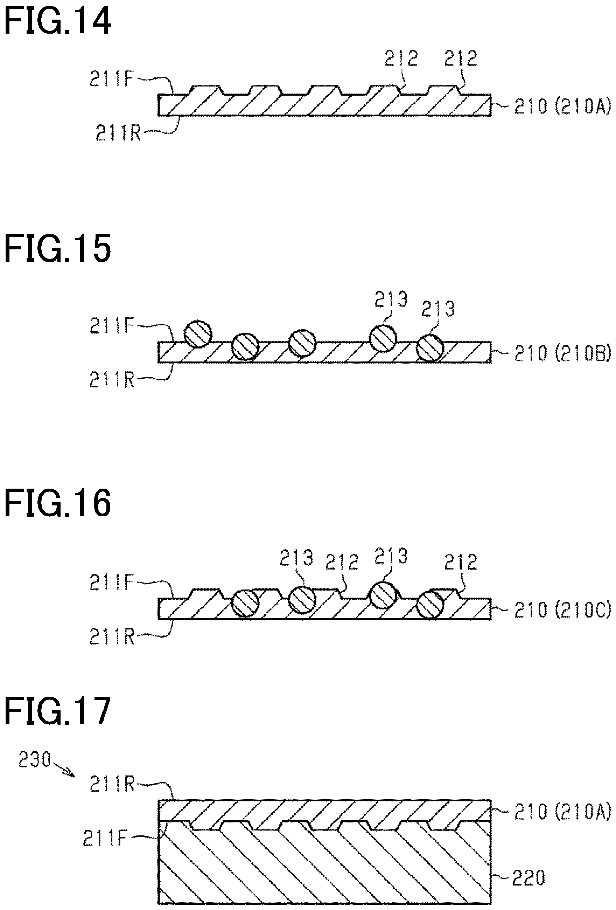

With reference to Figs. 14 to 17, the fourth embodiment of a skin-adhesive film and a transfer sheet will be described.

### [Configuration of Skin-Adhesive Film]

As shown in Fig. 14, a skin-adhesive film 210 has a first surface 211F, and a second surface 211R, which is a surface opposite to the first surface 211F. The second surface 211R is adhered to the skin. The first surface 211F is exposed to the outside when the skin-adhesive film 210 is adhered to the skin. The skin-adhesive film 210 may be configured as a single layer film, or may be configured as a multi-layered film.

The skin-adhesive film 210 is sufficiently thin to exhibit adhesion to the skin. When such properties are expressed by mass of the skin-adhesive film 210, the average mass per unit area of the skin-adhesive film 210 is 0.1 g/m² or more and 4.0 g/m² or less. In this case, the density of the skin-adhesive film 210 is, for example, 1 g/cm³ or more and 3 g/cm³ or less.

The average mass is obtained by measuring each mass of three film pieces, which are prepared by cutting out the skin-adhesive film 210 at three points into a square shape with sides of 100 mm in plan view, by using a precision balance, and multiplying the average of these masses of the three film pieces by 100.

When the average mass is 0.1 g/m² or more, the skin-adhesive film 210 has sufficient strength, and the skin-adhesive film 210 is prevented from being easily torn when adhered to the skin. When the average mass is 4.0 g/m² or less, the skin-adhesive film 210 has sufficient skin adhesion. Further, when the skin-adhesive film 210 is adhered to the skin, occurrence of creases in the skin-adhesive film 210 and discomfort that the user feels are reduced.

The above average mass increases when the skin-adhesive film 210 contains additives such as components that function as a plasticizer such as oil, various resin components, particles, and the like. When the skin-adhesive film 210 does not contain an additive, the average mass per unit of the skin-adhesive film 210 is preferably 0.1 g/m² or more and 2.0 g/m² or less.

The skin-adhesive film 210 has haze of 7% or more and 65% or less. The haze is a parameter indicating the ratio of the diffused component to the total transmitted light through the skin-adhesive film 210. The haze is measured by a method in accordance with JIS K 7136-2000. The haze described above is transmission haze, and is also referred to as haze. The haze is measured, for example, with the first surface 211F of the skin-adhesive film 210 directed toward an integrating sphere, and the second surface 211R directed toward a light source.

In order to improve the soft focus effect, it is necessary to increase the ratio of the component of light that is diffused without travelling straight to the light transmitted through the skin-adhesive film 210. That is, the greater the haze, the better the soft focus effect. Further, due to scattering of light occurring on the first surface 211F and the second surface 211R of the skin-adhesive film 210, glossy appearance of the surface of the skin-adhesive film 210 and interference colors caused by reflection on the front and rear surfaces of the skin-adhesive film 210 are suppressed.

Due to the haze being 7% or more, gloss and interference colors are suppressed. Accordingly, a site on the skin where the skin-adhesive film 210 is adhered is rendered inconspicuous, and the soft focus effect is improved. Further, due to the haze being 65% or less, the skin-adhesive film 210 appears cloudy, and a site where the skin-adhesive film 210 is adhered is rendered inconspicuous.

Scattering that increases the haze includes surface scattering at the first surface 211F and the second surface 211R of the skin-adhesive film 210, and internal scattering in the skin-adhesive film 210. The degree of surface scattering varies depending on the roughness on the respective surfaces, and the refractive index of resin constituting the skin-adhesive film 210. The degree of internal scattering varies depending on the shape of additives such as particles contained in the skin-adhesive film 210, and the difference in refractive index between the additive and the resin mainly constituting the skin-adhesive film 210. The greater the surface scattering, the greater the effect of suppressing gloss and interference colors.

Further, in order to allow an observer to recognize the presence of the skin through the skin-adhesive film 210 while ensuring the soft focus effect so that a site where the skin-adhesive film 210 is adhered has a natural, skin-like appearance, the total light transmittance of the skin-adhesive film 210 is preferably 45% or more, and more preferably 80% or more. However, when the skin-adhesive film 210 is colored, the lower limit of the total light transmittance is not limited to the above values. In addition, in order to facilitate an increase in haze, the total light transmittance is preferably 95% or less. The total light transmittance is measured in accordance with JIS K 7361-1-1997.

Indices for light scattering in the skin-adhesive film 210 include glossiness, reflection haze, transmission image sharpness, and distinctness of image (DOI), in addition to the above haze, which is transmission haze.

For the glossiness of the skin-adhesive film 210, measured in accordance with JIS Z 8741-1997 (ISO 2813:1994), a 60 degree glossiness is preferably 5 or more and 50 or less. The reflection haze of the skin-adhesive film 210, measured in accordance with ASTME 430 (ISO 13803), is preferably 10 HU or more and 30 HU or less. The transmission image sharpness of the skin-adhesive film 210, measured in accordance with JISK 7374-2007, is preferably 1% or more and 95% or less. DOI is also referred to as image clarity, and the DOI of the skin-adhesive film 210, measured in accordance with ASTMD 5767, is preferably 1% or more and 85% or less.

Further, a target surface for measurement of glossiness, reflection haze, and DOI may be either the first surface 211F or the second surface 211R. For example, the second surface 211R is measured. For both the first surface 211F and the second surface 211R, each of the glossiness, the reflection haze, and the DOI preferably satisfy the above respective ranges. For example, the transmission image sharpness is measured with the first surface 211F of the skin-adhesive film 210 directed toward an integrating sphere, and the second surface 211R directed toward a light source.

When the above parameters are within the above respective ranges, the skin-adhesive film 210 exhibits good light-scattering effect. Accordingly, gloss and interference colors are suppressed, and a good soft focus effect is obtained. In particular, the DOI is an index representing the sharpness of an image on a surface of a target. The smaller the DOI, the higher the light-scattering effect on a surface of the skin-adhesive film 210. The DOI is a parameter obtained by measuring the reflection component. Even when the transmission haze, which is obtained by measuring the transmission component, is the same between the samples, the DOI may be largely different between the samples. By using both the transmission haze and the DOI for evaluation, it is possible to more appropriately specify the physical properties of the skin-adhesive film 210 that can be provided with reduced gloss and interference colors and improved soft focus effect.

Further, in measurement of the glossiness, reflection haze, and DOI, in which a reflection component is to be measured, the measurement is performed using a sample prepared by, for example, adhering the skin-adhesive film 210 to a black polyethylene terephthalate film (manufactured by Toray Industries Inc.: Lumirror, X30 thickness 125 µm) via water, and drying the film.

Further, when the skin-adhesive film 210 has at least one parameter among the transmission haze, glossiness, reflection haze, transmission image sharpness, and DOI included within the above preferable ranges, at least one of prevention of the skin-adhesive film 210 from being conspicuous due to gloss and interference colors, and improvement in soft focus effect can be achieved.

A specific structure of the skin-adhesive film 210 for increasing a light-scattering effect will be described below by using three types, which includes a first type, a second type, and a third type. Fig. 14 illustrates a cross-sectional structure of a first type skin-adhesive film 210A.

As shown in Fig. 14, the first type skin-adhesive film 210A has a surface on which asperities are formed. The asperities are formed by shaping the surface of the skin-adhesive film 210A. Due to the asperities, light is scattered on the surface of the skin-adhesive film 210A.

The target surface having the asperities may be the first surface 211F or the second surface 211R. Further, both the first surface 211F and the second surface 211R may be target surfaces, that is, the skin-adhesive film 210A may have asperities on both the first surface 211F and the second surface 211R. When the first surface 211F has asperities, a light-scattering effect on the first surface 211F, which is a surface exposed to the observer, increases in the skin-adhesive film 210A adhered to the skin. Therefore, an effect of suppressing gloss on the skin-adhesive film 210A increases.

As illustrated in Fig. 14, the shape of the target surface may be formed of a plurality of projections 212 protruding from the target surface. Alternatively, the surface shape of the target surface may be formed by a plurality of recesses that are recessed from the target surface, or may be formed of a combination of the above projections and recesses. The size of the projections and recesses may or may not be constant. Further, the projections and recesses may be arranged regularly or irregularly.

The arithmetic average roughness Ra on the target surface is preferably 0.15 µm or more and 4.00 µm or less. When the arithmetic average roughness Ra is within the above range, it is possible to obtain appropriate light-scattering effect on the target surface, and control the haze to an appropriate value.

Fig. 15 illustrates a cross-sectional structure of a second type skin-adhesive film 210B. As shown in Fig. 15, the second type skin-adhesive film 210B contains particles 213.

Due to the particles 213 contained inside the skin-adhesive film 210B, light is scattered inside the skin-adhesive film 210B. Further, when the particles 213 protrude from the surface of the skin-adhesive film 210B, and thus the surface has asperities that follows the shape of the dispersed particles 213, light is scattered on the surface of the skin-adhesive film 210B as well. The surface having the protruding particles 213 may be the first surface 211F or the second surface 211R, or may be both the first surface 211F and the second surface 211R. An effect of suppressing gloss on the skin-adhesive film 210B increases when the particles 213 protrude from the first surface 211F.

In order to reliably obtain a light-scattering effect due to the protruding particles 213 on the surface of the skin-adhesive film 210B, the arithmetic average roughness Ra of the surface where the particles 213 protrude is preferably 0.15 µm or more and 4.00 µm or less.

Fig. 16 illustrates a cross-sectional structure of a third type skin-adhesive film 210C. As shown in Fig. 16, the third type skin-adhesive film 210C has asperities formed by shaping the surface, and contains particles 213.

Due to the asperities, light is scattered on the surface of the skin-adhesive film 210C. Further, when the particles 213 protrude from the surface of the skin-adhesive film 210C, the asperities on the surface of the skin-adhesive film 210C are formed of at least one of the projections, which are protruding portions of the particles 213, and projections and recesses formed by shaping the surface. In addition, due to the particles 213 contained inside the skin-adhesive film 210C, light is scattered inside the skin-adhesive film 210C.

The target surface having the asperities may be the first surface 211F or the second surface 211R, or may be both the first surface 211F and the second surface 211R. When the first surface 211F is the target surface, an effect of suppressing gloss on the skin-adhesive film 210C increases.

The arithmetic average roughness Ra on the target surface is preferably 0.15 µm or more and 4.00 µm or less. The arithmetic average roughness Ra described in the first to third types is measured in accordance with JIS B 0601-1994. Further, the skin-adhesive film 210 may also have a structure different from that in the first to third types as long as the haze is 7% or more and 65% or less.

### [Materials for Skin-Adhesive Film]

The material for forming the skin-adhesive film 210 is not particularly limited. The material of the skin-adhesive film 210 is preferably a resin having low toxicity, skin irritation, and skin sensitization. Examples of the material for the skin-adhesive film 210 include natural polymers such as hyaluronic acid, fibroin, and chitosan, and ester resins such as polylactic acid, polyglycolic acid, and polycaprolactone, and copolymer resins thereof. Further, resins for use as film-formers in cosmetics may also be used as the material for the skin-adhesive film 210. Examples of such resins include acrylic resin, silicone, and copolymer resins thereof, and cellulose derivatives such as cellulose acetate, cellulose acetate propionate, and cellulose acetate butyrate. Further, examples of the material for the skin-adhesive film 210 include resins that are commonly used as materials for medical products, such as polycarbonate, cycloolefin copolymer, styrene-butadiene elastomer, polyimide, acrylic resin, urethane resin, and copolymer resins thereof. The molecular weight of the materials for the skin-adhesive film 210 is not particularly limited. For example, the skin-adhesive film 210 may be made of one type of material having a predetermined average molecular weight, or may also be made of a plurality of types of materials having different average molecular weights.

The skin-adhesive film 210 may contain various additives. Examples of the additives include plasticizers such as oil, resins miscible with the main component, and particles such as a filler, added for improving the film strength.

The particles 213 contained in the second type and third type skin-adhesive film 210 are, for example, an inorganic filler or an organic filler. Materials for the inorganic filler include, for example, talc, silica, mica, alumina, zinc oxide, titanium oxide, barium sulfate, calcium carbonate, magnesium carbonate, barium silicate, calcium silicate, metal soap, silicone, and the like. Materials for the organic filler include, for example, polyamide, polyethylene, polypropylene, acrylic resin, polyurethane, polytetrafluoroethylene, cellulose, and the like. In order to cause internal scattering in the skin-adhesive film 210, the particles 213 are made of a material having a refractive index different from the main component of the skin-adhesive film 210. The volume average particle size of the particles 213 by laser diffraction scattering method is preferably 0.5 µm or more in view of reducing aggregation of the particles 213 in formation of the skin-adhesive film 210, and preferably 20.0 µm or less in view of reducing precipitation of the particles 213 in formation of the skin-adhesive film 210.

In addition, the skin-adhesive film 210 may contain functional materials that perform a predetermined function for the skin as an additive. Examples of the functional materials include cosmetics and cosmetic ingredients used for skin care, such as moisturizing creams and beauty essence, dye, drugs, pigment, proteins, and enzymes. The skin-adhesive film 210 may contain one type of functional material, or may contain two or more types of functional materials.

### [Configuration of Transfer Sheet]

A transfer sheet 230 used for adhering the skin-adhesive film 210 to the skin will be described. As shown in Fig. 17, the transfer sheet 230 includes the skin-adhesive film 210, and a support substrate 220 that supports the skin-adhesive film 210. Although Fig. 17 illustrates the transfer sheet 230 supporting the first type skin-adhesive film 210A, the transfer sheet 230 may also be provided with the second type skin-adhesive film 210B or the third type skin-adhesive film 210C.

The support substrate 220 is in contact with the first surface 211F of the skin-adhesive film 210. When the first surface 211F has asperities, the asperities may be or may not be filled with the support substrate 220. The support substrate 220 has a function of reducing deformation of the skin-adhesive film 210 during storage of the transfer sheet 230 and transport of the skin-adhesive film 210 onto the skin in use of the transfer sheet 230. Since the skin-adhesive film 210 is supported by the support substrate 220, ease of handling of the skin-adhesive film 210 can be increased.

The support substrate 220 is preferably a porous substrate. The porous substrate has a large number of fine pores inside the substrate so that liquid can infiltrate or penetrate through the pores. Examples of the porous substrate that can be used as the support substrate 220 include sheets made of fibrous materials such as non-woven fabric, paper, knitted fabric, and woven fabric, and resin sheets having a structure with gaps, such as a mesh. Among these substrates, a non-woven fabric is preferably used due to the ability to allow quick absorption and diffusion of water. Examples of fibers forming the non-woven fabric include natural fibers such as cotton, hemp, wool, and pulp, semi-synthetic fibers such as rayon, and synthetic fibers such as polyvinyl alcohol, polyacrylates. Among the above fibers, natural fibers, especially pulps, are preferably used. The non-woven fabric may be formed of a single type of fiber, or two or more types of fibers.

The methods for manufacturing the non-woven fabric used as the support substrate 220 are not specifically limited. The non-woven fabric may be manufactured by spunlacing, spunbonding, needle-punching, melt blowing, air-laying, flash spinning, or resin adhesion. The non-woven fabric used as the support substrate 220 preferably has a basis weight of 10 g/m² or more and 150 g/m² or less, and more preferably 20 g/m² or more and 50 g/m² or less in view of texture for good tactile feel.

Further, the support substrate 220 is not limited to the porous substrate, and may also be formed of a substrate such as a resin sheet or metal foil having no pore inside.

The transfer sheet 230 may further include a protective layer that covers the second surface 211R of the skin-adhesive film 210. The protective layer has a function of protecting the second surface 211R. The protective layer is preferably made of a porous substrate. The porous substrate for forming the protective layer may be made of the same material as those of the porous substrate for forming the support substrate 220. The protective layer and the support substrate 220 may be formed of the same type of porous substrates, or may be formed of different types of porous substrates. In addition, the protective layer is not limited to a porous substrate, and may also be formed of a substrate such as a resin sheet or metal foil having no pores inside.

### [Method for Producing Skin-Adhesive Film And Transfer Sheet]

An example of a method for producing the skin-adhesive film 210 and the transfer sheet 230 will be described below. The method for producing the skin-adhesive film 210 and the transfer sheet 230 may be different from the production method described below as long as the skin-adhesive film 210 and the transfer sheet 230 having the above configuration can be formed.

First, the skin-adhesive film 210 is formed on a surface of a film-formation substrate. As the film-formation substrate, a substrate made of a thermoplastic resin, a heat-curable resin, a water soluble resin, metal oxide, metal, or the like may be used. For example, the film-formation substrate may be a substrate made of a resin such as polyethylene terephthalate, polyethylene naphthalate, polyethylene, polypropylene, acrylic resin, polycarbonate, cycloolefin, polyamide, epoxy resin, urethane resin, polyvinyl alcohol, or the like, or may be a substrate obtained by applying stretching treatment, release treatment, or matte treatment to a substrate made of these resins. Alternatively, the film-formation substrate may be a substrate made of an inorganic substance such as glass, quartz, or aluminum, or may be a substrate obtained by applying stretching treatment, release treatment, or matte treatment to a substrate made of these inorganic substances.

A coating solution in which a material for the skin-adhesive film 210 is dissolved in a solvent is applied to a surface of the film-formation substrate to form a coating film. The coating film is then dried to form a skin-adhesive film 210. As the above solvent, ester solvents such as ethyl acetate or butyl acetate, ketone solvent such as acetone or ethyl methyl ketone, polar aprotic solvents such as toluene or hexane, or polar protic solvents such as water or alcohol may be used depending on the properties of the material for the skin-adhesive film 210.

The method of applying a coating solution is not specifically limited, and any method that is able to precisely control the amount of coating solution to be applied can be used. The method of applying a coating solution is preferably one of gravure coating, micro gravure coating, spin coating, spray coating, silk screening, die coating, and curtain coating.

The film-formation substrate may be used as a support substrate 220. Alternatively, a substrate different from the support substrate 220 may be used as a film-formation substrate, and the skin-adhesive film 210 formed on the film-formation substrate may be transferred to the support substrate 220. Known transfer methods such as peeling by suction and a method using a sacrificial film can be used.

Asperities can be formed on the first surface 111F and the second surface 111R by using known methods. For example, the following four methods can be used to form asperities. In the first method, a substrate on which asperities are formed in advance, that is, a substrate treated with matte finish, is used as a film-formation substrate. Since the asperities are formed on a surface of the film-formation substrate, asperities are formed on the surface of the skin-adhesive film 210 formed on the film-formation substrate. In the second method, in application of a coating solution to a film-formation substrate, a printing plate on which a random pattern is formed is used to form asperities on a surface of a coating film to thereby form asperities on a surface of the skin-adhesive film 210. In the third method, a plate on which asperities are formed is brought into contact with a surface of a coating film before the coating film is dried to thereby form asperities on a surface of the skin-adhesive film 210. In the fourth method, a heated plate on which asperities are formed is pressed against a surface of a film product obtained by drying a coating film, or the film product is heated and pressed against the plate which has been cooled, to thereby form asperities on a surface of the skin-adhesive film 210. The plate used for formation of asperities may be made of metal or may be made of resin.

In order to provide the skin-adhesive film 210 with the particles 213 contained therein, the particles 213 may be dispersed in the coating solution.

In order to form the transfer sheet 230 having a protective layer, a protective layer is disposed on the second surface 211R, which is a surface of the skin-adhesive film 210 on a side opposite to that in contact with the support substrate 220.

### [Method for Adhering Skin-Adhesive Film]

A method for adhering the skin-adhesive film 210, that is, a method for transferring the skin-adhesive film 210 by using the transfer sheet 230 will be described below. In a method described below, a porous substrate is used as the support substrate 220, and the support substrate 220 is moistened in transfer of the skin-adhesive film 210 to the skin.

First, liquid such as water is supplied to a site on the skin where the skin-adhesive film 210 is to be adhered. A supplied liquid, which is liquid to be supplied to the skin, may be any liquid that can moisten the transfer sheet 230. Specifically, liquid containing water, or oil such as massage oil can be used. For example, water or cosmetics such as a lotion can be used as the supplied liquid.

Subsequently, the transfer sheet 230 is placed on the skin with the second surface 211R of the skin-adhesive film 210 being in contact with the site on the skin where the skin-adhesive film 210 is to be adhered. The support substrate 220 of the transfer sheet 230 is pressed with a finger so that the supplied liquid infiltrates into the support substrate 220.

Then, the support substrate 220 is removed from the skin-adhesive film 210. Thus, the skin-adhesive film 210 is adhered to the skin. Since the transfer sheet 230 is moistened, the support substrate 220 made of a porous substrate swells, or the supplied liquid infiltrates between the support substrate 220 and the skin-adhesive film 210, which facilitates removal of the support substrate 220 from the skin-adhesive film 210.

In the above transfer method, the supplied liquid is supplied to the skin before the transfer sheet 230 is placed on the skin. However, the supplied liquid may also be supplied to the transfer sheet 230 after the transfer sheet 230 is placed on the skin. Further, the support substrate 220 may also be removed from the skin-adhesive film 210 without moistening the transfer sheet 230. In this case, the support substrate 220 may not be necessarily a porous substrate.

Further, the skin-adhesive film 210 may be adhered to the skin after cosmetics are applied to the skin, or may be adhered to the skin before cosmetics are applied to the skin. In addition, after the skin-adhesive film 210 is adhered to the skin, cosmetics may be applied to the skin-adhesive film 210. The skin-adhesive film 210 assists in skin care by moisturizing the skin, or assists with makeup by functioning as a makeup base.

### [Examples]

The skin-adhesive film and the transfer sheet of the fourth embodiment will be described by using specific examples and comparative examples.

### (Example 4-1)

DL-polylactic acid (manufactured by BMG Corporation) was dissolved in ethyl acetate, and the solution was adjusted to obtain a polylactic acid concentration of 10 wt% to generate a coating solution for forming a skin-adhesive film. A 1-mm thick float glass having a surface on which asperities were formed by sand-blasting, which was in turn subjected to release treatment, was used as a film-formation substrate. The above coating solution was applied to a surface of the film-formation substrate by using a wire bar to form a coating film. The surface of the film-formation substrate had an arithmetic average roughness Ra of 0.30 µm.

The coating film was heated in an oven for drying and curing to form a skin-adhesive film. A heating temperature during drying was selected from a range of 70°C or more and 120°C or less. In this step, the skin-adhesive film was formed with an average dry mass of 0.3 g/m².

Subsequently, the skin-adhesive film was transferred from the film-formation substrate to another substrate. Then, a non-woven fabric (manufactured by Futamura Chemical Co., Ltd.) as a support substrate was laminated on a surface of the skin-adhesive film, on which asperities have been formed by being in contact with the film-formation substrate, to transfer the skin-adhesive film to the support substrate. The main component of the non-woven fabric was cellulose, and the basis weight was 20 g/m².

Thus, a skin-adhesive film and a transfer sheet of Example 4-1 were obtained. The surface of the skin-adhesive film which had been in contact with the film-formation substrate was provided as a first surface, which was in contact with the support substrate.

### (Example 4-2)

A skin-adhesive film and a transfer sheet of Example 4-2 were prepared in the same manner as in Example 4-1 except that a float glass having a surface roughness different from that in Example 4-1 was used as a film-formation substrate, and the skin-adhesive film was formed with an average dry mass of 0.8 g/m². The float glass used in Example 4-2 had a surface on which asperities were formed by sand-blasting, and the surface had the arithmetic average roughness Ra of 3.00 µm.

### (Example 4-3)

A skin-adhesive film and a transfer sheet of Example 4-3 were prepared in the same manner as in Example 4-1 except that a float glass having a surface roughness different from that in Example 4-1 was used as a film-formation substrate, and the skin-adhesive film was formed with an average dry mass of 1.1 g/m². The float glass used in Example 4-3 had a surface on which asperities were formed by sand-blasting, and the surface had the arithmetic average roughness Ra of 4.00 µm.

### (Example 4-4)

A skin-adhesive film and a transfer sheet of Example 4-4 were prepared in the same manner as in Example 4-1 except that a float glass having a surface roughness different from that in Example 4-1 was used as a film-formation substrate, and the skin-adhesive film was formed with an average dry mass of 1.5 g/m². The float glass used in Example 4-4 had a surface on which asperities were formed by sand-blasting, and the surface had the arithmetic average roughness Ra of 4.50 µm.

### (Example 4-5)

DL-polylactic acid (manufactured by BMG Corporation) and titanium oxide particles having 1 µm average volume particle size were added and dissolved in ethyl acetate, and the solution was adjusted to obtain the polylactic acid concentration of 5 wt% and titanium oxide particle concentration of 10 wt% to generate a coating solution for forming a skin-adhesive film. Polyethylene terephthalate sheet having a 50 µm thickness was used as a film-formation substrate, and the coating solution was applied to a surface of the film-formation substrate by using a wire bar to form a coating film.

The coating film was heated in an oven for drying and curing to form a skin-adhesive film. A heating temperature during drying was selected from a range of 70°C or more and 120°C or less. In this step, the skin-adhesive film was formed with an average dry mass of 1.5 g/m².

Subsequently, the skin-adhesive film was transferred from the film-formation substrate to another substrate. Then, a non-woven fabric (manufactured by Futamura Chemical Co., Ltd.) as a support substrate was laminated on a surface of the skin-adhesive film which had been in contact with the film-formation substrate to transfer the skin-adhesive film to the support substrate. The main component of the non-woven fabric was cellulose, and the basis weight was 20 g/m².

Thus, a skin-adhesive film and a transfer sheet of Example 4-5 were obtained.

### (Example 4-6)

A skin-adhesive film and a transfer sheet of Example 4-6 were prepared in the same manner as in Example 4-5 except that the skin-adhesive film was formed with an average dry mass of 2.3 g/m².

### (Example 4-7)

A skin-adhesive film and a transfer sheet of Example 4-7 were prepared in the same manner as in Example 4-5 except that the skin-adhesive film was formed with an average dry mass of 3.1 g/m².

### (Example 4-8)

DL-polylactic acid (manufactured by BMG Corporation) and titanium oxide particles having 1 µm average volume particle size were added and dissolved in ethyl acetate, and the solution was adjusted to obtain the polylactic acid concentration of 5 wt% and titanium oxide particle concentration of 10 wt% to generate a coating solution for forming a skin-adhesive film. A 1-mm thick float glass having a surface on which asperities were formed by sand-blasting, which was in turn subjected to release treatment, was used as a film-formation substrate. The above coating solution was applied to a surface of the film-formation substrate by using a wire bar to form a coating film. The surface of the film-formation substrate had an arithmetic average roughness Ra of 0.30 µm.

The coating film was heated in an oven for drying and curing to form a skin-adhesive film. A heating temperature during drying was selected from a range of 70°C or more and 120°C or less. In this step, the skin-adhesive film was formed with an average dry mass of 1.5 g/m².

Subsequently, the skin-adhesive film was transferred from the film-formation substrate to another substrate. Then, a non-woven fabric (manufactured by Futamura Chemical Co., Ltd.) as a support substrate was laminated on a surface of the skin-adhesive film, on which asperities have been formed by being in contact with the film-formation substrate, to transfer the skin-adhesive film to the support substrate. The main component of the non-woven fabric was cellulose, and the basis weight was 20 g/m².

Thus, a skin-adhesive film and a transfer sheet of Example 4-8 were obtained.

### (Example 4-9)

A skin-adhesive film and a transfer sheet of Example 4-9 were prepared in the same manner as in Example 4-8 except that a float glass having a surface roughness different from that in Example 4-8 was used as a film-formation substrate, and the skin-adhesive film was formed with an average dry mass of 2.3 g/m². The float glass used in Example 4-9 had a surface on which asperities were formed by sand-blasting, and the surface had the arithmetic average roughness Ra of 3.00 µm.

### (Example 4-10)

A skin-adhesive film and a transfer sheet of Example 4-10 were prepared in the same manner as in Example 4-8 except that a float glass having a surface roughness different from that in Example 4-8 was used as a film-formation substrate, and the skin-adhesive film was formed with an average dry mass of 3.1 g/m². The float glass used in Example 4-10 had a surface on which asperities were formed by sand-blasting, and the surface had the arithmetic average roughness Ra of 4.50 µm.

### (Comparative Example 4-1)

DL-polylactic acid (manufactured by BMG Corporation) was dissolved in ethyl acetate, and the solution was adjusted to obtain a polylactic acid concentration of 10 wt% to generate a coating solution for forming a skin-adhesive film. Polyethylene terephthalate sheet having a 50 µm thickness was used as a film-formation substrate, and the coating solution was applied to a surface of the film-formation substrate by using a wire bar to form a coating film.

The coating film was heated in an oven for drying and curing to form a skin-adhesive film. A heating temperature during drying was selected from a range of 70°C or more and 120°C or less. In this step, the skin-adhesive film was formed with an average dry mass of 0.4 g/m².

Subsequently, the skin-adhesive film was transferred from the film-formation substrate to another substrate. Then, a non-woven fabric (manufactured by Futamura Chemical Co., Ltd.) as a support substrate was laminated on a surface of the skin-adhesive film which had been in contact with the film-formation substrate to transfer the skin-adhesive film to the support substrate. The main component of the non-woven fabric was cellulose, and the basis weight was 20 g/m².

Thus, a skin-adhesive film and a transfer sheet of Comparative Example 4-1 were obtained.

### (Comparative Example 4-2)

A skin-adhesive film and a transfer sheet of Comparative Example 4-2 were prepared in the same manner as in Comparative Example 4-1 except that the skin-adhesive film was formed with an average dry mass of 0.8 g/m².

### (Comparative Example 4-3)

A skin-adhesive film and a transfer sheet of Comparative Example 4-3 were prepared in the same manner as in Comparative Example 4-1 except that a 1-mm thick float glass having a surface on which asperities were formed by sand-blasting, which was in turn subjected to release treatment, was used as a film-formation substrate, and the skin-adhesive film was formed with an average dry mass of 1.5 g/m². The surface of the film-formation substrate had an arithmetic average roughness Ra of 5.50 µm.

### <Evaluation Method>

### (Haze, total light transmittance, and surface roughness)

A second surface of the skin-adhesive film in the transfer sheets of the respective examples and comparative examples, that is, a surface opposite to the surface that faces the support substrate was adhered to a 1-mm thick float glass via water, and the support substrate was removed. After the skin-adhesive film was dried for 1 day, a haze, a total light transmittance, and an arithmetic average roughness of the first surface were measured. The haze and the total light transmittance were measured by using a haze meter (Murakami Color Research Laboratory: HM-150) and a D65 light source, with the first surface of the skin-adhesive film directed toward an integrating sphere, and the second surface directed toward the light source. The haze and the total light transmittance were measured in accordance with JIS K 7136-2000 and JIS K 7361-1-1997, respectively. The arithmetic average roughness was measured by using a surface roughness tester (manufactured by Tokyo Seimitsu Co., Ltd.: Surfcom 130A) in accordance with JIS B 0601-1994.

### (DOI)

A first surface of the skin-adhesive film in the transfer sheets of the respective examples and comparative examples was adhered to a black polyethylene terephthalate film (manufactured by Toray Industries Inc.: Lumirror, X30 thickness 125 µm) via water, and the support substrate was removed. After, the skin-adhesive film was dried for 1 day, a DOI of the second surface was measured. The DOI was measured by using a gloss meter (manufactured by Konica Minolta Inc.: Rhopoint IQ 20/60/85°) in accordance with ASTM D5767.

### (Skin affinity and soft focus effect)

A second surface of the skin-adhesive film in the transfer sheets of the respective examples and comparative examples was adhered to a 1-mm thick float glass via water. Then, the support substrate was removed, and the skin-adhesive film was dried. The float glass on which the skin-adhesive film had been adhered was placed on a surface of BIOSKIN (manufactured by Beaulax Co., Ltd.) on which artificial wrinkles were formed with the second surface of the skin-adhesive film directed to the BIOSKIN. Then, skin affinity and soft focus effect were evaluated.

In evaluation of skin affinity, difference in appearance between a site of the BIOSKIN where the skin-adhesive film was present and a site where the skin-adhesive film was not present was observed. The difference in appearance was evaluated on a 4-point scale from 1 (the difference in appearance was most noticeable) to 4 (the difference in appearance was least noticeable). Specifically, the evaluation was made as to whether gloss and interference colors were observed on a site where the skin-adhesive film was present, and whether the skin-adhesive film appears cloudy.

In evaluation of soft focus effect, a site of the BIOSKIN where the skin-adhesive film was present was observed, and whether wrinkles on the skin-adhesive film were observed or not was evaluated on a 4-point scale from 1 (wrinkles were most noticeable) to 4 (wrinkles were least noticeable).

### <Evaluation Results>

Table 6 shows the measurement results of haze, total light transmittance, arithmetic average roughness, and DOI, and the evaluation results of skin affinity and soft focus effect for the respective examples and comparative examples. The evaluation results of skin affinity and soft focus effect are averages of the 4-point scale evaluation results by 5 evaluators.

**[Table 6]**

| | Haze (%) | DOI (%) | Total light transmittance (%) | Arithmetic average roughness Ra (µm) | Skin affinity | Soft focus effect |
|---|---|---|---|---|---|---|
| Example 4-1 | 7.0 | 2.3 | 86.1 | 0.15 | 4.0 | 3.8 |
| Example 4-2 | 26.8 | 5.1 | 84.3 | 2.79 | 4.0 | 4.0 |
| Example 4-3 | 31.2 | 1.7 | 83.8 | 3.25 | 4.0 | 4.0 |
| Example 4-4 | 35.7 | 4.9 | 83.3 | 3.97 | 4.0 | 4.0 |
| Example 4-5 | 36.7 | 77.5 | 88.8 | 0.22 | 3.2 | 4.0 |
| Example 4-6 | 41.7 | 80.7 | 88.2 | 0.21 | 3.6 | 4.0 |
| Example 4-7 | 50.0 | 82.5 | 87.3 | 0.42 | 3.4 | 4.0 |
| Example 4-8 | 45.7 | 6.3 | 83.5 | 0.80 | 4.0 | 4.0 |
| Example 4-9 | 49.7 | 4.0 | 83.0 | 0.55 | 4.0 | 4.0 |
| Example 4-10 | 62.0 | 1.7 | 82.5 | 0.66 | 4.0 | 4.0 |
| Comparative example 4-1 | 3.6 | 94.5 | 91.4 | 0.10 | 1.0 | 1.0 |
| Comparative example 4-2 | 4.3 | 93.3 | 90.5 | 0.05 | 1.0 | 1.0 |
| Comparative example 4-3 | 71.6 | 0.5 | 88.2 | 4.36 | 1.8 | 4.0 |

As seen from Table 6, in Examples 4-1 to 4-10, in which the haze is 7% or more and 65% or less, both the skin affinity and the soft focus effect are good. Specifically, compared with Examples 4-1 to 4-10, in Comparative Examples 4-1 and 4-2, in which the haze is less than 7%, gloss and interference colors are observed. In addition, the skin affinity is significantly reduced, and the soft focus effect is significantly reduced as well. Accordingly, it is suggested that the skin-adhesive film exhibits sufficient light-scattering effect when the haze is 7% or more. On the other hand, in Comparative Example 4-3, in which the haze is more than 65%, the soft focus effect is high, but the haze is too high to cause the skin-adhesive film to appear white and cloudy. As a consequence, a site where the skin-adhesive film is adhered is conspicuous, resulting in a low skin affinity. As described above, it is found that both the skin affinity and the soft focus effect increase when the haze is 7% or more and 65% or less.

In comparison between Examples 4-1 to 4-4, in which the haze is increased due to the asperities formed by shaping the skin-adhesive film, and Examples 4-5 to 4-7, in which the haze is increased due to the particles contained in the skin-adhesive film, it is found that Examples 4-5 to 4-7, having the haze similar to or higher than that in Examples 4-1 to 4-4, tend to have lower arithmetic average roughness Ra. That is, it is suggested that the haze in Examples 4-1 to 4-4 is increased mainly due to surface scattering, whereas the haze in Examples 4-5 to 4-7 is increased mainly due to internal scattering. Further, although the soft focus effect in Examples 4-5 to 4-7 is similar to that in Examples 4-1 to 4-4, the skin affinity tends to be lower due to gloss and interference colors. Therefore, it is suggested that the effect of suppressing gloss and interference colors increases as the surface scattering increases.

In Examples 4-1 to 4-10, the arithmetic average roughness Ra is 0.15 µm or more and 4.00 µm or less. With the skin-adhesive film having the arithmetic average roughness Ra within such a range, it is suggested that the light-scattering effect on the surface is sufficient to obtain good skin affinity and soft focus effect. Further, in Examples 4-1 to 4-10, the DOI is 1% or more and 85% or less, and the total light transmittance is 80% or more and 95% or less. With the parameters within these ranges, it is suggested that good skin affinity and soft focus effect can be obtained.

Furthermore, skin-adhesive films were prepared in the same manner as in the above examples and comparative examples, and then transfer sheets were prepared with the first surface and the second surface of the skin-adhesive film being directed opposite to those in the above examples and comparative examples, that is, in this case, the second surface was instead in contact with the film-formation substrate. Then, the skin-adhesive film was placed on the BIOSKIN with the second surface being directed to the BIOSKIN, and the appearance was observed. The appearances of these skin-adhesive films were not significantly different from those in the above respective examples and comparative examples. Therefore, it is suggested that, when the haze is measured with the second surface of the skin-adhesive film directed toward the integrating sphere, and the DOI is measured for the first surface, the evaluation results are similar to those shown in Table 6.

As described in the above embodiments and examples, the skin-adhesive film and the transfer sheet of the fourth embodiment can provide the following advantages.
(4-1) In the skin-adhesive film 210, due to the haze being 7% or more, the skin-adhesive film 210 provides a light-scattering effect sufficient to suppress gloss and interference colors. Accordingly, a site where the skin-adhesive film 210 is adhered is rendered inconspicuous, and the soft focus effect is improved. Further, due to the haze being 65% or less, the skin-adhesive film 210 appears cloudy, and a site where the skin-adhesive film 210 is adhered is rendered inconspicuous. As a result, the skin-adhesive film 210 has increased utility in cosmetic applications.
(4-2) At least one of the first surface 211F and the second surface 211R of the skin-adhesive film 210 has a DOI of 1% or more and 85% or less. Accordingly, gloss and interference colors are reliably suppressed, and good soft focus effect is obtained.
(4-3) Due to the total light transmittance of the skin-adhesive film 210 being 45% or more and 95% or less, the degree of haze can be appropriately controlled. In addition, the skin can be seen through the skin-adhesive film 210 at the site where the film is adhered, which makes the site where the film is adhered appear natural.
(4-4) At least one of the first surface 211F and the second surface 211R of the skin-adhesive film 210 has the arithmetic average roughness Ra of 0.15 µm or more and 4.00 µm or less. Accordingly, light-scattering effect on the surface of the skin-adhesive film 210 can be reliably obtained, and the haze value can be easily controlled to an appropriate value. Therefore, gloss and interference colors are reliably suppressed, and good soft focus effect is obtained.

### [Appendix]

The measure for solving the foregoing problems embraces the following items as technical concepts derived from the fourth embodiment.

### (Item 21)

A skin-adhesive film including: a first surface; and a second surface, which is a surface opposite to the first surface, the second surface being configured to be adhered to a skin surface, wherein the skin-adhesive film has an average mass of 0.1 g/m² or more and 4.0 g/m² or less, and a haze of 7% or more and 65% or less.

With this configuration, due to the haze being 7% or more, the skin-adhesive film provides a light-scattering effect sufficient to suppress gloss and interference colors. Accordingly, a site on the skin where the skin-adhesive film is adhered is rendered inconspicuous, and the soft focus effect is improved. Further, due to the haze being 65% or less, the skin-adhesive film appears cloudy, and a site where the skin-adhesive film is adhered is rendered inconspicuous. As a result, the skin-adhesive film has increased utility in cosmetic applications.

### (Item 22)

The skin-adhesive film according to Item 21, wherein at least one of the first surface and the second surface has the arithmetic average roughness Ra of 0.15 µm or more and 4.00 µm or less.

With this configuration, a light-scattering effect on the surface of the skin-adhesive film can be reliably obtained, and the haze can be easily controlled to an appropriate degree. As a result, gloss and interference colors are reliably suppressed, and good soft focus effect is obtained.

### (Item 23)

A transfer sheet including: the skin-adhesive film according to Item 21 or 22; and a support substrate that supports the skin-adhesive film.

With this configuration, the skin-adhesive film is prevented from becoming deformed during storage and transport of the transfer sheet. Further, since the skin-adhesive film is supported by the support substrate, ease of handling of the skin-adhesive film is increased.

### (Fifth Embodiment)

When the skin-adhesive film is thin while ensuring the strength of the skin-adhesive film, the film has high tendency to follow uneven texture of the skin, and thus skin adhesion of the skin-adhesive film increases. On the other hand, when the skin-adhesive film is thick, moisturizing properties required for skin care, UV blocking effect, decorative effect required for makeup, and abrasion resistance against rubbing with a finger or a cloth increase. That is, skin adhesion derived from a thin film and properties derived from a thick film have a trade-off relationship, in which increasing one will decrease the other.

An object of the fifth embodiment is to provide a skin-adhesive film and a transfer sheet that balance skin adhesion and properties derived from a thick film.

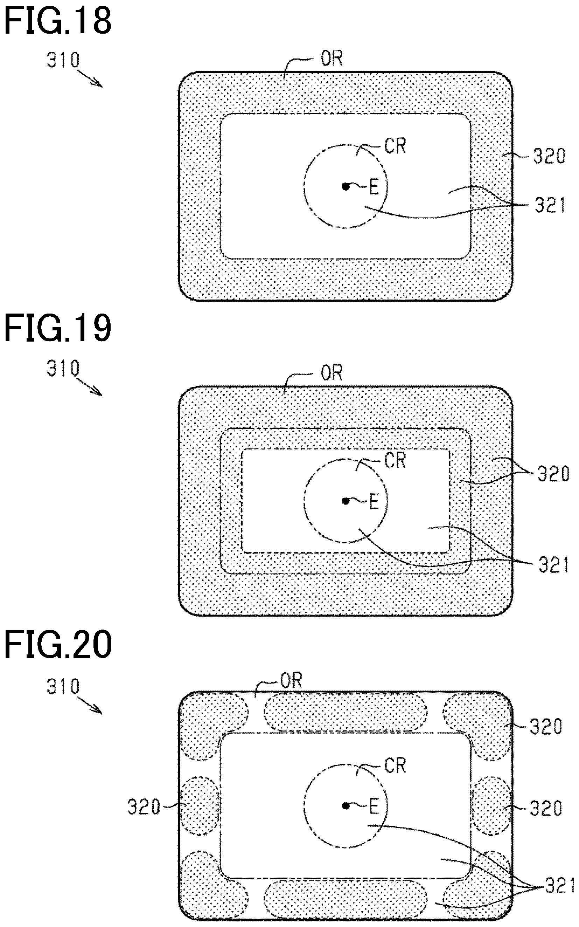

With reference to Figs. 18 to 37, the fifth embodiment of a skin-adhesive film and a transfer sheet will be described.

### [Schematic Configuration of Skin-Adhesive Film]

As shown in Fig. 18, in plan view perpendicular to a surface of the skin-adhesive film 310, a skin-adhesive film 310 includes an annular region OR, which is a peripheral region extending along of the outer periphery of the skin-adhesive film 310, and a center region CR located inside the annular region OR. The skin-adhesive film 310 includes a thin film portion 320 mainly located in the annular region OR, and a thick film portion 321 mainly located in the center region CR. In Figs. 18 to 21, a portion where the thin film portion 320 is located is shown dotted.

The annular region OR is a region having a strip shape with a predetermined width extending in a closed annular shape in the above plan view. The outer edge of the skin-adhesive film 310 coincides with the outer edge of the annular region OR. The center region CR includes a center of gravity E of a shape corresponding to the shape of the skin-adhesive film 310 in the above plan view.

The annular region OR is, for example, a region having a width of 3 mm. Alternatively, the annular region OR is a region having an area of 20% to a total area of the skin-adhesive film 310 in the above plan view. The center region CR is, for example, a circular region having a radius of 10 mm about the center of gravity E.

The shape of the skin-adhesive film 310 in the above plan view is not specifically limited, and the skin-adhesive film 310 may have a polygonal shape such as a rectangular shape, or may have a circular shape, or an elliptical shape. The skin-adhesive film 310 may also have a shape other than these shapes, and may have a shape surrounded by a straight shape or a curved shape.

The thin film portion 320 has an average thickness of 0.05 µm or more and less than 0.3 µm, and the thick film portion 321 has an average thickness of 0.3 µm or more. A maximum thickness of the thin film portion 320 is smaller than a maximum thickness of the thick film portion 321. The above average thickness is an average of the thicknesses of the skin-adhesive film 310 measured at a plurality of measurement points. The measurement points are provided, for example, at 5 or more positions per 10 cm².

The thin film portion 320 may be configured such that a plurality of regions intermittently arranged in the annular region OR in the circumferential direction thereof are included in the thin film portion 320.

For example, as shown in Fig. 18, the entirety of the annular region OR may be the thin film portion 320, and the entirety of a region inside the annular region OR may be the thick film portion 321. Alternatively, as shown in Fig. 19, the entirety of the annular region OR may be the thin film portion 320, and the thin film portion 320 may further extend to the inside of the annular region OR in the radial direction of the annular region OR. Further, the thick film portion 321 may be located inside the thin film portion 320. Alternatively, as shown in Fig. 20, a plurality of thin film portions 320 intermittently arranged in the circumferential direction of the annular region OR may be located in the annular region OR, and a portion other than the thin film portions 320 may be the thick film portion 321. In this case, the thick film portion 321 is located in the annular region OR in part.

As described above, when a plurality of target regions TS, which are regions intermittently arranged in the annular region OR in the circumferential direction thereof, are included in the thin film portion 320, an average of the film thicknesses of the skin-adhesive film 310 measured in each target region TS is 0.05 µm or more and less than 0.3 µm.

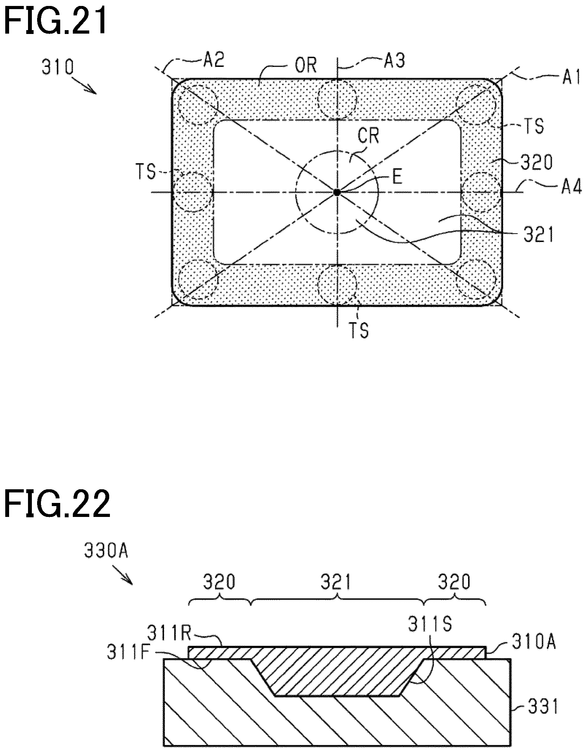

As shown in Fig. 21, the target region TS is a region virtually defined in the annular region OR, having a diameter which is the same as a width of the annular region OR, and having a center at a midpoint of the annular region OR in the width direction. Three measurement points are provided in one target region TS. The thicknesses at the respective measurement points are measured by using an optical thickness meter, for example.

The plurality of target regions TS are preferably evenly dispersed in the annular region OR. For example, as shown in Fig. 21, when a minimum rectangular shape in which the skin-adhesive film 310 is inscribed in plan view is virtually placed, the target regions TS are preferably arranged at eight positions, including four positions where diagonal lines A1 and A2 of the rectangular shape each intersect with the annular region OR, and four positions where straight lines A3 and A4, extending between midpoints of opposite sides of the rectangular shape, each intersect with the annular region OR.

Further, the thick film portion 321 may be at least partially located inside the annular region OR, but is preferably located in a region including the center region CR. That is, an average film thickness of the skin-adhesive film 310 measured in the center region CR may be 0.3 µm or more. When the center region CR is a circular region having a center at the center of gravity E and a radius of 10 mm, three measurement points are provided inside the center region CR.

According to the skin-adhesive film 310 of the present embodiment, since the thin film portion 320 has an average thickness of less than 0.3 µm, conformability to the texture of the skin increases, leading to good skin adhesion. Further, since the thin film portion 320 has an average thickness of 0.05 µm or more, it is possible to prevent excessive reduction in the strength of the thin film portion 320 while maintaining the thinness of the film.

The target regions TS in which the thin film portion 320 is located is included in the annular region OR, which is located at the edge of the skin-adhesive film 310. Therefore, skin adhesion at the edge of the skin-adhesive film 310 can be increased. In general, a skin-adhesive film, when adhered to the skin, tends to easily peel off from the edge. On the other hand, the skin-adhesive film 310 of the present embodiment has increased skin adhesion at the edge. Accordingly, the entirety of the skin-adhesive film 310 can be prevented from being peeled off from the skin.

In order to further enhance the skin adhesion at the edge of the skin-adhesive film 310, it is preferred that the entire annular region OR is the thin film portion 320. In this case, in view of increase in adhesion, the thin film portion 320 having an annular shape in plan view preferably has the width of 1 mm or more. Further, when a plurality of thin film portions 320 are intermittently arranged in the annular region OR as shown in Fig. 20, a plurality of thin film portions 320 are preferably evenly dispersed in the annular region OR. As described above, with a configuration in which the plurality of target regions TS evenly dispersed in the annular region OR are included in the thin film portion 320, skin adhesion at the edge of the skin-adhesive film 310 increases in a wide peripheral area of the film. Accordingly, the skin-adhesive film 310 is less likely to be peeled off from the skin.

On the other hand, since the thick film portion 321 has an average thickness of 0.3 µm or more, the thick film portion 321 in the skin-adhesive film 310 can exhibit moisturizing properties, UV blocking effect, and abrasion resistance against rubbing with a finger or a cloth. Further, when the skin-adhesive film 310 is colored in order to assist with makeup, good decorative effect can also be obtained by the skin-adhesive film 310. For further improvement in these properties, the average thickness of the thick film portion 321 is preferably twice or more the average thickness of the thin film portion 320. Further, in view of reducing discomfort that the user may feel when the skin-adhesive film 310 is adhered to the skin, the average thickness of the thick film portion 321 is preferably 5.0 µm or less.

In addition, in order to obtain the above properties in a larger area, a region where the thick film portion 321 is located is preferably larger than a circular region having a center at the center of gravity E and a diameter of 3 mm.

### [First Type]

In the following description, a first type to a thirteenth type will be described one by one as examples of specific structures of the skin-adhesive film 310, and the transfer sheet having the skin-adhesive film 310 described above. In the drawings, the thickness of the skin-adhesive film 310 is shown exaggerated.

A first type skin-adhesive film 310A shown in Fig. 22 is formed of a single layer, and the thin film portion 320 and the thick film portion 321 are made of the same material.

The material of the skin-adhesive film 310A is preferably a resin having low toxicity, skin irritation, and skin sensitization as well as water resistance so that the film does not easily dissolve with sweat. Examples of the material for the skin-adhesive film 310A include ester resins such as polylactic acid, polyglycolic acid, and polycaprolactone, and copolymer resins thereof. Further, resins for use as film-formers in cosmetics may also be used as the material for the skin-adhesive film 310A. Examples of such resins include acrylic resin, silicone, and copolymer resins thereof, and cellulose derivatives such as cellulose acetate, cellulose acetate propionate, and cellulose acetate butyrate. Further, examples of the material for the skin-adhesive film 310A include resins that are commonly used as materials for medical products, such as polycarbonate, cycloolefin copolymer, styrene-butadiene elastomer, and polyimide.

The skin-adhesive film 310A may contain functional materials that enhance functions of the film or add functions to the film. For example, the skin-adhesive film 310A may contain materials having moisturizing effect, such as hyaluronic acid, collagen, ceramide, pullulan, and sacran. With this configuration, a skin moisturizing effect due to the skin-adhesive film 310A is enhanced.

Further, for example, when the skin-adhesive film 310A has a configuration containing a UV absorber or a UV scattering agent, a UV blocking function of the skin-adhesive film 310A increases. Examples of the UV absorber include octyl methoxycinnamate, octyl dimethoxybenzylidene dioxoimidazolidine propionate, hexyl diethyl aminohydroxy benzoylbenzoate, t-butyl methoxydibenzoylmethane, octyl triazone, and 2-ethylhexyl paramethoxycinnamate, and examples of the UV scattering agent include titanium oxide and zinc oxide.

Further, for example, when the skin-adhesive film 310A has a decorative function such as a function as a concealer for concealing spots or the like, the skin-adhesive film 310A may contain a coloring pigment or an inorganic pigment.

Further, the skin-adhesive film 310A may contain beauty ingredients. Examples of the beauty ingredients include anti-wrinkle ingredients such as vitamin C and its derivatives, kojic acid, retinoic acid and their derivatives, whitening ingredients such as hydroquinone, vitamin A and its derivatives, α-lipoic acid, adenosine, and α-hydroxy acid. Further, the skin-adhesive film 310A may contain medicinal ingredients, including acne treatment ingredients such as salicylic acid, and anti-inflammatory ingredients such as glycyrrhetinic acid (salt). Furthermore, the skin-adhesive film 310A may contain stabilizers such as BHT (dibutyl hydroxy toluene), vitamin E, and tocopherol acetate, which is a vitamin E derivative.

The skin-adhesive film 310A has a first surface 311F, and a second surface 311R, which is a surface opposite to the first surface 311F. The second surface 311R constitutes a surface of the skin-adhesive film 310A, which is adhered to the skin. The first surface 311F is exposed to the outside when the skin-adhesive film 310A is adhered to the skin.

In Fig. 22, the second surface 311R is flat, and the first surface 311F has a projection and a recess corresponding to difference in thickness between the thin film portion 320 and the thick film portion 321. Alternatively, the first surface 311F may be flat, and the second surface 311R may have the above projection and recess, or both the first surface 311F and the second surface 311R may have the above projection and recess. The first surface 311F is preferably flat, in view of preventing the skin-adhesive film 310A from being torn by friction between the above projection and recess and a finger or a cloth.

In the first type skin-adhesive film 310A, the thin film portion 320 has a substantially uniform thickness, and the thick film portion 321 also has a substantially uniform thickness. The thickness of the skin-adhesive film 310A may change gradually or in a binary manner at the boundary between the thin film portion 320 and the thick film portion 321. In other words, a side face 311 S formed at the boundary between the thin film portion 320 and the thick film portion 321 may be inclined relative to the thickness direction of the skin-adhesive film 310A, or may extend in the thickness direction. The side face 311 S is preferably inclined, in view of preventing the skin-adhesive film 310A from being torn by friction between the side face 311 S and a finger or the like. Fig. 22 illustrates an example in which the side face 311S is inclined relative to the thickness direction.

A first type transfer sheet 330A includes the skin-adhesive film 310A, and a substrate 331 that supports the skin-adhesive film 310A. The support substrate 331 has an average thickness of, for example, 5 µm or more and 500 µm or less. The support substrate 331 has a function of reducing deformation of the skin-adhesive film 310A during storage of the transfer sheet 330A and transport of the skin-adhesive film 310A to a site to be adhered. Since the skin-adhesive film 310A is supported by the support substrate 331, ease of handling of the skin-adhesive film 310A can be increased.

The support substrate 331 supports the first surface 311F of the skin-adhesive film 310A. When the first surface 311F has a projection and a recess, a surface of the support substrate 331 in contact with the skin-adhesive film 310A may be flat, or may have a shape conforming to the above projection and recess. In Fig. 22, a surface of the support substrate 331 in contact with the skin-adhesive film 310A has a shape conforming to the projection and recess on the first surface 311F, that is, a shape having a recess, which is recessed along the projection on the first surface 311F. In other words, the projection on the skin-adhesive film 310A is embedded in the support substrate 331.

Further, in Fig. 22, the support substrate 331 extends to the outside of the first surface 311F of the skin-adhesive film 310A. That is, when viewed in a direction perpendicular to the second surface 311R of the skin-adhesive film 310A, the support substrate 331 is larger than the skin-adhesive film 310A, and the outer edge of the skin-adhesive film 310A is located inside the outer edge of the support substrate 331. Alternatively, when viewed in a direction perpendicular to the second surface 311R, the shape of the support substrate 331 may coincide with the shape of the skin-adhesive film 310A, and the outer edge of the support substrate 331 may be aligned with the outer edge of the skin-adhesive film 310A.

As the support substrate 331, for example, films made of polyethylene terephthalate, polyethylene naphthalate, polyethylene, polypropylene, cycloolefin polymer, cycloolefin copolymer, and nylon, non-woven fabric, paper, and the like are suitably used. The support substrate 331 may further include, on a surface of the layer made of these materials, a layer made of silicone, fluororesin, olefin, wax, film-former, water soluble resin, or the like for facilitating release of the support substrate 331 from the skin-adhesive film 310A. In particular, in view of ease of release from the skin-adhesive film 310A, the support substrate 331 preferably has a surface layer made of a release resin such as silicone or olefin, or the support substrate 331 is preferably made of a non-woven fabric or paper that easily swells by liquid such as water to increase releasability from the skin-adhesive film 310A. Further, the skin-adhesive film 310A and the support substrate 331 may be detachably fixed to each other via a weak pressure-sensitive adhesive or a weak adhesive. Further, the skin-adhesive film 310A and the support substrate 331 can be fixed to each other by applying heat and pressure.

Moreover, the transfer sheet 330A may include a protective layer that covers the second surface 311R of the skin-adhesive film 310A. The protective layer has a function of protecting the second surface 311R. The protective layer may be made of, for example, materials described above as the materials for the support substrate 331. The protective layer and the support substrate 331 may be made of the same material, or may be made of materials different from each other.

A method of adhering the skin-adhesive film 310A by using the transfer sheet 330A will be described below, taking the first type transfer sheet 330A as an example. The following description will be given for an example in which the skin-adhesive film 310A is adhered to the skin on which a cosmetic has been applied. The method of adhering the skin-adhesive film 310A described below is applied to a method of adhering the skin-adhesive film 310 of the second and subsequent types.

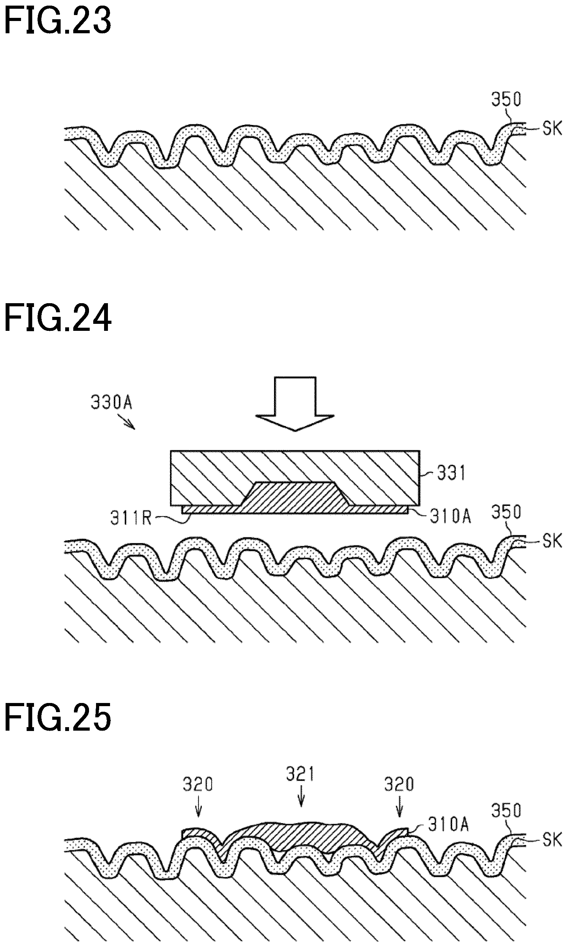

As shown in Fig. 23, a cosmetic 350 such as a lotion is first applied to the skin SK. Instead of the cosmetic 350, water may also be applied.

As shown in Fig. 24, subsequently, the transfer sheet 330A is pressed against the skin SK with the second surface 311R of the skin-adhesive film 310A facing the skin SK on which the cosmetic 350 has been applied. Then, the support substrate 331 is removed from the skin-adhesive film 310A.

Accordingly, as shown in Fig. 25, the skin-adhesive film 310A is transferred from the transfer sheet 330A to the skin SK, and the skin-adhesive film 310A is adhered to the skin SK. The skin-adhesive film 310A has the thin film portion 320 at the edge thereof. Since the thin film portion 320 is ultrathin, the thin film portion 320 follows the unevenness of the skin SK and is adhered to the skin SK. The thin film portion 320 has adhesion strength to a degree that it does not spontaneously detach from the skin SK. The thick film portion 321 does not follow the unevenness of the skin SK as much as the thin film portion 320 does. However, since the skin-adhesive film 310A is adhered to the skin SK at the edge where the thin film portion 320 is located, the skin-adhesive film 310A as a whole is held on the skin SK.

When the skin-adhesive film 310A covers the skin SK, it suppresses evaporation of sebum and sweat to provide a moisturizing effect. Further, the skin-adhesive film 310A can protect the skin SK from being exposed to harmful bacteria and substances. The skin-adhesive film 310A containing a pigment can provide a decorative effect. Thus, the skin-adhesive film 310A has functions of assisting in skin care and with makeup.

Further, the skin-adhesive film 310A may be adhered to the skin SK after the cosmetic 350 is applied to the skin as described above, or may be adhered to the skin SK before the cosmetic 350 is applied to the skin. In addition, after the skin-adhesive film 310A is adhered to the skin SK, a cosmetic such as foundation may be applied to the film.

### [Second Type]

The second type skin-adhesive film 310B and the transfer sheet 330B will be described, focusing on the difference from the first type. Further, configurations which are the same as those of the first type will be denoted by the same reference signs, and the description thereof will be omitted.

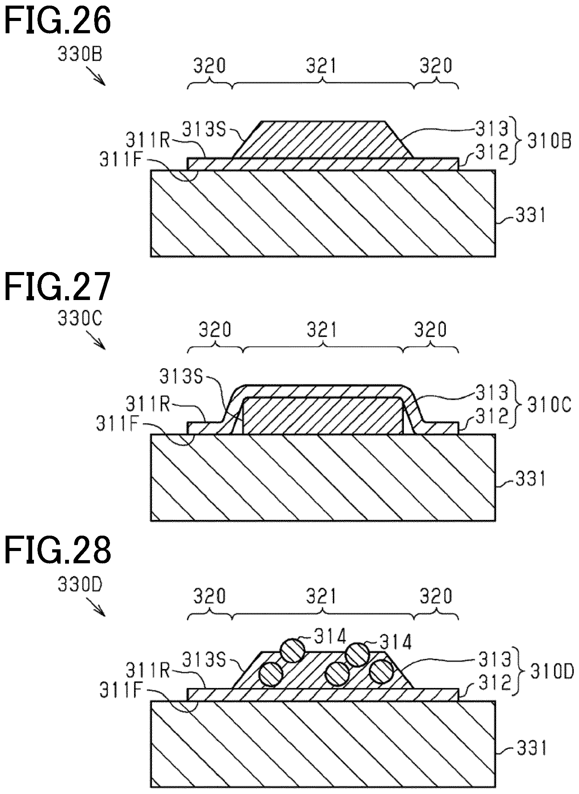

As shown in Fig. 26, a second type skin-adhesive film 310B has a two-layer structure, including a wide area layer 312 and a narrow area layer 313. When viewed in a direction perpendicular to the second surface 311R of the skin-adhesive film 310B, the wide area layer 312 is located on the entire skin-adhesive film 310B, and the narrow area layer 313 is located on part of the skin-adhesive film 310B. The wide area layer 312 is in contact with the support substrate 331, and the narrow area layer 313 is located on the wide area layer 312 on a side facing away from the support substrate 331. In other words, the wide area layer 312 and the narrow area layer 313 are disposed in this order in a direction from the first surface 311F of the skin-adhesive film 310B to the second surface 311R. The first surface 311F is entirely formed of the wide area layer 312. The second surface 311R is partially formed of the narrow area layer 313, and the remaining part of the second surface 311R is formed of the wide area layer 312.

For example, the wide area layer 312 and the narrow area layer 313 each have a substantially constant thickness. A difference in thickness of the skin-adhesive film 310B is formed by the narrow area layer 313 being stacked on the wide area layer 312 in part. That is, a portion of the wide area layer 312 on which the narrow area layer 313 is not disposed is the thin film portion 320. Thus, the thin film portion 320 is formed of only a portion of the wide area layer 312. On the other hand, a portion of the wide area layer 312 on which the narrow area layer 313 is disposed is the thick film portion 321. Thus, the thick film portion 321 is formed of a portion of the wide area layer 312 and the narrow area layer 313. Although the relationship between the thickness of the wide area layer 312 and the thickness of the narrow area layer 313 is not specifically limited, the average thickness of the narrow area layer 313 is preferably larger than the average thickness of the wide area layer 312 to provide a large difference in average thickness between the thin film portion 320 and the thick film portion 321.

An end face 313S of the narrow area layer 313 may be inclined relative to the thickness direction of the skin-adhesive film 310B, or may extend in the thickness direction. Fig. 26 illustrates an example in which the end face 313S is inclined relative to the thickness direction.

The transfer sheet 330B includes the skin-adhesive film 310B, and the support substrate 331 having the same configuration as that of the first type. As the skin-adhesive film 310B is transferred from the transfer sheet 330B to the skin, at a portion of the wide area layer 312 on which the narrow area layer 313 is disposed, that is, the thin film portion 320, the wide area layer 312 is in contact with the skin. Thus, the wide area layer 312 has a function of being in contact with the skin at the thin film portion 320. On the other hand, at a portion of the wide area layer 312 on which the narrow area layer 313 is disposed, that is, the thick film portion 321, the narrow area layer 313 is in contact with the skin and the narrow area layer 313 is covered with the wide area layer 312 Thus, the wide area layer 312 has a function of protecting the narrow area layer 313 at the thick film portion 321.

The wide area layer 312 and the narrow area layer 313 are made of materials described as materials for the first type skin-adhesive film 310A. The composition of the wide area layer 312 and the composition of the narrow area layer 313 may be the same, or may be different from each other. As described above, since the narrow area layer 313 is in contact with the skin while constituting the thick film portion 321, it is preferably made of a material that increases the properties derived from the thick film. Further, the wide area layer 312 is preferably made of a material suitable for the functions of adhesion to the skin or protecting the narrow area layer 313. For example, when the skin-adhesive film 310B is used mainly for moisturizing the skin, the narrow area layer 313 preferably contains resins or functional materials having moisturizing effect, and the wide area layer 312 preferably contains polylactic acid or copolymers thereof.

The thicknesses of the wide area layer 312 and the narrow area layer 313 may not necessarily be constant. Both the thin film portion 320 and the thick film portion 321 may be composed of the wide area layer 312 and the narrow area layer 313. That is, the thick film portion 321 may include a plurality of layers which include at least part of the wide area layer 312 and at least part of the narrow area layer 313.

### [Third Type]

The third type skin-adhesive film 310C and the transfer sheet 330C will be described, focusing on the difference from the second type. Further, configurations which are the same as those of the second type will be denoted by the same reference signs, and the description thereof will be omitted.

As shown in Fig. 27, in the third type skin-adhesive film 310C, the wide area layer 312 and the narrow area layer 313 are stacked in the order opposite from that in the second type. That is, the narrow area layer 313 is located on the wide area layer 312 on a side facing the support substrate 331. In other words, the wide area layer 312 and the narrow area layer 313 are disposed in this order in a direction from the second surface 311R of the skin-adhesive film 310C to the first surface 311F.

When viewed in a direction perpendicular to the second surface 311R, the wide area layer 312 extends across the entire skin-adhesive film 310C, and the narrow area layer 313 is located on part of the skin-adhesive film 310C. In other words, the narrow area layer 313 is in contact with the support substrate 331, and the wide area layer 312 covers the narrow area layer 313 and the outside thereof on the support substrate 331. The first surface 311F is partially formed of the narrow area layer 313, and the remaining part of the first surface 311F is formed of the wide area layer 312. The second surface 311R is entirely formed of the wide area layer 312.

For example, the wide area layer 312 and the narrow area layer 313 each have a substantially constant thickness. A difference in thickness of the skin-adhesive film 310C is formed by the wide area layer 312 being partially stacked on the narrow area layer 313. That is, a portion of the skin-adhesive film 310C on which the narrow area layer 313 is not disposed and which is formed of only the wide area layer 312 is the thin film portion 320, and a portion on which the wide area layer 312 and the narrow area layer 313 are disposed is the thick film portion 321. Although the relationship between the thickness of the wide area layer 312 and the thickness of the narrow area layer 313 is not specifically limited, the average thickness of the narrow area layer 313 is preferably larger than the average thickness of the wide area layer 312 to provide a large difference in average thickness between the thin film portion 320 and the thick film portion 321.

An end face 313S of the narrow area layer 313 may be inclined relative to the thickness direction of the skin-adhesive film 310C, or may extend in the thickness direction. Fig. 27 illustrates an example in which the end face 313 S extends in the thickness direction.

The transfer sheet 330C includes the skin-adhesive film 310C, and the support substrate 331 having the same configuration as that of the first type. In the skin-adhesive film 310C, the support substrate 331 is in contact with the wide area layer 312 at a portion of the first surface 311F formed of the wide area layer 312, and the support substrate 331 is in contact with the narrow area layer 313 at a portion of the first surface 311F formed of the narrow area layer 313. Further, the support substrate 331 may not necessarily be in contact with the entire surface of the first surface 311F. Although Fig. 27 illustrates that a gap is formed between the skin-adhesive film 310C and the support substrate 331 in a region surrounding the narrow area layer 313, such a gap may not be necessarily formed. The thickness of the skin-adhesive film 310C is a length between the first surface 311F and the second surface 311R in a solid portion of the film.

In the third type skin-adhesive film 310C, the wide area layer 312 has a function of adhering the skin at the thin film portion 320. The wide area layer 312 and the narrow area layer 313 are made of materials described as materials for the first type skin-adhesive film 310A. The composition of the wide area layer 312 and the composition of the narrow area layer 313 may be the same, or may be different from each other. As described above, since the narrow area layer 313 constitutes the thick film portion 321, it is preferably made of a material that increases the properties derived from the thick film. Further, the wide area layer 312 is preferably made of a material suitable for the function of adhesion to the skin.

The thicknesses of the wide area layer 312 and the narrow area layer 313 may not necessarily be constant. Both the thin film portion 320 and the thick film portion 321 may be composed of the wide area layer 312 and the narrow area layer 313. That is, the thick film portion 321 may include a plurality of layers which include at least part of the wide area layer 312 and at least part of the narrow area layer 313.

### [Fourth Type]

The fourth type skin-adhesive film 310D and the transfer sheet 330D will be described, focusing on the difference from the second type. Further, configurations which are the same as those of the second type will be denoted by the same reference signs, and the description thereof will be omitted.

As shown in Fig. 28, in the fourth type skin-adhesive film 310D, the narrow area layer 313 contains particles 314. The wide area layer 312 and narrow area layer 313 have the same configuration as that of the second type except that the narrow area layer 313 contains the particles 314. Further, the transfer sheet 330D includes the skin-adhesive film 310D, and the support substrate 331 having the same configuration as that of the first type.

The particles 314 are made of functional materials that enhance functions of the skin-adhesive film 310D or add functions to the film. For example, the particles 314 may be resin particles carrying UV scattering agents, coloring pigments, inorganic pigments, or medicinal ingredients. Since the narrow area layer 313 is in contact with the skin while constituting the thick film portion 321, the narrow area layer 313, which contains the particles 314 made of these functional materials, can increase functions applied from the skin-adhesive film 310D to the skin, or diversify these functions. In addition, since the narrow area layer 313 constitutes the thick film portion 321, it can be formed thicker than the wide area layer 312. Accordingly, the degree of freedom in types and contents of the particles 314 that can be contained can be increased compared with the case where the wide area layer 312 contains the particles 314.

Further, the particle size of the particle 314 may be smaller or larger than the thickness of a portion of the narrow area layer 313 which does not contain the particles 314. In addition, the particles 314 may be exposed from the surface of the narrow area layer 313 as shown in Fig. 28, or may not be exposed.

### [Fifth Type]

The fifth type skin-adhesive film 310E and the transfer sheet 330E will be described, focusing on the difference from the third type. Further, configurations which are the same as those of the third type will be denoted by the same reference signs, and the description thereof will be omitted.

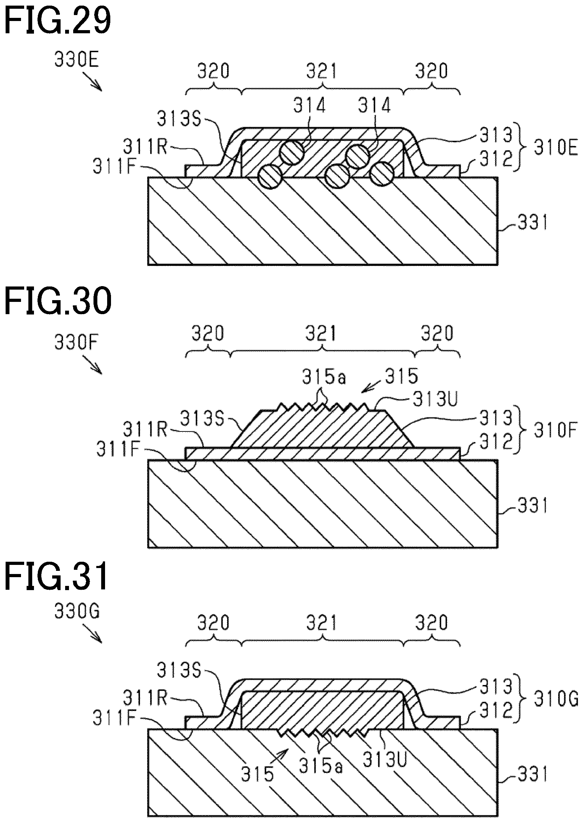

As shown in Fig. 29, in the fifth type skin-adhesive film 310E, the narrow area layer 313 contains the particles 314. The wide area layer 312 and narrow area layer 313 have the same configuration as that of the third type except that the narrow area layer 313 contains the particles 314. Further, the transfer sheet 330E includes the skin-adhesive film 310E, and the support substrate 331 having the same configuration as that of the first type.

The particles 314 are made of functional materials that enhance functions of the skin-adhesive film 310E or add functions to the film as with the fourth type. Since the narrow area layer 313 contains the particles 314 made of these functional materials, it can increase functions applied from the skin-adhesive film 310D to the skin, or diversify these functions. In addition, since the narrow area layer 313 constitutes the thick film portion 321, it can be formed thicker than the wide area layer 312. Accordingly, the degree of freedom in types and contents of the particles 314 that can be contained can be increased compared with the case where the wide area layer 312 contains the particles 314.

Further, the particle size of the particle 314 may be smaller or larger than the thickness of a portion of the narrow area layer 313 which does not contain the particles 314. In addition, the particles 314 may be exposed from the surface of the narrow area layer 313 as shown in Fig. 29, or may not be exposed. When the particles 314 are exposed from the surface of the narrow area layer 313 which is in contact with the support substrate 331, the asperities on the surface due to the particles 314 may or may not be embedded in the support substrate 331.

### [Sixth Type]

The sixth type skin-adhesive film 310F and the transfer sheet 330F will be described, focusing on the difference from the second type. Further, configurations which are the same as those of the second type will be denoted by the same reference signs, and the description thereof will be omitted.

As shown in Fig. 30, in the sixth type skin-adhesive film 310F, the narrow area layer 313 has an asperity structure 315 on an asperity-forming surface 313U, which is a surface on a side opposite to that facing the wide area layer 312. That is, the skin-adhesive film 310F has the asperity structure 315 on the second surface 311R. In Fig. 30, the asperity structure 315 is formed of a plurality of projections 315a protruding from the asperity-forming surface 313U. However, the asperity structure 315 may also be formed of a plurality of recesses that are recessed from the asperity-forming surface 313U, or may be formed of a combination of the above projections and recesses.

The wide area layer 312 and narrow area layer 313 have the same configuration as that of the second type except that the narrow area layer 313 has the asperity structure 315. Further, the transfer sheet 330F includes the skin-adhesive film 310F, and the support substrate 331 having the same configuration as that of the first type.

The asperity structure 315 has, for example, a function of scattering light. That is, since light is scattered by the asperity structure 315, a contour of an image behind the skin-adhesive film 310F becomes blurred. Accordingly, when the skin-adhesive film 310F is adhered to the skin, spots and wrinkles become less conspicuous. Further, since light is scattered by the asperity structure 315, the skin-adhesive film 310F can be prevented from appearing shiny, in other words, gloss on the skin-adhesive film 310F can be suppressed.

Moreover, the asperity structure 315 may have a function of puncturing the skin. That is, the projections 315a can be formed in a needle-like shape that can puncture the skin so that the projections 315a puncture the skin when the skin-adhesive film 310F is adhered to the skin. Then, as the projections 315a puncture the skin, beauty ingredients or medicinal ingredients included in the narrow area layer 313 or beauty ingredients or medicinal ingredients in a beauty essence applied to the skin are delivered into the skin through holes created by puncture. This facilitates permeation of the beauty ingredients or medicinal ingredients into the body.

The shape, size, arrangement pattern, and arrangement period of the projections and recesses constituting the asperity structure 315 may be set depending on the functions to be imparted to the asperity structure 315.

### [Seventh Type]

The seventh type skin-adhesive film 310G and the transfer sheet 330G will be described, focusing on the difference from the third type. Further, configurations which are the same as those of the third type will be denoted by the same reference signs, and the description thereof will be omitted.

As shown in Fig. 31, in the seventh type skin-adhesive film 310G, the narrow area layer 313 has the asperity structure 315 on the asperity-forming surface 313U, which is a surface on a side opposite to that facing the wide area layer 312. That is, the skin-adhesive film 310G has the asperity structure 315 on the first surface 311F. In Fig. 31, the asperity structure 315 is formed of a plurality of projections 315a protruding from the asperity-forming surface 313U. However, the asperity structure 315 may also be formed of a plurality of recesses that are recessed from the asperity-forming surface 313U, or may be formed of a combination of the above projections and recesses.

The wide area layer 312 and narrow area layer 313 have the same configuration as that of the third type except that the narrow area layer 313 has the asperity structure 315. Further, the transfer sheet 330G includes the skin-adhesive film 310G, and the support substrate 331 having the same configuration as that of the first type. In Fig. 31, a surface of the support substrate 331 follows the shape of the asperity structure 315, that is, regions between the projections 315a are filled with the support substrate 331. However, the asperity structure 315 may not necessarily be embedded in the support substrate 331.

The asperity structure 315 has, for example, a function of scattering light. That is, since light is scattered by the asperity structure 315, a contour of an image behind the skin-adhesive film 310F becomes blurred. Accordingly, when the skin-adhesive film 310G is adhered to the skin, spots and wrinkles become less conspicuous.

The shape, size, arrangement pattern, and arrangement period of the projections and recesses constituting the asperity structure 315 may be set depending on the functions to be imparted to the asperity structure 315.

### [Eighth Type]

The eighth type skin-adhesive film 310H and the transfer sheet 330H will be described, focusing on the difference from the second type. Further, configurations which are the same as those of the second type will be denoted by the same reference signs, and the description thereof will be omitted.

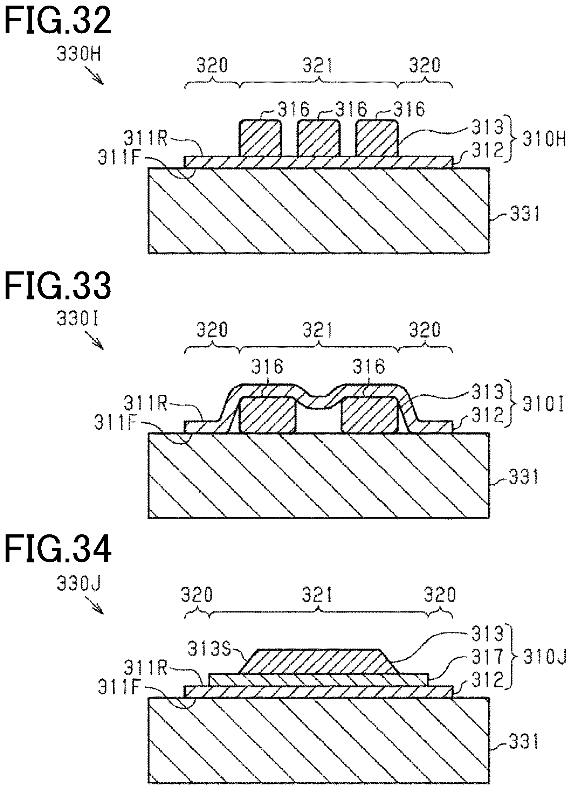

As shown in Fig. 32, in the eighth type skin-adhesive film 310H, the narrow area layer 313 is composed of a plurality of film pieces 316. The plurality of film pieces 316 are disposed spaced from each other on the wide area layer 312. The shape and size of the film piece 316 in plan view, and the arrangement pattern and arrangement period of the plurality of film pieces 316 are not specifically limited. However, a width of a gap between the adjacent film pieces 316 is preferably a size that cannot be easily recognized by visual observation, for example 200 µm or less, so that the strength of the skin-adhesive film 310H is not locally reduced in the thick film portion 321. Further, in Fig. 32, a size of the gap between the film pieces 316 is shown exaggerated.

The wide area layer 312 and narrow area layer 313 have the same configuration as that of the second type except that the narrow area layer 313 is divided into the plurality of film pieces 316. Further, the transfer sheet 330H includes the skin-adhesive film 310H, and the support substrate 331 having the same configuration as that of the first type.

Since the narrow area layer 313 is divided into the plurality of film pieces 316, the conformability of the thick film portion 321 to the skin increases, which reduces the discomfort such as a feeling of skin tightness felt by the user. From the viewpoint of enhancing this effect, the plurality of film pieces 316 are preferably arranged in a dot pattern as viewed in a direction perpendicular to the second surface 311R.

The wide area layer 312 may be in contact with the skin at least in a portion that is located in the annular region OR when the skin-adhesive film 310H is adhered to the skin, and a portion of the narrow area layer 313 exposed between the adjacent film pieces 316 may not be in contact with the skin.

### [Ninth Type]

The ninth type skin-adhesive film 3101 and the transfer sheet 3301 will be described, focusing on the difference from the third type. Further, configurations which are the same as those of the third type will be denoted by the same reference signs, and the description thereof will be omitted.

As shown in Fig. 33, in the ninth type skin-adhesive film 3101, the narrow area layer 313 is composed of a plurality of film pieces 316. The plurality of film pieces 316 are disposed spaced from each other on the support substrate 331. The shape and size of the film piece 316 in plan view, and the arrangement pattern and arrangement period of the plurality of film pieces 316 are not specifically limited. However, a width of a gap between the adjacent film pieces 316 is preferably a size that cannot be easily recognized by visual observation, for example 200 µm or less, so that the strength of the skin-adhesive film 310H is not locally reduced in the thick film portion 321. Further, in Fig. 33, a size of the gap between the film pieces 316 is shown exaggerated.

The wide area layer 312 and narrow area layer 313 have the same configuration as that of the third type except that the narrow area layer 313 is divided into the plurality of film pieces 316. Further, the transfer sheet 330I includes the skin-adhesive film 3101, and the support substrate 331 having the same configuration as that of the first type.

Since the narrow area layer 313 is divided into the plurality of film pieces 316, the conformability of the thick film portion 321 to the skin increases, which reduces the discomfort such as a feeling of skin tightness felt by the user.

### [Tenth Type]

The tenth type skin-adhesive film 310J and the transfer sheet 330J will be described, focusing on the difference from the second type. Further, configurations which are the same as those of the second type will be denoted by the same reference signs, and the description thereof will be omitted.

As shown in Fig. 34, the tenth type skin-adhesive film 310J includes an intermediate layer 317 between the wide area layer 312 and the narrow area layer 313. The intermediate layer 317 has a function as an anchor layer, that is, a function of increasing layer-to-layer adhesion in the layers constituting the skin-adhesive film 310J. The intermediate layer 317 may be sandwiched between the wide area layer 312 and the narrow area layer 313 at least in a region in which the narrow area layer 313 is located. That is, when viewed in a direction perpendicular to the second surface 311R, the intermediate layer 317 may be overlapped at least with the entire narrow area layer 313, and may be larger than the narrow area layer 313 as shown in Fig. 34. In the skin-adhesive film 310J, a portion that having a three-layer structure including the wide area layer 312, the intermediate layer 317, and the narrow area layer 313 constitutes the thick film portion 321. A portion having a two-layer structure including the narrow area layer 313 and the intermediate layer 317 may constitute the thin film portion 320, or may constitute the thick film portion 321 depending on the thicknesses of the narrow area layer 313 and the intermediate layer 317. A portion in which only the wide area layer 312 is located constitutes the thin film portion 320.

The wide area layer 312 and narrow area layer 313 have the same configuration as that of the second type except that the intermediate layer 317 is sandwiched between the wide area layer 312 and the narrow area layer 313. The transfer sheet 330J includes the skin-adhesive film 310J, and the support substrate 331 having the same configuration as that of the first type.

In the tenth type, the wide area layer 312 and the narrow area layer 313 have compositions different from each other. The intermediate layer 317 is configured to have adhesion to the narrow area layer 313 higher than adhesion of the wide area layer 312 to the narrow area layer 313, and have adhesion to the wide area layer 312 higher than adhesion of the narrow area layer 313 to the wide area layer 312. For example, when the wide area layer 312 is made of a resin having high water resistance, and the narrow area layer 313 contains hyaluronic acid or collagen, which is a water soluble constituent having high moisturizing effect, the adhesion between the wide area layer 312 and the narrow area layer 313 may be lowered. In this case, the intermediate layer 317 made of a copolymer of a water soluble resin and a hydrophobic resin, or made of a material obtained by mixing a water soluble resin with a hydrophobic resin can be used to increase layer-to-layer adhesion in the layers constituting the skin-adhesive film 310J.

In addition, a configuration having the intermediate layer 317 may be applied to the third type. That is, the intermediate layer 317 may be sandwiched between the wide area layer 312 and the narrow area layer 313 in a configuration in which the wide area layer 312 and the narrow area layer 313 are disposed in this order in a direction from the second surface 311R to the first surface 311F.

### [Eleventh Type]

The eleventh type skin-adhesive film 310K and the transfer sheet 330K will be described, focusing on the difference from the second type. Further, configurations which are the same as those of the second type will be denoted by the same reference signs, and the description thereof will be omitted.

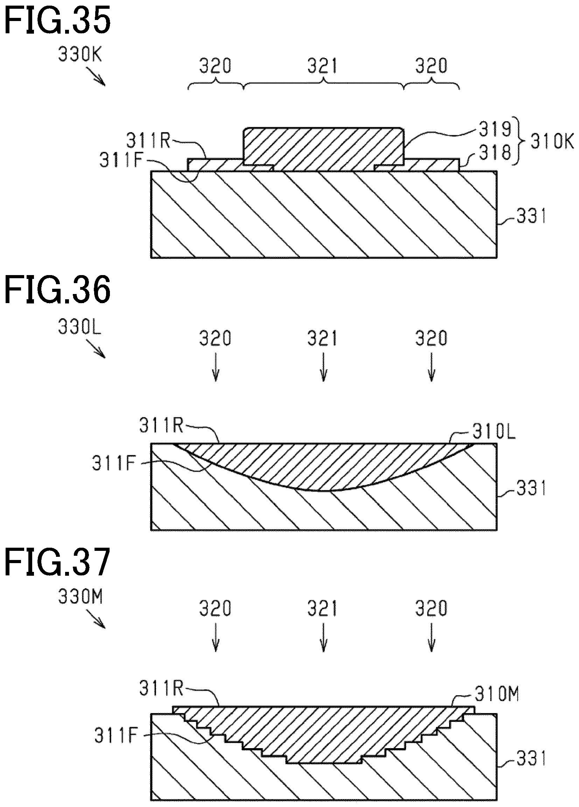

As shown in Fig. 35, the eleventh type skin-adhesive film 310K includes an end component 318 and a center component 319 having compositions different from each other. Both the end component 318 and the center component 319 are in contact with the support substrate 331. When viewed in a direction perpendicular to the second surface 311R, the end component 318 is located at least in the annular region OR, and the center component 319 is located at least in the center region CR. The end component 318 and the center component 319 are connected to each other at their ends.

The end component 318 and the center component 319 are in contact with each other in a plane parallel to the extending direction of the skin-adhesive film 310K, that is, in a plane parallel with the surface of the support substrate 331. Specifically, the end component 318 and the center component 319 are in contact with each other in the first surface 311F. That is, the first surface 311F is composed of the end component 318 and the center component 319. Thus, a portion of the skin-adhesive film 310K near the first surface 311F, extending along the surface of the support substrate 331, is made of materials different in part.

For example, the thickness of the end component 318 is substantially constant, and a portion of the end component 318 except for a connecting section to the center component 319 constitutes the thin film portion 320. Further, the thickness of the center component 319 is substantially constant and larger than the end component 318, and the center component 319 constitutes the thick film portion 321.

The transfer sheet 330K includes the skin-adhesive film 310K, and the support substrate 331 having the same configuration as that of the first type.

The end component 318 and the center component 319 are made of materials described as materials for the first type skin-adhesive film 310A. Since the skin-adhesive film 310K of the eleventh type is made of materials different in part in the same plane, air permeability through the skin-adhesive film 310K can be increased compared with a configuration in which layers made of different materials are laminated in the thickness direction. That is, the user is not likely to feel tightness at a site where the skin-adhesive film 310K is adhered.

The thicknesses of the end component 318 and the center component 319 may not necessarily be constant. Further, the end component 318 may partially constitute the thick film portion 321, and the center component 319 may partially constitute the thin film portion 320,

In addition, the end component 318 and the center component 319 may be in contact with each other in a plane spaced from the support substrate 331. Thus, the end component 318 and the center component 319 may be in contact with each other, not only in the first surface 311F, but also in a plane parallel to the extending direction of the skin-adhesive film 310K.

### [Twelfth Type]

The twelfth type skin-adhesive film 310L and the transfer sheet 330L will be described, focusing on the difference from the first type. Further, configurations which are the same as those of the first type will be denoted by the same reference signs, and the description thereof will be omitted.

As shown in Fig. 36, the twelfth type skin-adhesive film 310L has a thickness increasing continuously from the edge to the center region CR as viewed in a direction perpendicular to the second surface 311R. In other words, the thickness of the skin-adhesive film 310L increases continuously from the minimum thickness to the maximum thickness of the skin-adhesive film 310L. In the center region CR, the skin-adhesive film 310L is convex toward the support substrate 331. That is, in the cross-section taken along the thickness direction, the first surface 311F has a curved shape protruding toward the support substrate 331, and the second surface 311R has a flat shape.

According to this configuration, the first surface 311F and the second surface 311R of the skin-adhesive film 310 do not have stepped portions attributed to difference in thickness. Therefore, boundaries between different thicknesses are not likely to be recognized by visual observation. Further, when the skin-adhesive film 310L is adhered to the skin, the skin-adhesive film 310L is prevented from being torn by friction between the stepped portions and a finger or the like.

The skin-adhesive film 310L is made of materials described as materials for the first type skin-adhesive film 310A. Further, the transfer sheet 330L includes the skin-adhesive film 310L, and the support substrate 331 having the same configuration as that of the first type. In Fig. 36, a surface of the support substrate 331 follows the shape of the first surface 311F of the skin-adhesive film 310L, that is, the skin-adhesive film 310L is embedded in the support substrate 331. However, the skin-adhesive film 310L may not necessarily be embedded in the support substrate 331.

Even when the thickness of the skin-adhesive film 310L continuously varies as in the twelfth type, the target regions TS are regarded as being included in the thin film portion 320 as long as an average of the film thicknesses of the skin-adhesive film 310L measured in each of the plurality of target regions TS in the annular region OR is 0.05 µm or more and less than 0.3 µm. Further, the skin-adhesive film 310L is regarded as having the thick film portion 321 as long as an average of the film thicknesses of the skin-adhesive film 310L measured in the center region CR is 0.3 µm or more.

In addition, the skin-adhesive film 310L may have a shape which is convex in a direction away from the support substrate 331, or may have a shape which is convex in both directions toward the support substrate 331 and away from the support substrate 331. In other words, in the cross-section taken along the thickness direction, the second surface 311R may be curved and the first surface 311F may be flat, or both the first surface 311F and the second surface 311R may be curved.

### [Thirteenth Type]

The thirteenth type skin-adhesive film 310M and the transfer sheet 330M will be described, focusing on the difference from the first type. Further, configurations which are the same as those of the first type will be denoted by the same reference signs, and the description thereof will be omitted.

As shown in Fig. 37, the thirteenth type skin-adhesive film 310M has a thickness increasing stepwise from the edge to the center region CR in plan view as viewed in a direction perpendicular to the second surface 311R. In other words, the thickness of the skin-adhesive film 310M increases stepwise from the minimum thickness to the maximum thickness of the skin-adhesive film 310M. In the center region CR, the skin-adhesive film 310M is convex toward the support substrate 331. That is, in the cross-section taken along the thickness direction, the first surface 311F has a stepped shape formed of a plurality of steps, and the second surface 311R has a flat shape.

The skin-adhesive film 310M may be formed of a plurality of layers. When the skin-adhesive film 310M is formed by laminating a plurality of flat layers, the skin-adhesive film 310M can be produced easily compared with a method of producing the skin-adhesive film 310M by using a mold having fine stepped portions. Accordingly, the production cost can be reduced.

The skin-adhesive film 310M is made of materials described as materials for the first type skin-adhesive film 310A. Further, the transfer sheet 330M includes the skin-adhesive film 310M, and the support substrate 331 having the same configuration as that of the first type. In Fig. 37, a surface of the support substrate 331 follows the shape of the first surface 311F of the skin-adhesive film 310M, that is, asperities on the first surface 311F of the skin-adhesive film 310M are embedded in the support substrate 331. However, the asperities may not necessarily be embedded in the support substrate 331.

Even when the thickness of the skin-adhesive film 310M varies stepwise as in the thirteenth type, the target regions TS are regarded as being included in the thin film portion 320 as long as an average of the film thicknesses of the skin-adhesive film 310L measured in each of the plurality of target regions TS in the annular region OR is 0.05 µm or more and less than 0.3 µm. Further, the skin-adhesive film 310M is regarded as having the thick film portion 321 as long as an average of the film thicknesses of the skin-adhesive film 310M measured in the center region CR is 0.3 µm or more.

In addition, the skin-adhesive film 310M may have a shape which is convex in a direction away from the support substrate 331, or may have a shape which is convex in both directions toward the support substrate 331 and away from the support substrate 331. In other words, in the cross-section taken along the thickness direction, the second surface 311R may have a stepped shape and the first surface 311F may be flat, or both the first surface 311F and the second surface 311R may have a stepped shape.

### [Method for Producing Transfer Sheet]

A method for producing the above skin-adhesive film and the transfer sheet will be described below.

The skin-adhesive film 310 can be produced by thin film formation methods such as melt extrusion and melt casting. For production of thin layers or patterned layers as in the present embodiment, melt casting is preferably used. That is, materials for the skin-adhesive film 310 are dissolved or dispersed in a solvent to form a coating solution, and the coating solution is applied to the substrate to form a coating film, which is then dried and cured.

The coating solution can be applied to the substrate by known coating methods such as direct gravure, reverse gravure, small diameter reverse gravure, Mayer coating, die, curtain, spray, spin coating, screen printing, comma, knife, gravure offset, and roll coating.

The thickness of the layer to be formed is controlled by the proportion of the solid content contained in the coating solution and the coating method. In the case where the skin-adhesive film 310 is formed of a plurality of layers, application of the coating solution and drying of the coating film are repeated for each layer. When layers are partially formed, that is, when patterned layers are formed, suitable coating methods include direct gravure, spray, screen printing, and gravure offset.

As a film-formation substrate, the support substrate 331 may be used, or a substrate different from the support substrate 331 may be used.

In formation of the skin-adhesive film 310 on the support substrate 331, the support substrate 331 can be a substrate that is non-porous, and has recesses formed in reversed shapes of the asperities provided on the skin-adhesive film 310 to be formed. Thus, the support substrate 331 can be used as a die to form the skin-adhesive film 310 having locally different thickness.

In formation of the skin-adhesive film 310 on a substrate different from the support substrate 331, the transfer sheet is formed by transferring the skin-adhesive film 310 from the substrate in film formation onto the support substrate 331. When the support substrate 331 is a highly flexible substrate, such as a substrate made of a soft urethane or a non-woven fabric, it is preferred that the skin-adhesive film 310 formed on a substrate different from the support substrate 331 is transferred to the support substrate 331. As the substrate in film formation, a resin substrate such as a flat olefin film or a polyethylene terephthalate film can be used.

Examples of the method for forming the skin-adhesive film 310 having locally different thickness on a flat substrate include the following methods in addition to the method described above, in which a plurality of layers are formed by applying the coating solution plurality of times. For example, the examples include a method of forming a plurality of layers on different substrates, and then transferring the layers onto the support substrate 331 in sequence; a method of using a printing plate used for printing methods such as screen printing, the printing plate having a dot density that varies stepwise to locally change the amount of the coating solution to be applied; and a method of performing smoothing while varying a drying time.

A configuration in which the asperities on the skin-adhesive film 310 are embedded in the support substrate 331 is formed by a method which uses the support substrate 331 as a die to form the skin-adhesive film 310 as described above. When a highly flexible substrate is used as the support substrate 331, the surface of the support substrate 331 is likely to follow the surface shape of the skin-adhesive film 310. In this case, a configuration in which the asperities on the skin-adhesive film 310 are embedded in the support substrate 331 can be formed by transferring the skin-adhesive film 310 formed on a substrate different from the support substrate 331 onto the support substrate 331.

Further, when the skin-adhesive film 310 has the asperity structure 315, the asperity structure 315 can be formed by using a plate having a surface on which recesses and projections are formed in reversed shapes of the asperities to be formed, and filling the plate with a coating solution or pressing the plate against a surface of the layer on which coating has been applied.

### [Other Embodiments]

The above first type to the thirteenth type can be implemented in combination. For example, when the fourth type is combined with the eighth type, the narrow area layer 313 contains the particles 314, and is divided into a plurality of film pieces 316.

As illustrated in the sixth type or the seventh type, the thickness of the thick film portion 321 may not necessarily be constant in the thick film portion 321. Similarly, the thickness of the thin film portion 320 may not necessarily be constant in the thin film portion 320. In short, when an average of the film thickness of the plurality of target regions TS in the annular region OR is 0.05 µm or more and less than 0.3 µm, the target regions TS are regarded as being included in the thin film portion 320. Further, when an average of the film thicknesses in the center region CR, which is a region inside the annular region OR, is 0.3 µm or more, the skin-adhesive film 310 is regarded as having the thick film portion 321. As long as satisfying these conditions, the skin-adhesive film 310 may have a structure different from that in the first type to the thirteenth type.

### [Examples]

The skin-adhesive film and the transfer sheet of the fifth embodiment will be described by using specific examples and comparative examples.

### (Example 5-1)

In example 5-1, a skin-adhesive film and a transfer sheet corresponding to the second type were formed.

First, poly-DL-lactic acid was dissolved in ethyl acetate to generate a coating solution. A polyethylene terephthalate film having a surface coated with polyvinyl alcohol was used as a support substrate. The coating solution was applied to the support substrate in a pattern of a 60 mm diameter circle by a direct gravure method. The coating film was dried with hot air to form a wide area layer having an average thickness of 0.05 µm.

Subsequently, the coating solution was applied to the wide area layer in a pattern of a 58 mm diameter circle concentrically with the wide area layer by a direct gravure method. The coating film was dried with hot air to form a narrow area layer having an average thickness of 0.3 µm. Thus, a transfer sheet of Example 5-1 in which the wide area layer and the narrow area layer were laminated in this order on the support substrate was obtained. The transfer sheet of Example 5-1 has the thin film portion in a 1 mm-width annular shape at the edge in plan view, and a thick film portion having a 58-mm diameter circular shape at the center in plan view.

### (Example 5-2)

In example 5-2, a skin-adhesive film and a transfer sheet corresponding to the third type were formed.

First, poly-DL-lactic acid was dissolved in ethyl acetate to generate a coating solution. A polyethylene terephthalate film having a silicone release layer on a surface was used as a support substrate. The coating solution was applied to the entire surface of the substrate having a 300 mm width by a direct gravure method. The coating film was dried with hot air to form a wide area layer having an average thickness of 0.2 µm.

Subsequently, a hyaluronic acid aqueous solution was applied to the wide area layer in a pattern of a 50 mm diameter circle by a screen printing method. The coating film was dried with hot air to form a narrow area layer having an average thickness of 1.0 µm. Then, a laminate made of the wide area layer and the narrow area layer was transferred to a non-woven fabric used as the support substrate. The laminate made of the support substrate, the narrow area layer, and the wide area layer was punched out into a circular shape of 60 mm diameter concentrically with the narrow area layer. Thus, a transfer sheet of Example 5-2 in which the narrow area layer and the wide area layer were laminated in this order on the support substrate was obtained. The transfer sheet of Example 5-2 has the thin film portion in a 5 mm-width annular shape at the edge in plan view, and a thick film portion having a 50-mm diameter circular shape at the center in plan view.

### (Comparative Example 5-1)

Poly-DL-lactic acid was dissolved in ethyl acetate to generate a coating solution. A polyethylene terephthalate film having a silicone release layer on a surface was used as a support substrate. The coating solution was applied to the substrate by a micro gravure method. The coating film was dried with hot air to form a polylactic acid layer having an average thickness of 0.2 µm. The polylactic acid layer was transferred to a non-woven fabric used as the support substrate. Then, a laminate made of the support substrate and the polylactic acid layer was punched out into a circular shape of 60 mm diameter to obtain a transfer sheet of Comparative Example 5-1. The skin-adhesive film, which is the polylactic acid layer in the transfer sheet of Comparative Example 5-1, does not have a thick film portion.

### (Comparative Example 5-2)

Poly-DL-lactic acid was dissolved in ethyl acetate to generate a coating solution. A polyethylene terephthalate film having a silicone release layer on a surface was used as a support substrate. The coating solution was applied to the substrate by a micro gravure method. The coating film was dried with hot air to form a polylactic acid layer having an average thickness of 0.6 µm. The polylactic acid layer was transferred to a non-woven fabric used as the support substrate. Then, a laminate made of the support substrate and the polylactic acid layer was punched out into a circular shape of 60 mm diameter to obtain a transfer sheet of Comparative Example 5-2. The skin-adhesive film, which is the polylactic acid layer in the transfer sheet of Comparative Example 5-2, does not have a thin film portion.

### (Comparative Example 5-3)

Poly-DL-lactic acid was dissolved in ethyl acetate to generate a coating solution. A polyethylene terephthalate film having a surface coated with polyvinyl alcohol was used as a support substrate. The coating solution was applied to the support substrate in a pattern of a 60 mm diameter circle by a direct gravure method. The coating film was dried with hot air to form a polylactic acid layer having an average thickness of 0.1 µm.

Subsequently, the coating solution was applied to the polylactic acid layer in a pattern of a 58 mm diameter circle concentrically with the polylactic acid layer by a direct gravure method. The coating film was dried with hot air to form a polylactic acid layer having an average thickness of 0.05 µm. Thus, a transfer sheet of Comparative Example 5-3 having a skin-adhesive film made of two polylactic acid layers on the support substrate was obtained. The skin-adhesive film of Comparative Example 5-3 does not have a thick film portion.

### (Comparative Example 5-4)

Poly-DL-lactic acid was dissolved in ethyl acetate to generate a coating solution. A polyethylene terephthalate film having a silicone release layer on a surface was used as a support substrate. The coating solution was applied to the entire surface of the substrate by a direct gravure method. The coating film was dried with hot air to form a polylactic acid layer having an average thickness of 0.2 µm.

Subsequently, a hyaluronic acid aqueous solution was applied to the entire surface of the polylactic acid layer by a micro gravure method. The coating film was dried with hot air to form a hyaluronic acid layer having an average thickness of 1.0 µm. A laminate made of the polylactic acid layer and the hyaluronic acid layer was transferred to a non-woven fabric used as the support substrate. Then, the laminate made of the support substrate, the hyaluronic acid layer, and the polylactic acid layer was punched out into a circular shape of 60 mm diameter. Thus, a transfer sheet of Comparative Example 5-4 in which the hyaluronic acid layer and the polylactic acid layer were laminated in this order on the support substrate was obtained. The skin-adhesive film of Comparative Example 5-4 does not have a thin film portion.

### <Evaluation Method>

### (Adhesion)

The skin of the arm was moistened with water, and the transfer sheets of the respective examples and comparative examples were placed with the skin-adhesive film in contact with the arm. Then, the support substrate was removed so that the skin-adhesive film was adhered to the arm. The edge of the adhered skin-adhesive film was rubbed with a finger. The case where the film was not peeled at the edge was evaluated as "good," and the case where the film was peeled was evaluated as "poor."

### (Breaking strength)

For the respective examples and comparative examples, the breaking strength was measured as an index of resistance to breakage of the skin-adhesive film. The breaking strength is a minimum force required to break the film, and is measured by the following procedure. The support substrate was removed from the transfer sheet to obtain a skin-adhesive film in a circular shape of 6 mm diameter in plan view. The skin-adhesive film having the above circular shape as a sample was placed in a table-top compression and tensile tester (manufactured by Shimazu Corporation: EZ-Test, Load cell: 100N). The cylindrical indenter having a diameter of 3 mm was inserted into the sample at a compression speed of 10 mm/min. A strength (mN) at the time when the sample was ruptured was measured to obtain a breaking strength. When the breaking strength was 50 mN or more, the skin-adhesive film is regarded as having a film strength enough to withstand daily use. Accordingly, a breaking strength of 50 mN or more was evaluated as "good," and a breaking strength of less than 50 mN was evaluated as "poor." A larger breaking strength indicates a higher abrasion resistance.

### <Evaluation Results>

Table 7 shows the results of adhesion and breaking strength for the examples and comparative examples.

**[Table 7]**

| | Adhesion | Breaking strength |
|---|---|---|
| Example 5-1 | Good | Good |
| Example 5-2 | Good | Good |
| Comparative example 5-1 | Good | Poor |
| Comparative example 5-2 | Poor | Good |
| Comparative example 5-3 | Good | Poor |
| Comparative example 5-4 | Poor | Good |

As seen from Table 7, Examples 5-1 and 5-2 having the thin film portion at the edge and having the thick film portion at the center have good adhesion and good breaking strength. On the other hand, Comparative Examples 5-1 and 5-3 having only the thin film portion have good adhesion, but the breaking strength is poor. Further, Comparative Examples 5-2 and 5-4 having only thick film portion have good breaking strength, but the adhesion is poor.

As described above, it was found that Examples 5-1 and 5-2 having the thin film portion at the edge and having the thick film portion at the center can balance the skin adhesion derived from the thin film with the properties derived from the thick film.

As described in the above embodiments and examples, the skin-adhesive film and the transfer sheet of the fifth embodiment can provide the following advantages.
(5-1) The skin-adhesive film 310 includes the thin film portion 320 and the thick film portion 321, and the plurality of target regions TS arranged in the annular region OR in the circumferential direction thereof are included in the thin film portion 320. With this configuration, since the skin-adhesive film 310 has a small thickness at the edge of the skin-adhesive film 310, in which the thin film portion 320 is located, skin adhesion is increased. On the other hand, the thick film portion 321 can provide improvement in moisturizing properties, UV-blocking effect, decorative effect, and abrasion resistance. Accordingly, when the skin-adhesive film 310 is adhered to the skin, the film can successfully obtain the above properties while preventing peeling at the edge of the film. Thai is, both the skin adhesion derived from the thin film and the properties derived from the thick film can be achieved. As a result, the skin-adhesive film 310 has increased utility in cosmetic applications.
(5-2) when the thin film portion 320 has an annular shape extending along an outer edge of the skin-adhesive film 310, and the thin film portion 320 has a width of 1 mm or more as viewed in a direction perpendicular to the surface of the skin-adhesive film 310, skin adhesion at the edge of the skin-adhesive film 310 increases in the entire peripheral area of the film. Accordingly, when the skin-adhesive film 310 is adhered to the skin, the skin-adhesive film 310 is less likely to be peeled off from the skin.
(5-3) When the skin-adhesive film 310 includes the wide area layer 312 extending across the entire skin-adhesive film 310, and the narrow area layer 313 located on part of the skin-adhesive film 310, and the thick film portion 321 includes a plurality of layers including at least part of the wide area layer 312 and at least part of the narrow area layer 313, the skin-adhesive film 310 having a locally different thickness can be readily and appropriately achieved by laminating a plurality of layers.
(5-4) When the wide area layer 312 and the narrow area layer 313 have compositions different from each other, the wide area layer 312 can be made of a material suitable for a function of skin adhesion at the edge of the film, and the narrow area layer 313 can be made of a material that increases a function derived from the thick film. Accordingly, functions of the respective layers can be further increased.
(5-5) When the wide area layer 312 and the narrow area layer 313 are arranged in this order in a direction from the first surface 311F of the skin-adhesive film 310 to the second surface 311R, the wide area layer 312 covers the narrow area layer 313 when the skin-adhesive film 310 is adhered to the skin. Accordingly, since the narrow area layer 313 is protected by the wide area layer 312, separation between the wide area layer 312 and the narrow area layer 313 due to friction with a finger or the like can be suppressed compared with a configuration in which the narrow area layer 313 is exposed.
(5-6) When the wide area layer 312 and the narrow area layer 313 are arranged in this order in a direction from the second surface 311R of the skin-adhesive film 310 to the first surface 311F, the wide area layer 312 is entirely in contact with the skin when the skin-adhesive film 310 is adhered to the skin. Accordingly, gaps are not likely to be formed between the skin-adhesive film 310 and the skin.
(5-7) When the narrow area layer 313 contains the particles 314, it is possible to increase functions applied from the skin-adhesive film 310 to the skin depending on the properties of the particles, or diversify these functions. In addition, since the narrow area layer 313 constitutes the thick film portion 321, it can be formed thicker than the wide area layer 312. Accordingly, the degree of freedom in types and contents of the particles 314 that can be contained can be increased compared with the case where the wide area layer 312 contains the particles 314.
(5-8) When the narrow area layer 313 has the asperity structure 315 on whichever of two surfaces of the narrow area layer 313 located farther from the wide area layer 312, it is possible to obtain an effect of making spots or the like on the skin less conspicuous due to light scattering by the asperity structure 315, and an effect of penetrating various ingredients into the skin by puncturing the skin with the asperity structure 315.
(5-9) When the narrow area layer 313 is formed of the plurality of film pieces 316 arranged with a space from each other across a plane parallel to the wide area layer 312, the conformability of the thick film portion 321 to the skin increases, which reduce the discomfort such as the feeling of skin tightness felt by the user.
(5-10) When the skin-adhesive film 310 includes the intermediate layer 317 as an anchor layer between the wide area layer 312 and the narrow area layer 313, layer-to-layer adhesion between layers constituting the skin-adhesive film 310 increases.
(5-11) When the skin-adhesive film 310 includes the end component 318 and the center component 319 which have compositions different from each other, and are in contact with each other in a plane parallel to the extending direction of the skin-adhesive film 310, air permeability through the skin-adhesive film 310 can be increased compared with a configuration in which layers made of different materials are laminated in the thickness direction. That is, the user is not likely to feel tightness at a site where the skin-adhesive film 310 is adhered.
(5-12) When the thickness of the skin-adhesive film 310 varies continuously from the minimum thickness in the thin film portion 320 to the maximum thickness in the thick film portion 321, the front surface and the rear surface of the skin-adhesive film do not have stepped portions attributed to difference in thickness. Therefore, boundaries between different thicknesses are not likely to be recognized by visual observation. Further, the skin-adhesive film 310 is prevented from being torn by friction between the stepped portions and a finger or the like.
(5-13) When the thickness of the skin-adhesive film 310 varies stepwise from the minimum thickness in the thin film portion 320 to the maximum thickness in the thick film portion 321, the skin-adhesive film 310 can be formed by laminating a plurality of flat layers. Accordingly, the skin-adhesive film 310 can be readily formed compared with a configuration in which the thickness varies continuously.

### [Appendix]

The measure for solving the foregoing problems embraces the following items as technical concepts derived from the fifth embodiment.

### (Item 31)

A skin-adhesive film including: a thin film portion having an average thickness of 0.05 µm or more and less than 0.3 µm; and a thick film portion having an average thickness of 0.3 µm or more, wherein a plurality of regions are virtually defined and arranged in a circumferential direction in a peripheral region extending along an outer edge of the skin-adhesive film as viewed in a direction perpendicular to a surface of the skin-adhesive film, the plurality of regions being included in the thin film portion.

With this configuration, since the film has a small thickness at the peripheral region in which the thin film portion is located, that is, at the edge of the skin-adhesive film, skin adhesion is increased. On the other hand, the thick film portion can provide improvement in moisturizing properties, UV blocking effect, decorative effect, and abrasion resistance. Accordingly, when the skin-adhesive film is adhered to the skin, the film can successfully exhibit functions derived from the thick film while preventing peeling at the outer edge of the film. That is, both adhesion to the skin and properties derived from the thick film can be achieved. As a result, the skin-adhesive film has increased utility in cosmetic applications.

### (Item 32)

The skin-adhesive film according to Item 31, wherein the thickness of the skin-adhesive film varies continuously from the minimum thickness in the thin film portion to the maximum thickness in the thick film portion.

With this configuration, the front surface and the rear surface of the skin-adhesive film do not have stepped portions attributed to difference in thickness. Therefore, boundaries between different thicknesses are not likely to be recognized by visual observation. Further, the skin-adhesive film is prevented from being torn by friction between the stepped portions and a finger or the like.

### (Item 33)

The skin-adhesive film according to Item 31, wherein the thickness of the skin-adhesive film varies stepwise from the minimum thickness in the thin film portion to the maximum thickness in the thick film portion.

With this configuration, the skin-adhesive film can be formed by laminating a plurality of flat layers. Accordingly, the skin-adhesive film can be readily formed compared with a configuration in which the thickness varies continuously.

### (Item 34)

The skin-adhesive film according to Item 31, including: a wide layer extending across an entirety of the skin-adhesive film when viewed in a direction perpendicular to the surface of the skin-adhesive film; and a narrow layer extending in a part of the skin-adhesive film when viewed in a direction perpendicular to the surface of the skin-adhesive film, wherein the thick film portion includes a plurality of layers which include at least part of the wide layer and at least part of the narrow layer.

With this configuration, the skin-adhesive film having a locally different thickness can be readily and appropriately achieved by laminating a plurality of layers.

### (Item 35)

The skin-adhesive film according to Item 34, including: a second surface to be adhered to the skin; and a first surface, which is a surface opposite to the second surface, wherein the wide area layer and the narrow area layer are arranged in this order in a direction from the first surface to the second surface.

With this configuration, when the skin-adhesive film is adhered to the skin, the wide area layer covers the narrow area layer. Accordingly, since the narrow area layer is protected by the wide area layer, separation between the wide area layer and the narrow area layer due to friction with a finger or the like can be suppressed compared with a configuration in which the narrow area layer is exposed.

### (Item 36)

The skin-adhesive film according to Item 34, including: a second surface to be adhered to the skin; and a first surface, which is a surface opposite to the second surface, wherein the wide area layer and the narrow area layer are arranged in this order in a direction from the second surface to the first surface.

With this configuration, since the wide area layer is entirely in contact with the skin when the skin-adhesive film is adhered to the skin, gaps are not likely to be formed between the skin-adhesive film and the skin.

### (Item 37)

The skin-adhesive film according to any one of Items 34 to 36, wherein the narrow area layer contains particles.

With this configuration, it is possible to increase functions applied from the skin-adhesive film to the skin depending on the properties of the particles, or diversify these functions. In addition, since the narrow area layer constitutes the thick film portion, it can be formed thicker than the wide area layer. Accordingly, the degree of freedom in types and contents of the particles that can be contained can be increased compared with the case where the wide area layer contains the particles.

### (Item 38)

The skin-adhesive film according to any one of Items 34 to 36, wherein the narrow area layer has the asperity structure on whichever of two surfaces of the narrow area layer located farther from the wide area layer.

With this configuration, it is possible to obtain an effect of making spots or the like on the skin less conspicuous due to scattering of light on the asperity structure, and an effect of puncturing the skin with the asperity structure to allow various ingredients to permeate into the skin.

### (Item 39)

The skin-adhesive film according to any one of Items 34 to 36, wherein the narrow area layer is formed of a plurality of portions arranged with a space from each other across a plane parallel to the wide area layer.

With this configuration, the conformability of the thick film portion to the skin increases, which reduces the discomfort such as the feeling of skin tightness at a site where the thick film portion is located felt by the user.

### (Item 40)

The skin-adhesive film according to any one of Items 34 to 36, including an anchor layer between the wide area layer and the narrow area layer.

With this configuration, layer-to-layer adhesion between layers constituting the skin-adhesive film increases.

### (Item 41)

The skin-adhesive film according to Item 31, including a plurality of portions which have compositions different from each other, and are in contact with each other in a plane parallel to the skin-adhesive film.

With this configuration, air permeability through the skin-adhesive film can be increased compared with a configuration in which layers made of different materials are laminated in the thickness direction. That is, the user is not likely to feel tightness at a site where the skin-adhesive film is adhered.

### (Item 42)

A transfer sheet including: the skin-adhesive film according to any one of Item 31 to 41; and a support substrate that supports the skin-adhesive film.

With this configuration, the skin-adhesive film is prevented from becoming deformed during storage and transport of the transfer sheet. Further, since the skin-adhesive film is supported by the support substrate, ease of handling of the skin-adhesive film is increased.

## Claims

1. A skin-adhesive film having an average thickness of 5 µm or less, wherein
a blockage ratio, which is a ratio of blocking evaporation of water having a temperature equal to a human body temperature at a site where the skin-adhesive film is provided relative to that at a site where the skin-adhesive film is not provided, is 5% or more and 60% or less.

2. A skin-adhesive film having an average thickness of 5 µm or less, comprising:
a first surface; and
a second surface, which is a surface opposite to the first surface, the second surface being configured to be adhered to a skin surface, wherein
the skin-adhesive film has a plurality of recesses that are recessed from the first surface, and
a ratio of the recesses identified by binarizing an image of the first surface by a discriminant analysis method, which is a ratio of a total area occupied by the recesses per unit area calculated based on the image binarized, is 0.05% or more and 30% or less.

3. The skin-adhesive film according to claim 2, wherein
the ratio of the total area occupied by the recesses is 0.05% or more and 25% or less.

4. The skin-adhesive film according to claim 2 or 3, wherein
each of the plurality of recesses is either a bottomed section having a bottom in the skin-adhesive film or a penetrating section penetrating through the skin-adhesive film.

5. A skin-adhesive film comprising:
a first surface; and
a second surface, which is a surface opposite to the first surface, the second surface being configured to be adhered to a skin surface, wherein
the skin-adhesive film has a portion having a thickness of 5 µm or less, and
the first surface has an arithmetic average roughness Ra of 100 nm or more and 5 µm or less, a ten-point average roughness Rz of 1 µm or more and 50 µm or less, and an average interval between peaks and valleys Sm of 10 µm or more and 500 µm or less.

6. The skin-adhesive film according to claim 5, wherein
the second surface has an arithmetic average roughness Ra of 100 nm or more and 5 µm or less, a ten-point average roughness Rz of 1 µm or more and 50 µm or less, and an average interval between peaks and valleys Sm of 10 µm or more and 500 µm or less.

7. A skin-adhesive film comprising:
a first surface; and
a second surface, which is a surface opposite to the first surface, the second surface being configured to be adhered to a skin surface, wherein
the skin-adhesive film has an average mass of 0.1 g/m² or more and 4.0 g/m² or less, and
a haze of 7% or more and 65% or less.

8. The skin-adhesive film according to claim 7, wherein
at least one of the first surface and the second surface has a DOI of 1% or more and 85% or less.

9. The skin-adhesive film according to claim 7 or 8, wherein
the skin-adhesive film has a total light transmittance of 45% or more and 95% or less.

10. A skin-adhesive film comprising:
a thin film portion having an average thickness of 0.05 µm or more and less than 0.3 µm; and
a thick film portion having an average thickness of 0.3 µm or more, wherein
a plurality of regions are virtually defined and arranged in a circumferential direction in a peripheral region extending along an outer edge of the skin-adhesive film as viewed in a direction perpendicular to a surface of the skin-adhesive film, the plurality of regions being included in the thin film portion.

11. The skin-adhesive film according to claim 10, wherein
the thin film portion has an annular shape extending along an outer edge of the skin-adhesive film when viewed in a direction perpendicular to the surface of the skin-adhesive film, the thin film portion having a width of 1 mm or more.

12. The skin-adhesive film according to claim 10, comprising:
a wide area layer extending across an entirety of the skin-adhesive film when viewed in a direction perpendicular to the surface of the skin-adhesive film; and
a narrow area layer extending in a part of the skin-adhesive film when viewed in a direction perpendicular to the surface of the skin-adhesive film, wherein
the thick film portion includes a plurality of layers which include at least part of the wide area layer and at least part of the narrow area layer.

13. The skin-adhesive film according to claim 12, wherein
the wide area layer and the narrow area layer have compositions different from each other.

14. A transfer sheet comprising:
the skin-adhesive film according to any one of claims 1 to 13; and
a support substrate that supports the skin-adhesive film.
